(19) Europäisches Patentamt
European Patent Office
Office européen des brevets

(11) EP 3 390 394 B1

(12) **EUROPEAN PATENT SPECIFICATION**

(45) Date of publication and mention of the grant of the patent:
**25.05.2022 Bulletin 2022/21**

(21) Application number: **16816359.0**

(22) Date of filing: **16.12.2016**

(51) International Patent Classification (IPC):
***C07D 405/14** (2006.01)* ***A61K 31/4439** (2006.01)*
***A61P 25/02** (2006.01)* ***A61P 25/04** (2006.01)*
***A61P 25/08** (2006.01)* ***A61P 25/16** (2006.01)*
***A61P 25/18** (2006.01)* ***A61P 25/30** (2006.01)*
***A61P 27/16** (2006.01)*

(52) Cooperative Patent Classification (CPC):
**C07D 405/14; A61P 25/02; A61P 25/04; A61P 25/08; A61P 25/16; A61P 25/18; A61P 25/20; A61P 25/22; A61P 25/24; A61P 25/30; A61P 27/16; A61P 29/00; A61P 39/02; A61P 43/00**

(86) International application number:
**PCT/GB2016/053958**

(87) International publication number:
**WO 2017/103604 (22.06.2017 Gazette 2017/25)**

(54) **HYDANTOIN MODULATORS OF KV3 CHANNELS**

HYDANTOIN-MODULATOREN VON KV3-KANÄLEN

HYDANTOÏNES UTILISÉES EN TANT QUE MODULATEURS DE CANAUX KV3

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2015 GB 201522179**

(43) Date of publication of application:
**24.10.2018 Bulletin 2018/43**

(73) Proprietor: **Autifony Therapeutics Limited**
**Stevenage Hertfordshire, SG1 2FX (GB)**

(72) Inventors:
- **ALVARO, Giuseppe**
  **Stevenage SG1 2FX (GB)**
- **MARASCO, Agostino**
  **Stevenage SG1 2FX (GB)**
- **DÉCOR, Anne**
  **37135 Verona (IT)**
- **HAMPRECHT, Dieter**
  **37135 Verona (IT)**

(74) Representative: **Kinch, Alison**
**Sagittarius IP**
**Marlow International**
**Parkway**
**Marlow SL7 1YL (GB)**

(56) References cited:
**WO-A1-2013/175211** **WO-A1-2013/182851**
**WO-A1-2015/031036** **US-A1- 2013 030 012**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 390 394 B1

## Description

### Technical field

This invention relates to novel compounds, pharmaceutical compositions containing them and to the compounds for use

in therapy, in particular as antipsychotic agents. Other uses of the compounds include the prophylaxis or treatment of hearing and hearing related disorders, including hearing loss and tinnitus, as well as schizophrenia, bipolar disorder, epilepsy, sleep disorders, and disorders where cognitive decline is a symptom.

### Background to the invention

The Kv3 voltage-gated potassium channel family includes four members, Kv3.1, Kv3.2, Kv3.3, and Kv3.4.

Kv3 channels are activated by depolarisation of the plasma membrane to voltages more positive than - 20mV; furthermore, the channels deactivate rapidly upon repolarisation of the membrane. These biophysical properties ensure that the channels open towards the peak of the depolarising phase of the neuronal action potential to initiate repolarisation. Rapid termination of the action potential mediated by Kv3 channels allows the neuron to recover more quickly to reach sub-threshold membrane potentials from which further action potentials can be triggered. As a result, the presence of Kv3 channels in certain neurons contributes to their ability to fire at high frequencies (Rudy *et al.,* 2001). Kv3.1-3 subtypes are predominant in the CNS, whereas Kv3.4 channels are found predominantly in skeletal muscle and sympathetic neurons (Weiser *et al.,* 1994). Kv3.1-3 channel subtypes are differentially expressed by sub-classes of interneurons in cortical and hippocampal brain areas (e.g. Chow *et al.,* 1999; Martina *et al.,* 1998; McDonald *et al.,* 2006; Chang *et al.,* 2007), in the thalamus (e.g. Kasten *et al.,* 2007), cerebellum (e.g. Sacco *et al.,* 2006; Puente *et al.,* 2010), and auditory brain stem nuclei (Li *et al.,* 2001).

Tetraethylammonium (TEA) has been shown to inhibit the channels at low millimolar concentrations (Rudy *et al.,* 2001), and blood-depressing substance (BDS) toxins from the sea anemone, *Anemonia sulcata* (Diochot *et al.,* 1998), have been shown to selectively inhibit Kv3 channels with high affinity (Yeung *et al.,* 2005).

Kv3 channels are important determinants of the function of the cerebellum, a region of the brain important for motor control (Joho *et al.,* 2009). Characterisation of mice in which one or more of the Kv3 subtypes has been deleted shows that the absence of Kv3.1 gives rise to increased locomotor activity, altered electroencephalographic activity, and a fragmented sleep pattern (Joho *et al.,* 1999). The deletion of Kv3.2 leads to a reduction in seizure threshold and altered cortical electroencephalographic activity (Lau *et al.,* 2000). Deletion of Kv3.3 is associated with mild ataxia and motor deficits (McMahon *et al.,* 2004). Double deletion of Kv3.1 and Kv3.3 gives rise to a severe phenotype characterised by spontaneous seizures, ataxia, and an increased sensitivity to the effects of ethanol (Espinosa *et al.,* 2001; Espinosa *et al.,* 2008). Mutations of the Kv3.3 gene in humans have been associated with forms of spinocerebellar ataxia (SCA13) (Figueroa *et al.,* 2010).

Bipolar disorder, schizophrenia, anxiety, and epilepsy are serious disorders of the central nervous system that have been associated with reduced function of inhibitory interneurons and gamma-amino butyric acid (GABA) transmission (Reynolds *et al.,* 2004; Benes *et al.,* 2008; Brambilla *et al.,* 2003; Aroniadou-Anderjaska *et al.,* 2007; Ben-Ari, 2006). Parvalbumin positive basket cells that express Kv3 channels in the cortex and hippocampus play a key role in generating feedback inhibition within local circuits (Markram *et al.,* 2004). Given the relative dominance of excitatory synaptic input over inhibitory input to glutamatergic pyramidal neurons in these circuits, fast-firing of interneurons supplying inhibitory input is essential to ensure balanced inhibition. Furthermore, accurate timing of inhibitory input is necessary to sustain network synchronisation, for example, in the generation of gamma frequency field potential oscillations that have been associated with cognitive function (Fisahn *et al.,* 2005; Engel *et al.,* 2001). Notably, a reduction in gamma oscillations has been observed in patients with schizophrenia (Spencer *et al.,* 2004). Consequently, positive modulators of Kv3 channels might be expected to enhance the firing capabilities of specific groups of fast-firing neurons in the brain. These effects may be beneficial in disorders associated with abnormal activity of these neuronal groups. In addition, Kv3.2 channels have been shown to be expressed by neurons of the superchiasmatic nucleus (SCN) the main circadian pacemaker in the CNS (Schulz *et al.,* 2009).

Voltage-gated ion channels of the Kv3 family are expressed at high levels in auditory brainstem nuclei (Li *et al.,* 2001) where they permit the fast firing of neurons that transmit auditory information from the cochlear to higher brain regions. Phosphorylation of Kv3.1 and Kv3.3 channels in auditory brainstem neurons is suggested to contribute to the rapid physiological adaptation to sound levels that may play a protective role during exposure to noise (Desai *et al.,* 2008; Song *et al.,* 2005). Loss of Kv3.1 channel expression in central auditory neurons is observed in hearing impaired mice (von Hehn *et al.,* 2004); furthermore, a decline in Kv3.1 expression may be associated with loss of hearing in aged mice (Jung *et al.* 2005), and loss of Kv3 channel function may also follow noise-trauma induced hearing loss (Pilati *et al.,* 2012). Furthermore, pathological plasticity of auditory brainstem networks is likely to contribute to symptoms that

are experienced by many people suffering from hearing loss of different types. Recent studies have shown that regulation of Kv3.1 channel function and expression has a major role in controlling auditory neuron excitability (Kaczmarek *et al.,* 2005), suggesting that this mechanism could account for some of the plastic changes that give rise to tinnitus. Tinnitus may follow noise-induced hearing loss as a result of adaptive changes in central auditory pathways from brainstem to auditory cortex (Roberts *et al.,* 2010). Kv3.1 and/or Kv3.2 channels are expressed in many of these circuits and contribute to the function of GABAergic inhibitory interneurons that may control the function of these circuits.

In the broadest sense, pain can be grouped in to acute pain and chronic pain. Acute pain is defined as pain that is self-limited and generally requires treatment for no more than up to a few weeks, for example postoperative or acute musculoskeletal pain, such as fractures (US Food and Drug Administration, 2014). Chronic pain can be defined either as pain persisting for longer than 1 month beyond resolution of the initial trauma, or pain persisting beyond three months. There is often no clear cause of chronic pain, and a multitude of other health problems such as fatigue, depression, insomnia, mood changes and reduction in movement, often accompany chronic pain.

Chronic pain can be sub-divided in to the following groups: neuropathic pain, chronic musculoskeletal pain and miscellaneous chronic pain. Neuropathic pain usually accompanies tissue injury and is initiated or caused by damage to the nervous system (peripheral nervous system and/or central nervous system), such as amputation, stroke, diabetes, or multiple sclerosis. Chronic musculoskeletal pain can be a symptom of diseases such as osteoarthritis and chronic lower back pain and can occur following damage to muscle tissue as well as trauma to an area for example, fractures, sprains and dislocation. Miscellaneous chronic pain encompasses all other types of long term pain and includes non-neuropathic pain conditions such as cancer pain and fibromyalgia as well as headaches and tendinitis.

Chronic pain is a highly heterogeneous condition that remains amongst the most troublesome and difficult to manage of clinical indications (McCarberg *et al.,* 2008; Woolf 2010; Finnerup *et al.,* 2015). Despite years of research and drug development, there has been little progress in identifying treatments that can match the opioids for efficacy without significant side effects and risk of dependence. Voltage-gated ion channels have been important targets for the management of specific pain indications, in particular neuropathic pain states. Furthermore, genetic mutations in specific ion channels have been linked to some chronic pain disorders (Bennett *et al.,* 2014). Examples of voltage-gated ion channels that are being explored as pharmaceutical targets include: *Sodium channels (in particular NaV1.7)* - Sun *et al.,* 2014; Dib-Hajj *et al.,* 2013; *N-type calcium channels* - Zamponi *et al.,* 2015; *Kv7 potassium channels* - Devulder 2010; Wickenden *et al.,* 2009; and *SLACK* - Lu *et al.,* 2015.

The basic hypothesis underlying these approaches is that chronic pain states are associated with increased excitability and/or aberrant firing of peripheral sensory neurons, in particular neurons involved in the transmission of painful sensory stimuli, such as the C-fibres of the dorsal root ganglia and specific circuits within the spinal cord (Baranauskas *et al.,* 1998; Cervero 2009; Woolf *et al.,* 2011; Baron *et al.,* 2013). Animal models of neuropathic and inflammatory chronic pain provide the main support for this hypothesis, although demonstration of causality is still lacking (Cervero 2009).

Drugs targeting hyperexcitability, such as sodium channel blockers (e.g. CNV1014802, lamotrigine, carbamazepine, and local anaesthetics), Kv7 positive modulators (e.g. flupertine and retigabine), and N-type calcium channel modulators (e.g. gabapentin, which interacts with the $\alpha2\delta$ subunit of the N-type calcium channel, and ziconitide, derived from a cone snail toxin) show efficacy in models of inflammatory and/or neuropathic pain. However, amongst these drugs, there is mixed evidence for clinical efficacy, for example, balancing efficacy and increased burden of side effects on the central nervous system. The disparity between efficacy in animal models and efficacy in humans is likely to be due to a range of factors, but in particular, drug concentration achievable in humans (due to poor tolerability) and heterogeneity of human pain conditions are likely to be the main culprits. For pain indications, there is also a need to identify targets through which pain relief can be achieved with reduced tolerance or tachyphylaxis and reduced abuse liability and/or risk of dependence.

Thus, improving the pharmacological management of pain is focused on mechanisms that can deliver good efficacy with a reduced side-effect burden, reduced tolerance or tachyphylaxis, and reduced abuse liability and/or risk of dependence.

Recently, Kv3.4 channels have become a target of interest for the treatment of chronic pain. Kv3.4 channels are expressed on neurons of the dorsal root ganglia (Ritter *et al.,* 2012; Chien *et al.,* 2007), where they are predominantly expressed on sensory C-fibres (Chien *et al.,* 2007). Kv3 channels are also expressed by specific subsets of neurons in the spinal cord. Specifically, Kv3.1b (Deuchars *et al.,* 2001; Brooke *et al.,* 2002), Kv3.3 (Brooke *et al.,* 2006), and Kv3.4 subunits (Brooke *et al.,* 2004) have been identified in rodent spinal cord, although not always in association with circuits involved with sensory processing.

Recent animal model data suggest a down-regulation of Kv3.4 channel surface expression in DRG neurons following spinal cord injury associated with hypersensitivity to painful stimuli (Ritter *et al.,* 2015). Similarly, it has been observed that there is a down-regulation of Kv3.4 expression in DRGs of rodents following spinal cord ligation (Chien *et al.,* 2007). This latter study also showed that intrathecal administration to rats of an antisense oligonucleotide to supress the expression of Kv3.4 led to hypersensitivity to mechanical stimuli. It has been shown that Kv3.4 channel

inactivation could be influenced by protein kinase C-dependent phosphorylation of the channels, and that this physiological mechanism might allow DRG neurons to alter their firing characteristics in response to painful stimuli (Ritter *et al.,* 2012). These studies suggest a causal relationship between the emergence of mechanical allodynia and reduced Kv3.4 channel expression or function. No evaluation of Kv3.1, Kv3.2, or Kv3.3 expression in SC or DRG neurons was conducted in any of these studies, and expression of these two subtypes has not been explicitly demonstrated on DRG neurons (although as mentioned above, they are abundant within specific regions of the spinal cord). The *in vivo* studies reported above provide a rationale for modulation of Kv3.4 as a novel approach to the treatment of certain neuropathic pain states. There are currently no data specifically linking Kv3.1 and/or Kv3.2 and/or Kv3.3 channel subtypes to pain processing.

Dementia with Lewy Bodies (DLB) and Parkinson's disease (PD) are serious neurodegenerative disorders that are associated with the accumulation of the protein, alpha-synuclein in Lewy bodies, which leads to loss of connectivity and neuronal cell death. Symptoms of DLB include progressive cognitive deficits, in particular difficulties with planning and attention. Visual hallucinations are also common, occurring in approximately 60% of patients. PD is associated initially with motor deficits, primarily due to loss of dopamine neurons. While there are currently no studies directly linking Kv3 channels to DLB or PD, the location and role of Kv3 channels, in particular Kv3.1, in cortical and basal ganglia circuits suggests that modulators of these channels could improve symptoms of DLB or PD, either alone, or in combination with current treatments, such as acetyl-cholinesterase inhibitors for DLB or L-DOPA for PD.

Patent applications WO2011/069951, WO2012/076877, WO2012/168710, WO2013/175215 and WO2013/182851 disclose compounds which are modulators of Kv3.1 and Kv3.2. Further, the utility of such compounds is demonstrated in animal models of seizure, hyperactivity, sleep disorders, psychosis, hearing disorders and bipolar disorders.

Patent application WO2013/175211 discloses that modulation of Kv3.1, Kv3.2 and/or Kv3.3 channels has been found to be beneficial in preventing or limiting the establishment of a permanent hearing loss resulting from acute noise exposure. The benefits of such prevention may be observed even after administration of the Kv3.1, Kv3.2 and/or Kv3.3 modulator has ceased.

There remains a need for the identification of alternative modulators of Kv3.1, Kv3.2 and/or Kv3.3, in particular modulators of Kv3.1 and/or Kv3.2. Such modulators may demonstrate high *in vivo* potency, channel selectivity or desirable pharmacokinetic parameters, for example high brain availability, that reduces the dose required for therapeutic effect *in vivo.* Compounds which have balanced Kv3.1, Kv3.2 and/or Kv3.3 modulatory properties may be desirable e.g. compounds with modulate Kv3.1 and Kv3.2 to the same, or a similar extent. For certain therapeutic indications, there is also a need to identify compounds with a different modulatory effect on Kv3.1, Kv3.2 and/or Kv3.3 channels, for example, compounds that alter the kinetics of channel gating or channel inactivation, and which may behave *in vivo* as negative modulators of the channels.

## Summary of the invention

The present invention provides a compound of formula (I):

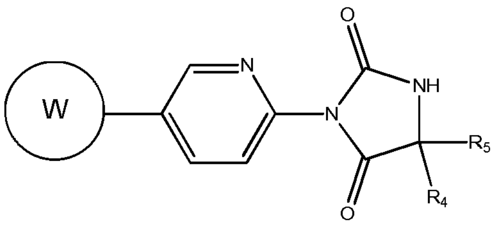

(I)

wherein:

W is group (Wa), group (Wb) or group (Wc):

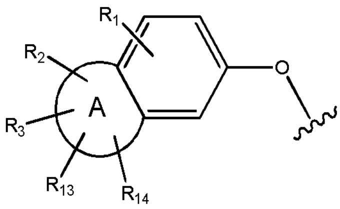 (Wa); 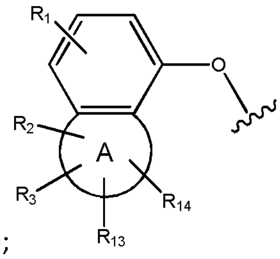 (Wb); 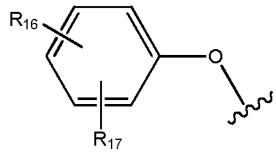 (Wc);

wherein:

$R_1$ is H, $C_{1-4}$alkyl, halo, halo$C_{1-4}$alkyl, CN, $C_{1-4}$alkoxy, or halo$C_{1-4}$alkoxy;
$R_2$ is H, $C_{1-4}$alkyl, $C_{3-5}$ spiro carbocyclyl, halo$C_{1-4}$alkyl or halo;
$R_3$ is H, $C_{1-4}$alkyl, halo$C_{1-4}$alkyl, halo; or $R_3$ is absent;
$R_{13}$ is H, $C_{1-4}$alkyl, halo$C_{1-4}$alkyl, halo; or $R_{13}$ is absent;
$R_{14}$ is H, $C_{1-4}$alkyl, halo$C_{1-4}$alkyl, halo; or $R_{14}$ is absent;
A is a 5 or 6 membered saturated or unsaturated heterocycle, with at least one O atom; which heterocycle is optionally fused with a cyclopropyl group, or a cyclobutyl group, or a cyclopentyl group to form a tricycle when considered together with the phenyl;
$R_{16}$ is halo, $C_{1-4}$ alkyl, $C_{1-4}$ alkoxy, halo-$C_{1-4}$alkyl, halo-$C_{1-4}$alkoxy, or CN;
$R_{17}$ is H, halo, cyano, $C_{1-4}$alkyl or $C_{1-4}$ alkoxy; with the proviso that when $R_{17}$ is H, $R_{16}$ is not in the para position;
$R_4$ is $C_{1-4}$ alkyl;
$R_5$ is H or $C_{1-4}$ alkyl;
or $R_4$ and $R_5$ can be fused to form $C_{3-4}$ spiro carbocyclyl;
wherein $R_2$ and $R_3$ may be attached to the same or a different ring atom; $R_2$ may be attached to a fused ring atom; and wherein $R_{13}$ and $R_{14}$ may be attached to the same or a different ring atom.

A compound of formula (I) may be provided in the form of a pharmaceutically acceptable salt and/or solvate thereof and/or prodrug thereof, wherein the prodrug is functionalised at the secondary nitrogen of the hydantoin, as defined below:

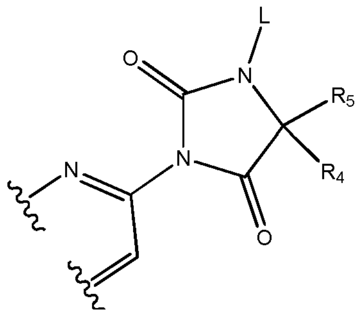

wherein L is selected from:

a) $-PO(OH)O^- \bullet M^+$, wherein $M^+$ is a pharmaceutically acceptable monovalent counterion,
b) $-PO(O^-)_2 \bullet 2M^+$,
c) $-PO(O^-)_2 \bullet D^{2+}$, wherein $D^{2+}$ is a pharmaceutically acceptable divalent counterion,
d) $-CH(R^X)-PO(OH)O^- \bullet M^+$, wherein $R^X$ is hydrogen or $C_{1-3}$ alkyl,
e) $-CH(R^X)-PO(O^-)_2 \bullet M^+$,
f) $-CH(R^X)-PO(O^-)_2 \bullet D^{2+}$
g) $-SO_3 \bullet M^+$,
h) $-CH(R^X)-SO_3^- \bullet M^+$, and
i) $-CO-CH_2CH_2-CO_2 \bullet M^+$.

In one embodiment of the invention a compound of formula (I) is provided in the form of a pharmaceutically acceptable salt.

The compounds of formula (I) may be used as medicaments, in particular for the prophylaxis or treatment of

hearing disorders, including hearing loss and tinnitus, as well as schizophrenia, bipolar disorder, epilepsy, sleep disorders, cognition impairment or ataxia. The compounds of formula (I) may also be used for the prophylaxis or treatment of substance abuse disorders or pain such as neuropathic pain, inflammatory pain or miscellaneous pain. Compounds of formula (I) may also be used in the prophylaxis of acute noise-induced hearing loss.

Also provided are pharmaceutical compositions containing a compound of formula (I) and a pharmaceutically acceptable carrier or excipient.

Additionally provided are prodrug derivatives of the compounds of formula (I).

## Brief description of the Figures

The present invention is illustrated, by way of example only, with reference to the following figures in which:

Figure 1a hKv3.2 currents recorded using the assay described in Biological Example 1. Data shown are the individual currents over the period of the depolarising voltage step to -15mV recorded from 4 different cells at two concentrations of the compound of Reference Example RE1. The data are fitted by a single exponential curve (solid lines) using the fitting procedure in Prism version 5 (Graphpad Software Inc).

Figure 1b hKv3.2 currents recorded using the assay described in Biological Example 1. Data shown are the individual currents over the period of the depolarising voltage step to -15mV recorded from 2 different cells at two concentrations of compound of Reference Example RE3. The data are fitted by a single exponential curve (solid lines) using the fitting procedure in Prism version 5 (Graphpad Software Inc).

Figure 2 Recordings made from identified "fast-firing" interneurons in the somatosensory cortex of the mouse.

## Detailed description of the invention

The present invention provides compounds of formula (I):

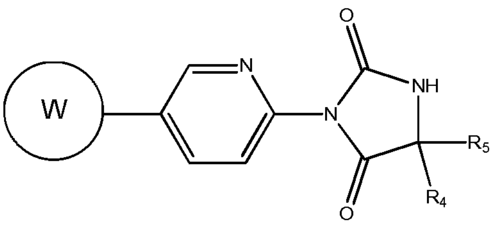

(I)

wherein:

W is group (Wa), group (Wb) or group (Wc):

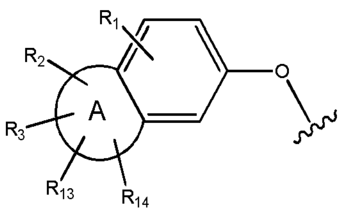

(Wa);

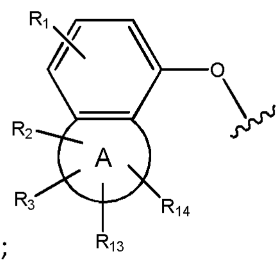

(Wb);

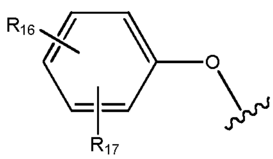

(Wc);

wherein:

$R_1$ is H, $C_{1-4}$alkyl, halo, halo$C_{1-4}$alkyl, CN, $C_{1-4}$alkoxy, or halo$C_{1-4}$alkoxy;
$R_2$ is H, $C_{1-4}$alkyl, $C_{3-5}$ spiro carbocyclyl, halo$C_{1-4}$alkyl or halo;
$R_3$ is H, $C_{1-4}$alkyl, halo$C_{1-4}$alkyl, halo; or $R_3$ is absent;
$R_{13}$ is H, $C_{1-4}$alkyl, halo$C_{1-4}$alkyl, halo; or $R_{13}$ is absent;
$R_{14}$ is H, $C_{1-4}$alkyl, halo$C_{1-4}$alkyl, halo; or $R_{14}$ is absent;
A is a 5 or 6 membered saturated or unsaturated heterocycle, with at least one O atom; which heterocycle is optionally

fused with a cyclopropyl group, or a cyclobutyl group, or a cyclopentyl group to form a tricycle when considered together with the phenyl;
$R_{16}$ is halo, $C_{1-4}$ alkyl, $C_{1-4}$ alkoxy, halo-$C_{1-4}$alkyl, halo-$C_{1-4}$alkoxy, or CN;
$R_{17}$ is H, halo, cyano, $C_{1-4}$alkyl or $C_{1-4}$ alkoxy; with the proviso that when $R_{17}$ is H, $R_{16}$ is not in the para position;
$R_4$ is $C_{1-4}$ alkyl;
$R_5$ is H or $C_{1-4}$ alkyl;
or $R_4$ and $R_5$ can be fused to form $C_{3-4}$ spiro carbocyclyl;
wherein $R_2$ and $R_3$ may be attached to the same or a different ring atom; $R_2$ may be attached to a fused ring atom; and wherein $R_{13}$ and $R_{14}$ may be attached to the same or a different ring atom;

or a pharmaceutically acceptable salt and/or solvate thereof, and/or prodrug thereof, wherein the prodrug is functionalised at the secondary nitrogen of the hydantoin, as defined below:

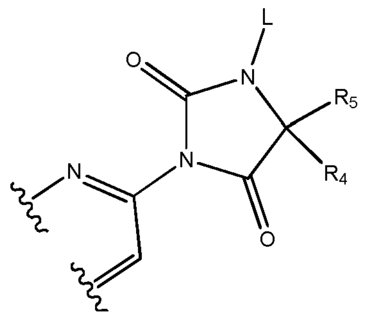

wherein L is selected from:

j) $-PO(OH)O^{-} \bullet M^{+}$, wherein $M^{+}$ is a pharmaceutically acceptable monovalent counterion,
k) $-PO(O^{-})_2 \bullet M^{+}$,
l) $-PO(O^{-})_2 \bullet D^{2+}$, wherein $D^{2+}$ is a pharmaceutically acceptable divalent counterion,
m) $-CH(R^{X})-PO(OH)O^{-} \bullet M^{+}$, wherein $R^{X}$ is hydrogen or $C_{1-3}$ alkyl,
n) $-CH(R^{X})-PO(O^{-})_2 \bullet M^{+}$,
o) $-CH(R^{X})-PO(O^{-})_2 \bullet D^{2+}$
p) $-SO_3^{-} \bullet M^{+}$,
q) $-CH(R^{X})-SO_3^{-} \bullet M^{+}$, and
r) $-CO-CH_2CH_2-CO_2 \bullet M^{+}$.

Compounds of formula (I) may optionally be provided in the form of a pharmaceutically acceptable salt and/or solvate. In one embodiment of the invention a compound of formula (I) is provided in the form of a pharmaceutically acceptable salt. In a second embodiment of the invention a compound of formula (I) is provided in the form of a pharmaceutically acceptable solvate. In a third embodiment of the invention a compound of formula (I) is not in the form of a salt or solvate.
In one embodiment of the invention, $R_1$ is H, $C_{1-4}$alkyl, halo, halo$C_{1-4}$alkyl or CN. Suitably, $R_1$ is H, $C_{1-4}$alkyl, halo or halo$C_{1-4}$alkyl. In one embodiment of the invention $R_1$ is H, methyl or CN. In another embodiment of the invention $R_1$ is H or methyl. In one embodiment of the invention $R_1$ is H. In another embodiment of the invention $R_1$ is $C_{1-4}$alkyl, in particular methyl. When W is group (Wa), suitably $R_1$ is H. When W is group (Wb), suitably $R_1$ is H or methyl.
When W is group (Wb), suitably $R_1$ is positioned at the para position of the phenyl ring, as illustrated below:

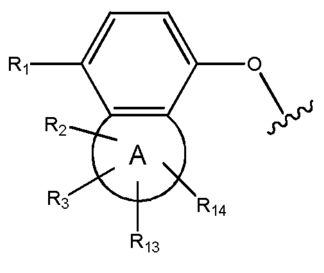

In this embodiment, suitably $R_1$ is not H.

Suitably $R_2$ is H, $C_{1-4}$alkyl, $C_{3-5}$spiro carbocyclyl, or halo$C_{1-4}$alkyl. In one embodiment of the invention, $R_2$ is H, $C_{1-4}$alkyl, $C_{3-5}$spiro carbocyclyl or halo. In one embodiment of the invention $R_2$ is $C_{1-4}$alkyl, in particular methyl, ethyl, isopropyl, tert-butyl or cyclopropyl, especially methyl, ethyl, isopropyl or tert-butyl. In one embodiment of the invention, $R_2$ is methyl. In one embodiment of the invention $R_2$ is $C_{3-5}$spiro carbocyclyl. In one embodiment of the invention $R_2$ is $C_3$spiro carbocyclyl. In another embodiment of the invention $R_2$ is $C_4$ spiro carbocyclyl. In a further embodiment of the invention $R_2$ is $C_5$spiro carbocyclyl. In one embodiment of the invention $R_2$ is halo$C_{1-4}$alkyl, in particular trifluoromethyl or 2,2,2-trifluoroethyl. In one embodiment of the invention $R_2$ is halo, in particular fluoro. In another embodiment of the invention $R_2$ is H.

In one embodiment of the invention $R_3$ is H, $C_{1-4}$alkyl, halo$C_{1-4}$alkyl or halo. Alternatively, $R_3$ is H, $C_{1-4}$alkyl, or halo$C_{1-4}$alkyl. Suitably $R_3$ is H or $C_{1-4}$alkyl. In one embodiment of the invention $R_3$ is H. In one embodiment of the invention $R_3$ is $C_{1-4}$alkyl, in particular methyl, ethyl, isopropyl, tert-butyl or cyclopropyl, especially methyl, ethyl, isopropyl or tert-butyl, such as methyl or ethyl. In one embodiment of the invention, $R_3$ is methyl. In one embodiment of the invention, $R_3$ is halo$C_{1-4}$alkyl, in particular trifluoromethyl or 2,2,2-trifluoroethyl. In one embodiment of the invention $R_3$ is halo, in particular fluoro. The skilled person will appreciate that, depending on the size, presence of heteroatoms and the degree of unsaturation of the A ring, $R_3$ may be absent. Consequently, in another embodiment of the invention $R_3$ is absent. Suitably $R_3$ is H, methyl or trifluoromethyl.

In one embodiment of the invention $R_2$ may be H, $C_{1-4}$alkyl, halo$C_{1-4}$alkyl or $C_{3-5}$spiro carbocycyl and $R_3$ may be H, $C_{1-4}$alkyl, or halo$C_{1-4}$alkyl. In a particular embodiment of the invention, $R_2$ may be methyl, ethyl, isopropyl, tert-butyl, cyclopropyl, $C_{3-5}$spiro carbocyclyl, trifluoromethyl or 2,2,2-trifluoroethyl and $R_3$ may be H, methyl, ethyl or trifluoromethyl. In certain embodiments of the invention $R_3$ is H and $R_2$ is H, methyl, ethyl, isopropyl or $C_{3-4}$ spiro carbocyclyl. In further embodiments of the invention $R_3$ and $R_2$ are both fluoro (such as attached to the same ring carbon atom). In one embodiment of the invention $R_2$ is $C_{1-4}$alkyl and $R_3$ is H, for example $R_2$ is methyl, ethyl, tert-butyl or cyclopropyl. In one embodiment of the invention $R_2$ is $C_{1-4}$alkyl and $R_3$ is $C_{1-4}$alkyl, for example $R_2$ is methyl and $R_3$ is methyl, $R_2$ is ethyl and $R_3$ is ethyl or $R_2$ is methyl and $R_3$ is ethyl. In another embodiment of the invention $R_2$ is trifluoromethyl and $R_3$ is methyl.

In one embodiment of the invention $R_2$ and $R_3$ are attached to the same ring atom. In an alternative embodiment of the invention $R_2$ and $R_3$ are attached to different ring atoms.

In one embodiment of the invention $R_{13}$ is H, F or methyl. In one embodiment of the invention $R_{13}$ is H. In another embodiment of the invention $R_{13}$ is $C_{1-4}$alkyl, in particular methyl. In a further embodiment of the invention $R_{13}$ is halo, in particular fluoro. In an additional embodiment of the invention $R_{13}$ is halo$C_{1-4}$alkyl, such as trifluoromethyl. The skilled person will appreciate that, depending on the size, presence of heteroatoms and the degree of unsaturation of the A ring, $R_{13}$ may be absent. Consequently, in another embodiment of the invention $R_{13}$ is absent.

In one embodiment of the invention $R_{14}$ is H, F or methyl. In one embodiment of the invention $R_{14}$ is H. In another embodiment of the invention $R_{14}$ is $C_{1-4}$alkyl, in particular methyl. In a further embodiment of the invention $R_{14}$ is halo, in particular fluoro. In an additional embodiment of the invention $R_{13}$ is halo$C_{1-4}$alkyl, such as trifluoromethyl. The skilled person will appreciate that, depending on the size, presence of heteroatoms and the degree of unsaturation of the A ring, $R_{14}$ may be absent. Consequently, in another embodiment of the invention $R_{14}$ is absent.

In one embodiment of the invention $R_{13}$ and $R_{14}$ are attached to the same ring atom. In an alternative embodiment of the invention $R_{13}$ and $R_{14}$ are attached to different ring atoms.

In certain embodiments of the invention $R_2$, $R_3$, $R_{13}$ and $R_{14}$ are each independently selected from H, $C_{1-4}$alkyl, halo$C_{1-4}$alkyl and halo, such as H, $C_{1-4}$alkyl and halo$C_{1-4}$alkyl. Suitably $R_2$, $R_3$, $R_{13}$ and $R_{14}$ are each independently selected from H, F, methyl and trifluoromethyl.

Suitably, A is a 5 or 6 membered saturated or unsaturated heterocycle, with at least one O atom; which heterocycle is optionally fused with a cyclopropyl group to form a tricycle when considered together with the phenyl. In one embodiment of the invention A is a 5 membered saturated or unsaturated heterocycle, with at least one O atom; which heterocycle is optionally fused with a cyclopropyl group, a cyclobutyl group or a cyclopentyl group to form a tricycle when considered together with the phenyl. In another embodiment of the invention A is a 6 membered saturated or unsaturated heterocycle, with at least one O atom; which heterocycle is optionally fused with a cyclopropyl group, a cyclobutyl group or a cyclopentyl group to form a tricycle when considered together with the phenyl.

In one embodiment of the invention A is a 5 membered saturated or unsaturated heterocycle with at least one O atom, which heterocycle is fused with a cyclopropyl group to form a tricycle when considered together with the phenyl. In another embodiment of the invention A is a 6 membered saturated or unsaturated heterocycle with at least one O atom, which heterocycle is fused with a cyclopropyl group to form a tricycle when considered together with the phenyl. In one embodiment of the invention A is a 5 membered saturated or unsaturated heterocycle with at least one O atom. In one embodiment of the invention A is a 6 membered saturated or unsaturated heterocycle with at least one O atom. In one embodiment, ring A is a 5 membered saturated heterocycle, with at least one O atom; which heterocycle is optionally fused with a cyclopropyl group, or a cyclobutyl group, or a cyclopentyl group to form a tricycle when considered together with the phenyl.

In certain embodiments of the invention the ring A contains one heteroatom. In one embodiment, ring A contains one heteroatom which is oxygen. In other embodiments of the invention the ring A contains two heteroatoms (e.g. two oxygen atoms, one oxygen atom and one nitrogen atom, or one oxygen atom and one sulphur atom), in particular two oxygen atoms or one oxygen atom and one nitrogen atom.

In one embodiment, A is dihydrofuran, isoxazole, dihydropyran, 1,3-dioxolane, 1,3-oxazine or dihydropyran. Suitably, A is dihydrofuran, isoxazole, dihydropyran, 1,3-dioxolane, 1,3-oxazine or dihydropyran fused with a cyclopropyl group.

In one embodiment of the invention A is dihydrofuran. In one embodiment of the invention A is dihydropyran. In another embodiment of the invention A is dihydrofuran fused with a cyclopropyl group, a cyclobutyl group or a cyclopentyl group. In another embodiment of the invention A is dihydropyran fused with a cyclopropyl group, a cyclobutyl group or a cyclopentyl group. In a further embodiment the invention A is dihydrofuran fused with a cyclopropyl group. In still further embodiment the invention A is dihydropyran fused with a cyclopropyl group.

In one embodiment of the invention A is fused with a cyclopropyl group. In another embodiment A is fused with a cyclobutyl group. In a further embodiment of the invention A is fused with a cyclopentyl group. In one embodiment of the invention A is not fused with a cyclopropyl group, a cyclobutyl group or a cyclopentyl group.

In one embodiment of the invention W is group (Wa):

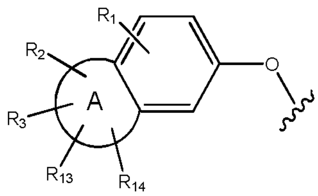

(Wa).

In one embodiment of the invention W is group (Wb):

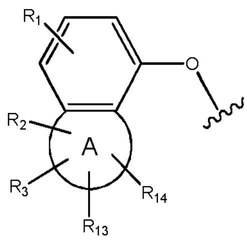

(Wb).

In one embodiment of the invention A is dihydrofuran, dihydropyran, furan, pyran, oxazole, isoxazole, oxazine, dioxine or 1,3-dioxalane. In another embodiment A is dihydrofuran, dihydropyran or 1,3-dioxalane.

In one embodiment of the invention A is:

**1** 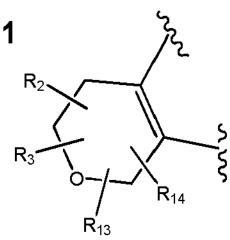 ,

**2** 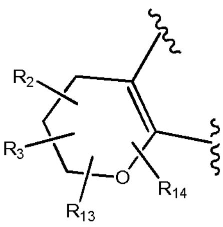 ,

**3** 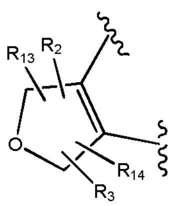 ,

**4** 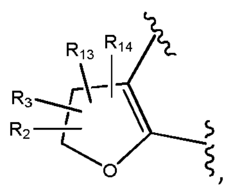 ,

**5** 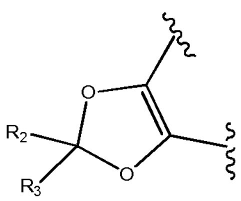 , , ,

**6** 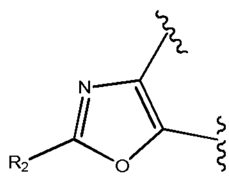 ,

**7** 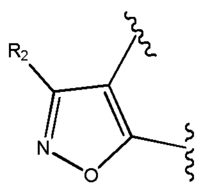

**8** 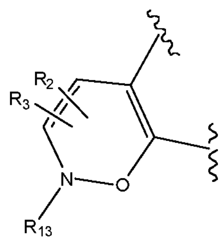 ,

**9** 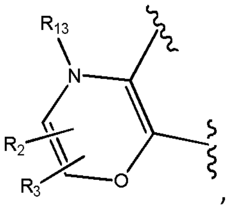 , or **10** 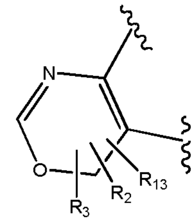

wherein

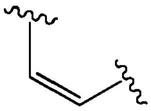

denotes a portion of the phenyl ring to which ring A is fused.

In one embodiment of the invention A is:

**11** 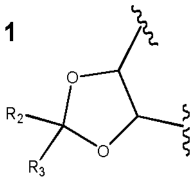

wherein

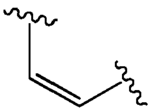

denotes a portion of the phenyl ring to which ring A is fused.

In another embodiment of the invention A is:

**1** 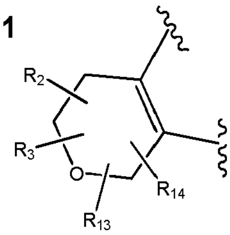 , **2** 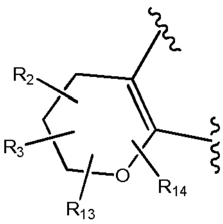 , **3** 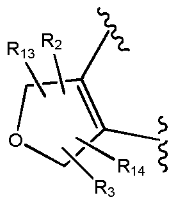 , **4** 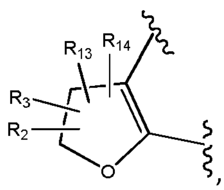 ,

**5** 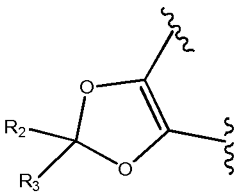 , **7** 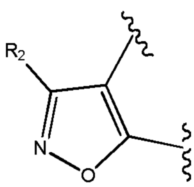 , or . **10** 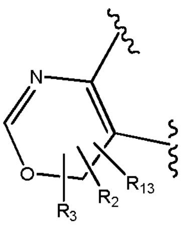 .

wherein

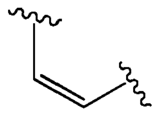

denotes a portion of the phenyl ring to which ring A is fused.

In one embodiment of the invention, ring A is selected from the group consisting of:

**1**

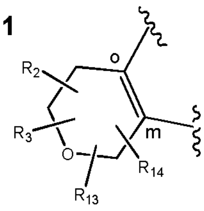

;

**2**

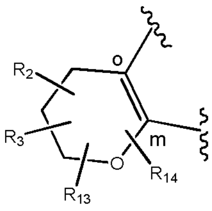

;

**3**

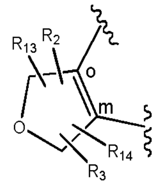

;

**4**

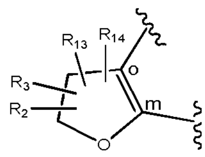

;

**5**

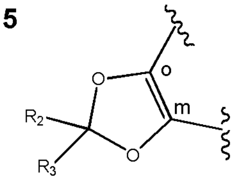

;

**6**

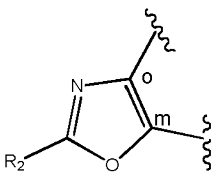

;

**7**

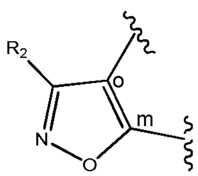

;

**8**

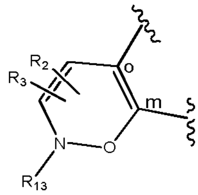

;

**9**

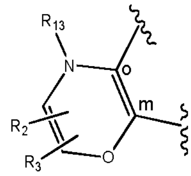

;

**10**

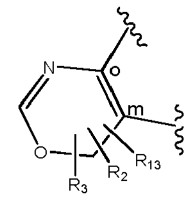

; and

**11**

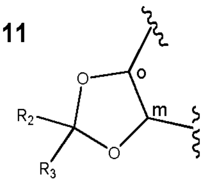

wherein

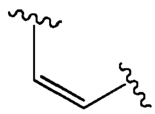

denotes a point at which ring A is fused to the phenyl ring, and "o" and "m" indicate the ortho- and meta-positions of the phenyl ring to which group A is fused.

In one embodiment of the invention, ring A is selected from the group consisting of:

**1**

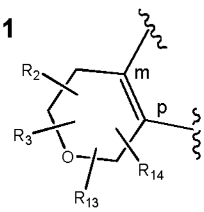

;

**2**

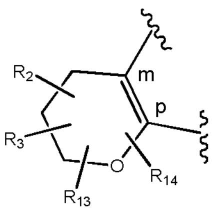

;

**3**

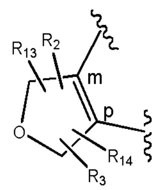

;

**4**

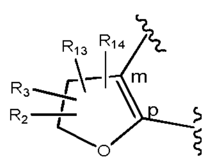

;

**5**

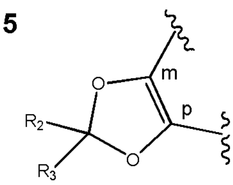

;

**6**

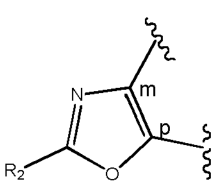

;

**7**

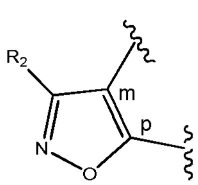

;

**8**

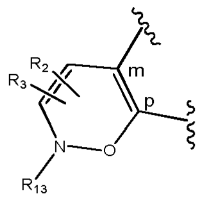

;

**9**

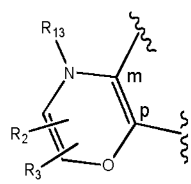

;

10 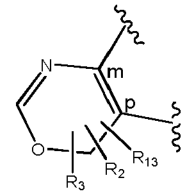 ; and 11 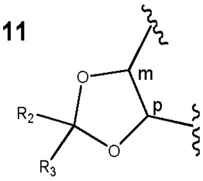

wherein

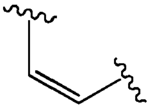

denotes a point at which ring A is fused to the phenyl ring, wherein "m" and "p" indicate the meta- and para-positions of the phenyl ring to which group A is fused.

In one embodiment of the invention, ring A is:

1 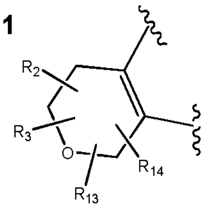 ; 2 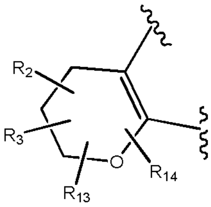 ; 3 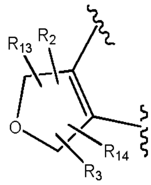 ; 4 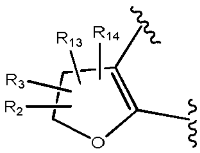 or 11 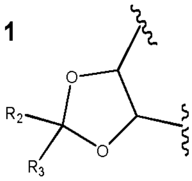

wherein

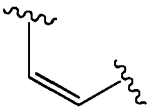

denotes a portion of the phenyl ring to which ring A is fused.

In one embodiment of the invention, ring A is:

1 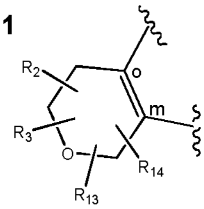 ; 2 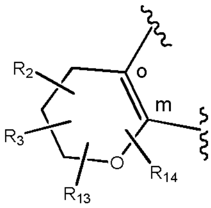 ; 3 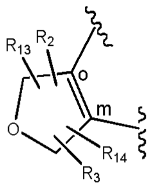 ; 4 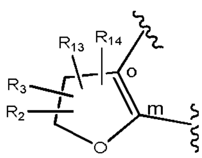 or 11 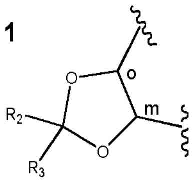

wherein

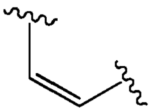

denotes a portion of the phenyl ring to which ring A is fused.

In one embodiment of the invention, ring A is:

**1** 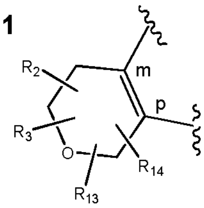 ; **2** 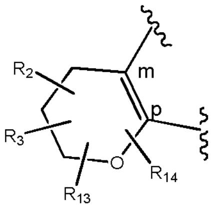 ; **3** 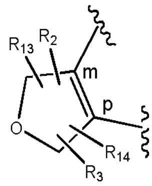 ; **4** 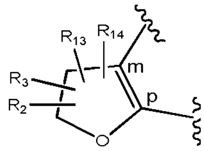 or **11** 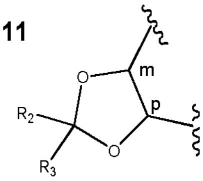

wherein

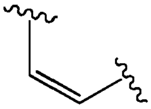

denotes a portion of the phenyl ring to which ring A is fused.

In a further embodiment of the invention A is:

**1** 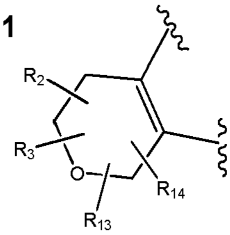 , **2** 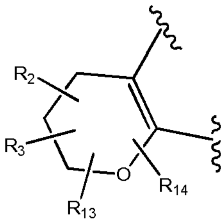 , **3** 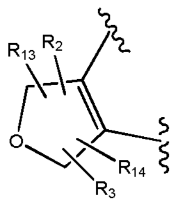 or **4** 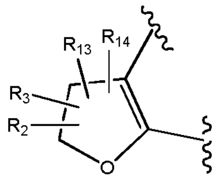

wherein

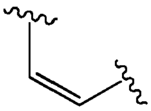

denotes a portion of the phenyl ring to which ring A is fused.

In a particular embodiment of the invention, Ring A is:

**3** 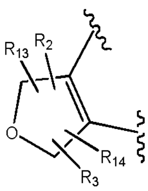 ; or **4** 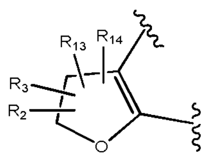

wherein

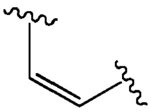

denotes a portion of the phenyl ring to which ring A is fused.

In a particular embodiment, Ring A is:

3 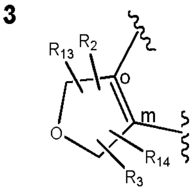 4 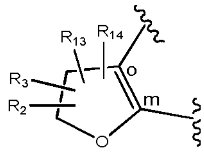

; or

wherein

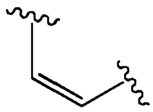

denotes a portion of the phenyl ring to which ring A is fused.

In a particular embodiment of the invention, Ring A is:

3 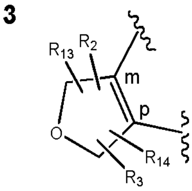 4 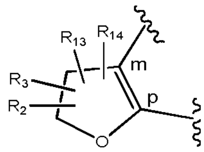

; or

wherein

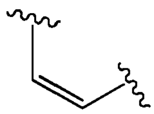

denotes a portion of the phenyl ring to which ring A is fused.

In a further embodiment of the invention, ring A is:

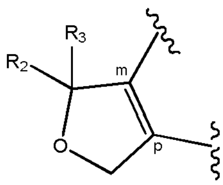, or 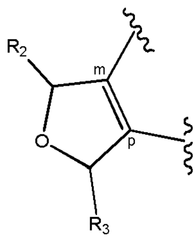

wherein

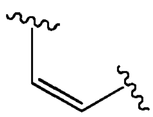

denotes a point at which ring A is fused to the phenyl ring.

In a further embodiment of the invention, ring A is:

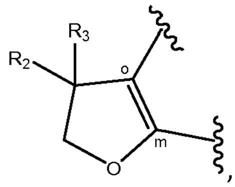

wherein

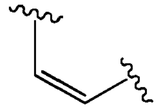

denotes a point at which ring A is fused to the phenyl ring.

When A is a 5 membered heterocycle containing one oxygen atom, suitably the heterocycle is dihydrofuran.

When A is a 5 membered heterocycle containing one oxygen atom, suitably the oxygen atom is located at the benzylic position relative to the phenyl ring. In one embodiment, when ring A is a 5 membered heterocycle containing one heteroatom which is oxygen, suitably the oxygen atom is located at the phenolic position relative to the phenyl ring.

When W is group (Wa), suitably A is a 5 membered heterocycle containing one heteroatom, wherein the oxygen atom is located at the benzylic or para position relative to the phenyl ring.

When W is group (Wb), suitably A is a 5 membered heterocycle containing one heteroatom, wherein the oxygen atom is located at the benzylic or meta position relative to the phenyl ring.

When W is group (Wa), in one embodiment of the invention, group (Wa) is:

**11**

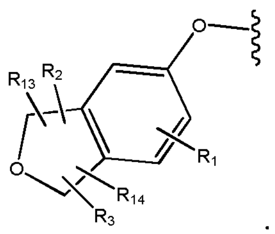

When W is group (Wa), in another embodiment of the invention, (Wa) is:

**12**

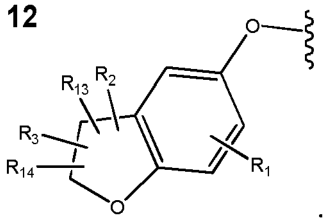

When W is group (Wb), in one embodiment of the invention, (Wb) is:

**13**

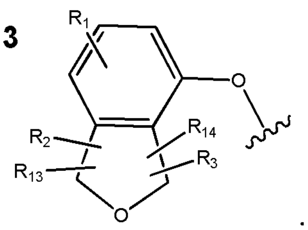

When W is group (Wb), in another embodiment of the invention, (Wb) is:

14

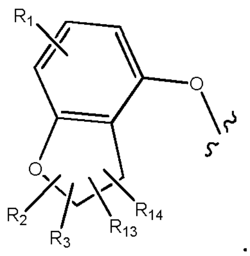

When W is group (Wb) in a further embodiment of the invention, (Wb) is:

15

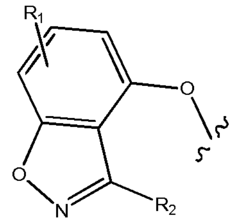

When A contains a 6 membered heterocycle containing one oxygen atom, suitably the heterocycle is dihydropyran.

When W is group (Wa), suitably A is a 6 membered heterocycle containing one oxygen atom, wherein the oxygen atom is located at the para position relative to the phenyl ring.

When W is group (Wb), suitably A contains a 6 membered heterocycle containing one oxygen atom, wherein the oxygen atom is located at the meta position relative to the phenyl ring.

When W is group (Wa), in one embodiment of the invention, (Wa) is:

16

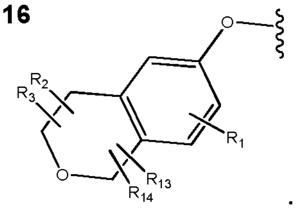

When W is group (Wa), in another embodiment of the invention, (Wa) is:

17

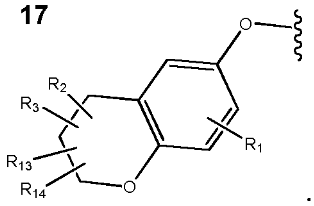

When W is group (Wb), in one embodiment of the invention, (Wb) is:

18

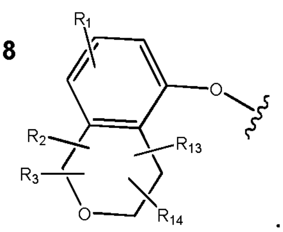

When W is group (Wb), in one embodiment of the invention, (Wb) is:

**19**

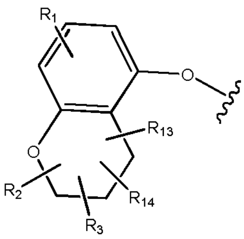

.

When W is group (Wb), in one embodiment of the invention, (Wb) is:

**20**

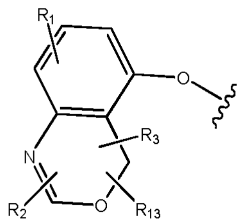

When W is group (Wa), in one embodiment of the invention, A is:

**1**

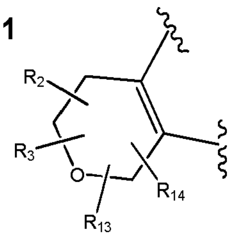

,

**2**

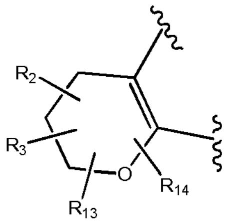

,

**3**

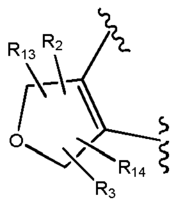

, or

**4**

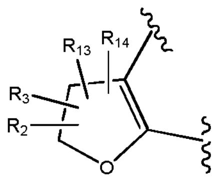

.

When W is group (Wa), in one embodiment of the invention, A is:

**21**

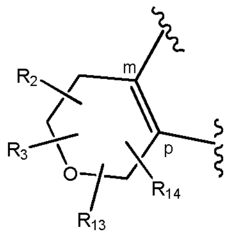

,

**22**

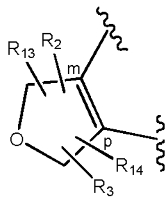

, or

**23**

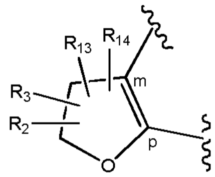

;

wherein m and p denote the meta and para positions, respectively, of ring A relative to the phenyl ring.

When W is group (Wa), in a further embodiment of the invention, A is selected from the group consisting of:

**24**

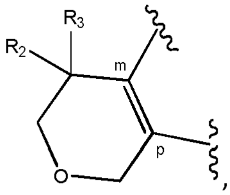

,

**25**

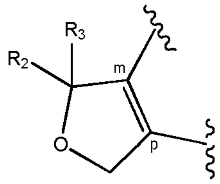

,

**26**

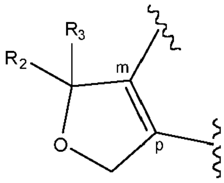

, and

27
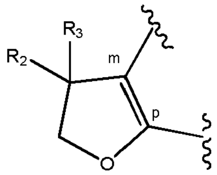

wherein m and p denote the meta and para positions, respectively, of ring A relative to the phenyl ring.

When W is group (Wb), in one embodiment of the invention, A is:

1
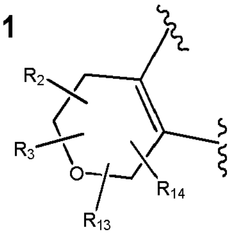
,
2
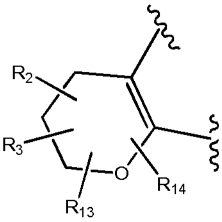
,
3
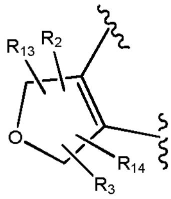
,
4
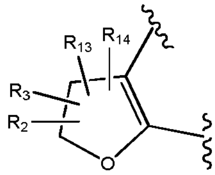
,

5
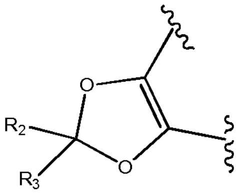
,
7
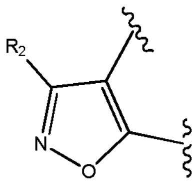
or
10
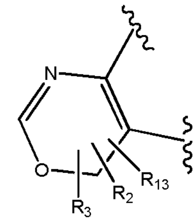
.

When W is group (Wb), in one embodiment of the invention, A is:

28
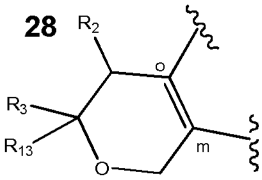
,
29
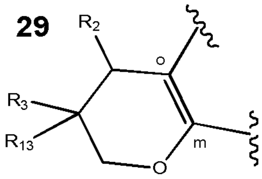
,
30
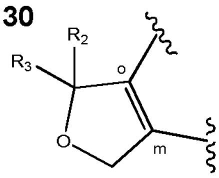
,
31
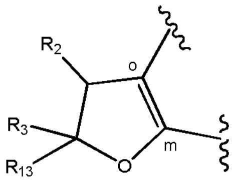
,

5
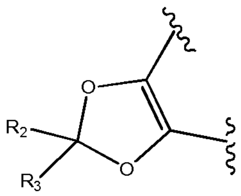
,
32
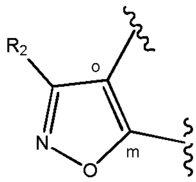
, or
33
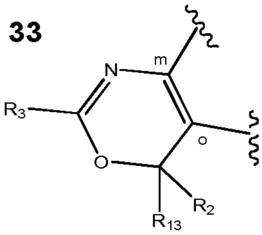
.

When W is group (Wb), in one embodiment of the invention, A is:

1
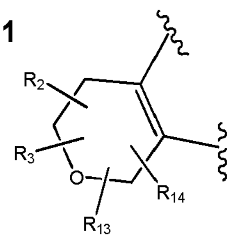
,
2
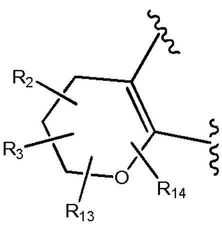
,
3
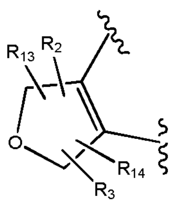
, or
4
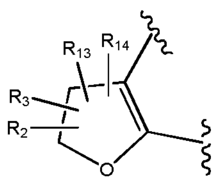
.

When W is group (Wb), in another embodiment of the invention, A is:

34 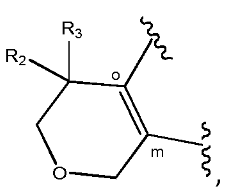 35 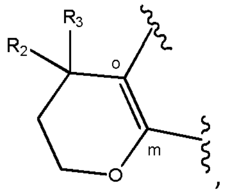 30 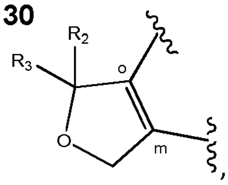 32 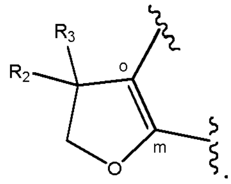

Suitably, $R_4$ is methyl, ethyl, isopropyl or t-butyl. In one embodiment of the invention $R_4$ is methyl. In another embodiment of the invention $R_4$ is ethyl. In a further embodiment of the invention $R_4$ is propyl, such is isopropyl. In a yet further embodiment of the invention $R_4$ is butyl, such as t-butyl.

Suitably, $R_5$ is H or methyl. In one embodiment of the invention $R_5$ is H. In a second embodiment of the invention $R_5$ is $C_{1-4}$alkyl, in particular $R_5$ is methyl.

In one embodiment of the invention $R_4$ and $R_5$ together form a $C_3$ spiro carbocycle. In a second embodiment of the invention $R_4$ and $R_5$ together form a $C_4$ spiro carbocycle. In a further embodiment of the invention $R_4$ is methyl and $R_5$ is methyl. In an embodiment of particular interest, $R_4$ is ethyl and $R_5$ is methyl. In another embodiment, $R_4$ is ethyl and $R_5$ is ethyl. In an additional embodiment, $R_4$ is ethyl and $R_5$ is H.

Suitably, $R_4$ and $R_5$ have the stereochemical arrangement:

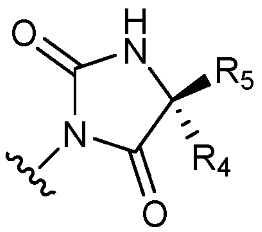

Suitably, the compound of formula (I) contains a (Wa) group corresponding to one of the following phenol groups:

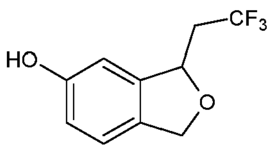 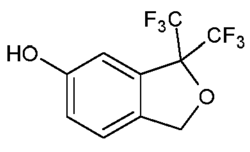 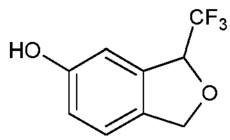 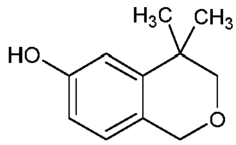

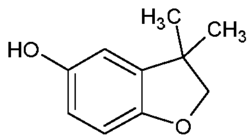 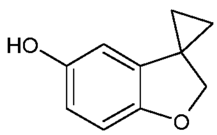 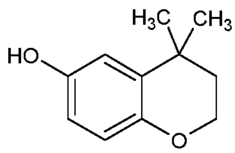

Suitably, the compound of formula (I) contains a (Wb) group corresponding to one of the following phenol groups:

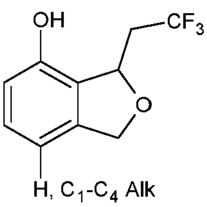 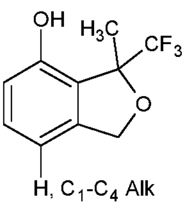 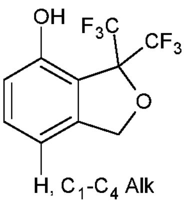 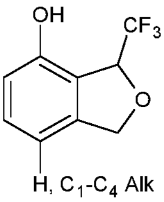

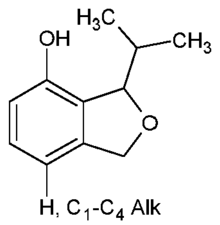

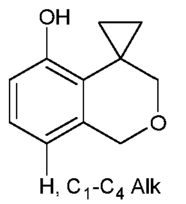

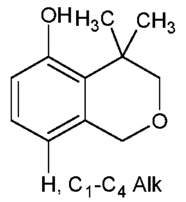

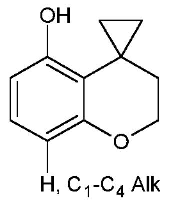

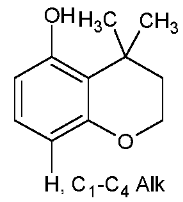

Alternatively, when the compound of formula (I) contains a (Wb) group corresponding to one of the following phenol groups:

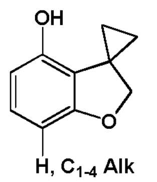

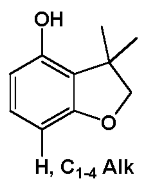

In one embodiment W is the group Wc.

When W is group (Wc), in one embodiment of the invention $R_{16}$ is $C_{1-4}$alkoxy. In another embodiment of the invention $R_{16}$ is methoxy. In one embodiment of the invention $R_{16}$ is $C_{1-4}$alkyl. In another embodiment of the invention $R_{16}$ is methyl. In a further embodiment of the invention $R_{16}$ is ethyl. In a yet further embodiment of the invention $R_{16}$ is propyl. In a yet further embodiment of the invention $R_{16}$ is butyl. In one embodiment of the invention $R_{16}$ is halo. In another embodiment of the invention $R_{16}$ is chloro. In a further embodiment of the invention $R_{16}$ is fluoro. In one embodiment of the invention $R_{16}$ is halo-$C_{1-4}$alkoxy. In another embodiment of the invention $R_{16}$ is trifluoromethoxy. In one embodiment of the invention $R_{16}$ is halo-$C_{1-4}$alkyl. In another embodiment of the invention $R_{16}$ is trifluoromethyl. In one embodiment of the invention $R_{16}$ is cyano.

In one embodiment of the invention, $R_{16}$ is $C_{1-4}$alkyl, $C_{1-4}$alkoxy, halo$C_{1-4}$alkyl, halo$C_{1-4}$alkoxy or CN. In one embodiment, $R_{16}$ is $C_{1-4}$alkyl, $C_{1-4}$alkoxy, halo$C_{1-4}$alkyl or halo$C_{1-4}$alkoxy. In one embodiment of the invention, $R_{16}$ is $C_{1-4}$alkyl, $C_{1-4}$alkoxy or halo$C_{1-4}$alkoxy. In one embodiment of the invention, $R_{16}$ is halo, $C_{1-4}$alkyl or $C_{1-4}$alkoxy. In one embodiment of the invention, $R_{16}$ is methyl, ethyl, propyl, butyl, cyclopropyl, chloro, fluoro, methoxy, ethoxy, propoxy, trifluoromethyl, trifluoromethoxy or CN.

In one embodiment of the invention, $R_{17}$ is H. In one embodiment of the invention $R_{17}$ is $C_{1-4}$alkyl. In another embodiment of the invention $R_{17}$ is methyl. In one embodiment of the invention $R_{17}$ is halo. In another embodiment of the invention, $R_{17}$ is chloro. In a further embodiment of the invention $R_{17}$ is fluoro. In one embodiment of the invention $R_{17}$ is cyano.

In one embodiment of the invention, $R_{17}$ is H, halo, CN, $C_{1-4}$alkyl or $C_{1-4}$alkoxy. In one embodiment of the invention, $R_{17}$ is H, CN, $C_{1-4}$alkyl, $C_{1-4}$alkoxy or halo$C_{1-4}$alkoxy. In one embodiment of the invention, $R_{17}$ is $C_{1-4}$alkyl or $C_{1-4}$alkoxy. In one embodiment of the invention, $R_{17}$ is H, CN or $C_{1-4}$alkyl. In one embodiment of the invention, $R_{17}$ is H, CN or methyl. In one embodiment of the invention, $R_{17}$ is methyl, ethyl, propyl, butyl, cyclopropyl, chloro, fluoro, methoxy, ethoxy, propoxy, trifluoromethoxy or CN.

In one embodiment of the invention $R_{16}$ is $C_{1-4}$alkoxy and $R_{17}$ is $C_{1-4}$alkyl. In one embodiment of the invention $R_{16}$ is methoxy and $R_{17}$ is methyl. In one embodiment of the invention $R_{16}$ is $C_{1-4}$alkoxy in the meta position and $R_{17}$ is $C_{1-4}$alkyl in the para position. In another embodiment of the invention $R_{16}$ is methoxy in the meta position and $R_{17}$ is methyl in the para position. In a further embodiment of the invention $R_{16}$ is methoxy in the meta position, $R_{17}$ is methyl in the para position, $R_4$ is $C_{1-4}$alkyl, $R_5$ is H, $R_4$ is in the R configuration.

Suitably one of $R_{16}$ and $R_{17}$ is in the meta position and the remaining one of $R_{16}$ or $R_{17}$ is in the para position. Alternatively, one of $R_{16}$ and $R_{17}$ is in the meta position and the remaining $R_{16}$ or $R_{17}$ is in the ortho position.

In one embodiment of the invention, when W is group (Wc) and $R_{16}$ and/or $R_{17}$ are CN, then the $R_{16}$ and/or

$R_{17}$ which is(are) CN is(are) not at the para position of the phenyl ring.

In another embodiment of the invention the compound is selected from the group consisting of:

(5R)-5-ethyl-5-methyl-3-[5-(7-methylspiro[2H-benzofuran-3,1'-cyclopropane]-4-yl)oxy-2-pyridyl]imidazolidine-2,4-dione;
(5R)-3-[5-[(3,3-dimethyl-2H-benzofuran-4-yl)oxy]-2-pyridyl]-5-ethyl-5-methyl-imidazolidine-2,4-dione; 5,5-dimethyl-3-[5-(7-methylspiro[2H-benzofuran-3,1'-cyclopropane]-4-yl)oxy-2-pyridyl]imidazolidine-2,4-dione;
(5R)-5-ethyl-3-[5-(7-methylspiro[2H-benzofuran-3,1'-cyclopropane]-4-yloxy-2-pyridyl]imidazolidine-2,4-dione;
5,5-dimethyl-3-(5-spiro[2H-benzofuran-3,1'-cyclopropane]-4-yloxy-2-pyridyl)imidazolidine-2,4-dione; (5R)-5-ethyl-3-(5-spiro[2H-benzofuran-3,1'-cyclopropane]-4-yloxy-2-pyridyl)imidazolidine-2,4-dione;
or a pharmaceutically acceptable salt and/or solvate thereof and/or prodrug thereof.

For the avoidance of doubt, the embodiments of any one feature of the compounds of the invention may be combined with any embodiment of another feature of compounds of the invention to create a further embodiment.

The term 'halo' or 'halogen' as used herein, refers to a fluorine, chlorine, bromine or iodine atom. Particular examples of halo are fluorine and chlorine, especially fluorine.

When the compound contains a $C_{1-4}$alkyl group, whether alone or forming part of a larger group, e.g. $C_{1-4}$alkoxy, the alkyl group may be straight chain, branched, cyclic, or a combination thereof. Examples of $C_{1-4}$alkyl are methyl, ethyl, n-propyl, isopropyl, n-butyl, isobutyl, sec-butyl, tert-butyl, cyclopropyl and cyclobutyl. A particular group of exemplary $C_{1-4}$alkyl groups are methyl, ethyl, n-propyl, isopropyl, n-butyl, isobutyl, sec-butyl, tert-butyl. An example of $C_{1-4}$alkoxy is methoxy.

The term 'halo$C_{1-4}$alkyl' as used herein, includes straight chain, branched chain or cyclic alkyl groups containing 1 to 4 carbon atoms substituted by one or more halo atoms, for example fluoromethyl, difluoromethyl and trifluoromethyl. A particular group of exemplary halo$C_{1-4}$ alkyl include methyl and ethyl groups substituted with one to three halo atoms, in particular one to three fluoro atoms, such as trifluoromethyl or 2,2,2-trifluoroethyl.

The term 'halo$C_{1-4}$alkoxy' as used herein, includes straight chain, branched chain or cyclic alkoxy groups containing 1 to 4 carbon atoms substituted by one or more halo atoms, for example fluoromethoxy, difluoromethoxy and trifluoromethoxy. A particular group of exemplary halo$C_{1-4}$ alkyl include methoxy and ethoxy groups substituted with one to three halo atoms, in particular one to three fluoro atoms.

The term '5 or 6 membered saturated or unsaturated heterocycle, with at least one O atom' includes for example dihydrofuran, dihydropyran, furan, pyran, oxazole, isoxazole, oxazine, dioxine, morpholine or 1,3-dioxalane.

It will be appreciated that for use in medicine the salts of the compounds of formula (I) should be pharmaceutically acceptable. Suitable pharmaceutically acceptable salts will be apparent to those skilled in the art. Pharmaceutically acceptable salts include those described by Berge (1977). Such pharmaceutically acceptable salts include acid addition salts formed with inorganic acids e.g. hydrochloric, hydrobromic, sulphuric, nitric or phosphoric acid and organic acids e.g. succinic, maleic, acetic, fumaric, citric, tartaric, benzoic, p-toluenesulfonic, methanesulfonic or naphthalenesulfonic acid. Other salts e.g. oxalates or formates, may be used, for example in the isolation of compounds of formula (I) and are included within the scope of this invention.

Certain of the compounds of formula (I) may form acid addition salts with one or more equivalents of the acid. The present invention includes within its scope all possible stoichiometric and nonstoichiometric forms.

The compounds of formula (I) may be prepared in crystalline or non-crystalline form and, if crystalline, may optionally be solvated, e.g. as the hydrate. This invention includes within its scope stoichiometric solvates (e.g. hydrates) as well as compounds containing variable amounts of solvent (e.g. water).

It will be understood that the invention includes pharmaceutically acceptable derivatives of compounds of formula (I) and that these are included within the scope of the invention.

As used herein "pharmaceutically acceptable derivative" includes any pharmaceutically acceptable prodrug such as an ester or salt of such ester of a compound of formula (I) which, upon administration to the recipient is capable of providing (directly or indirectly) a compound of formula (I) or an active metabolite or residue thereof.

Suitably, a pharmaceutically acceptable prodrug is formed by functionalising the secondary nitrogen of the hydantoin, for example with a group "L" as illustrated below:

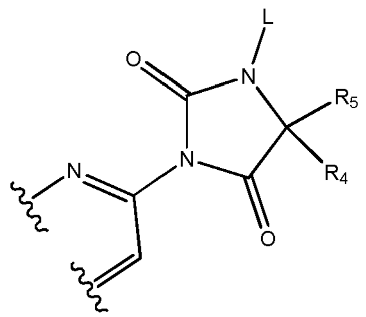

wherein $R_4$ and $R_5$ are as hereinabove defined.

In one embodiment of the invention, a compound of formula (I) is functionalised via the secondary nitrogen of the hydantoin with a group L, wherein L is selected from:

- $PO(OH)O^- \bullet M^+$, wherein $M^+$ is a pharmaceutically acceptable monovalent counterion,
- $PO(O^-)_2 \bullet M^+$,
- $PO(O^-)_2 \bullet D^{2+}$, wherein $D^{2+}$ is a pharmaceutically acceptable divalent counterion,
- $CH(R^X)-PO(OH)O^- \bullet M^+$, wherein $R^X$ is hydrogen or $C_{1-3}$ alkyl,
- $CH(R^X)-PO(O^-)_2 \bullet M^+$,
- $CH(R^X)-PO(O^-)_2 \bullet D^{2+}$
- $SO_3^- \bullet M^+$,
- $CH(R^X)-SO_3^- \bullet M^+$, and
- $CO-CH_2CH_2-CO_2 \bullet M^+$.

It is to be understood that the present invention encompasses all geometric isomers, tautomeric forms, optical forms, and mixtures thereof (e.g. racemic mixtures) of the compounds of formula (I). Where additional chiral centres are present in compounds of formula (I), the present invention includes within its scope all possible diastereoisomers, including mixtures thereof. The different isomeric forms may be separated or resolved one from the other by conventional methods, or any given isomer may be obtained by conventional synthetic methods or by stereospecific or asymmetric syntheses.

The subject invention also includes isotopically-labelled compounds which are identical to those recited in formula (I) but for the fact that one or more atoms are replaced by an atom having an atomic mass or mass number different from the atomic mass or mass number most commonly found in nature. The skilled person will appreciate that in many circumstances the proportion of an atom having an atomic mass or mass number found less commonly in nature can also be been increased (referred to as "isotopic enrichment"). Examples of isotopes that can be incorporated into compounds of the invention include isotopes of hydrogen, carbon, nitrogen, oxygen, fluorine, iodine and chlorine such as $^3H$, $^{11}C$, $^{14}C$, $^{18}F$, $^{123}I$ or $^{125}I$. Another isotope of interest is $^{13}C$. Another isotope of interest is $^2H$ (deuterium).

Compounds of the present invention and pharmaceutically acceptable salts of said compounds that contain the aforementioned isotopes and/or other isotopes of other atoms are within the scope of the present invention. Isotopically labelled compounds of the present invention, for example those into which radioactive isotopes such as $^3H$ or $^{14}C$ have been incorporated, are useful in drug and/or substrate tissue distribution assays. Tritiated, i.e. $^3H$, and carbon-14, i.e. $^{14}C$, isotopes are particularly preferred for their ease of preparation and detectability. $^{11}C$ and $^{18}F$ isotopes are particularly useful in PET (positron emission tomography).

Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

The following schemes detail synthetic routes to compounds of the invention. In the following schemes reactive groups can be protected with protecting groups and deprotected according to well established techniques.

In general, the compounds of formula (I) may be made according to the organic synthesis techniques known to those skilled in this field, as well as by the representative methods set forth below, those in the Examples and modifications thereof.

Patent applications WO2011/069951, WO2012/076877, WO2012/168710 and WO2013/175215 provide methods for the synthesis of intermediates which may be of use in the production of compounds of the present invention.

Scheme 1

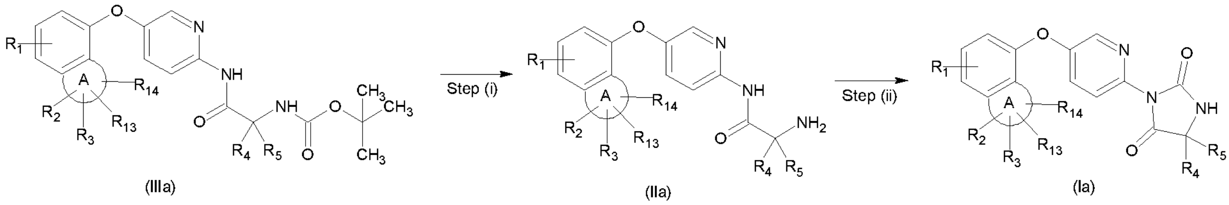


(IIIa) (IIa) (Ia)

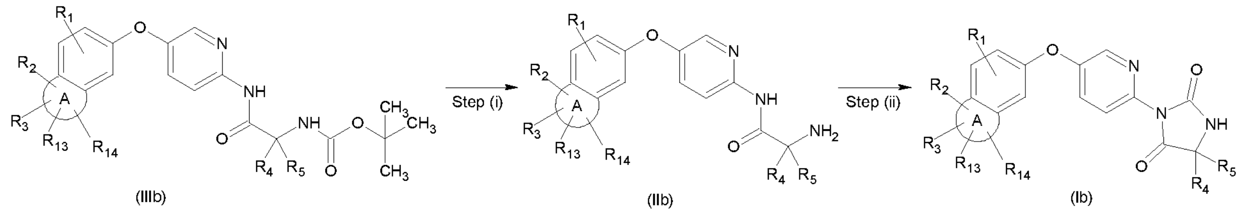


(IIIb) (IIb) (Ib)

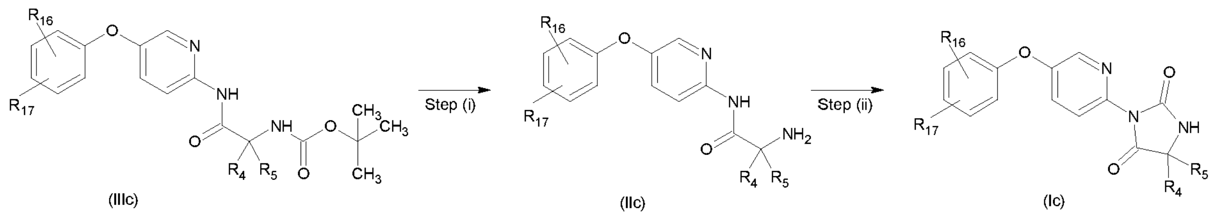


(IIIc) (IIc) (Ic)

**step (ii):** Compounds of formula (I) can be prepared by cyclization of compounds of formula (II) in a solvent *e.g.* dichloromethane with a carbonylating agent *e.g.* triphosgene preferentially prediluted in the same solvent and added in a second time at 0ºC in presence of a base e.g. triethylamine. In some cases, ethyl acetate could be used as a solvent. Optionally a catalytic amount of DMAP can be added.

**step (i):** Compounds of formula (II) can be prepared from compounds of formula (III) by removal of the BOC protective group in acidic conditions *e.g.* TFA in a solvent *e.g.* dichloromethane at 0ºC, RT.

Scheme 2

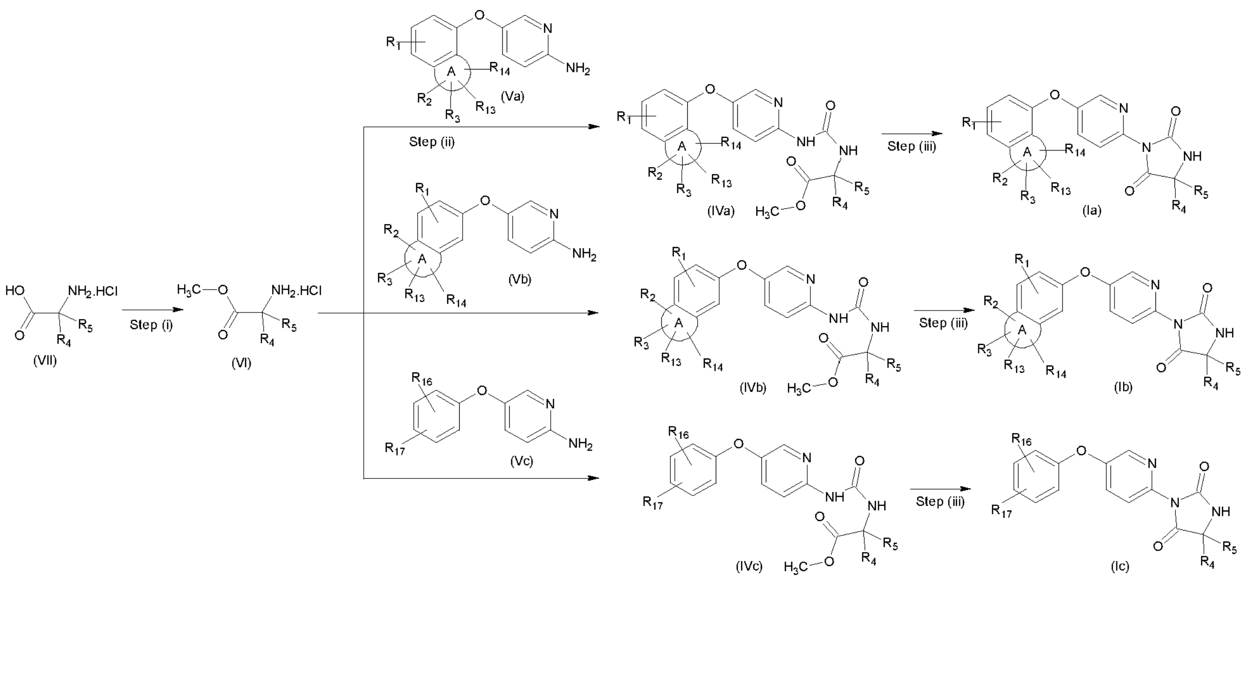


(VII) (VI) (Va) (Vb) (Vc) (IVa) (IVb) (IVc) (Ia) (Ib) (Ic)

**step** (iii): Compounds of formula (I) wherein $R_4$ and $R_5$ are not H can be prepared by reaction of a urea of formula (IV) and a base such as sodium methoxide in a solvent such as Methanol at temperature ranging from 0°C to 60°C.

**step** (ii): The urea of formula (IV) can be prepared by reaction of anilines of formula (V) and amino esters (hydrochloride salt) of formula (VI) in a suitable solvent, e.g. dichloromethane or ethyl acetate, with a carbonylating agent, e.g. triphosgene, preferentially prediluted in the same solvent in presence of a base, e.g. triethylamine or diisopropylethylamine, at temperature ranging from 0°C to 60°C, optionally adding a catalytic or stechiometric amount of DMAP.

**step** (i): Amino esters (hydrochloride salt) of formula (VI) (if not commercially available) can be prepared from commercially available aminoacids (hydrochloride salt) of formula (VII) by reaction with methanol in presence of a catalytic or stechiometric amount of thyonyl chloride at temperature ranging from r.t. to reflux.

Scheme 3

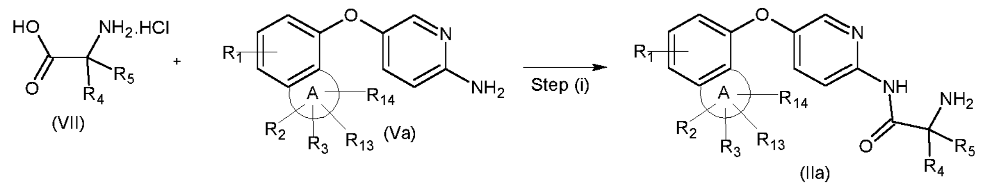

(VII) + (Va) → (IIa)

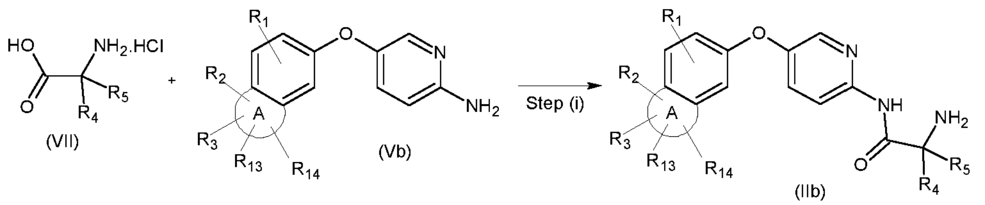

(VII) + (Vb) → (IIb)

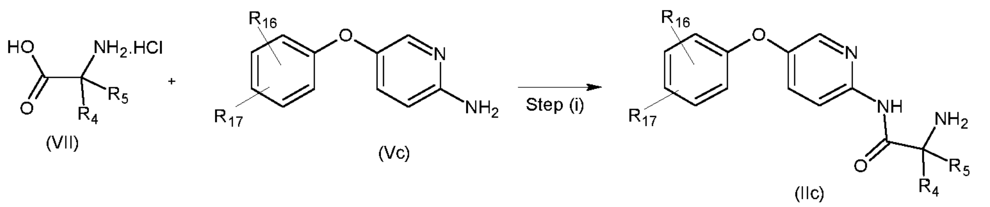

(VII) + (Vc) → (IIc)

**step (i):** Compounds of formula (II) can be prepared from anilines of formula (V) and amino acids hydrochloride salt of formula (VII) by amidic coupling in the presence of a coupling agent e.g. T3P in a solvent such as ethyl acetate, acetonitrile or a mixture of them at temperature ranging from room temperature to reflux.

Scheme 4

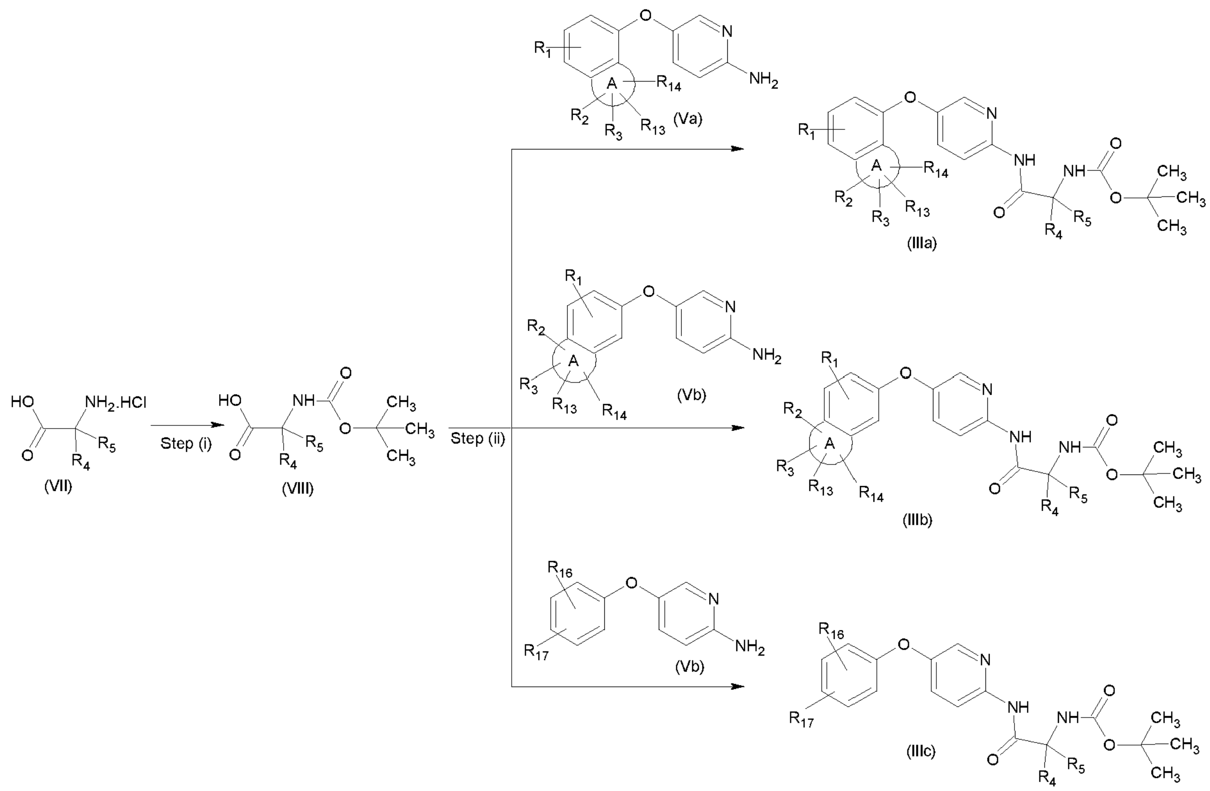


**step (ii):** Compounds of formula (III) can be prepared from anilines of formula (V) and N-protected amino acids of formula (VIII) by amidic coupling in the presence of a base e.g. DIPEA and of a coupling agent e.g. HATU, TBTU in a solvent such as N,N-dimethylformamide.

**step (i):** Some N-Boc protected amino acids of formula (V) are commercially available *e.g. N*-{[(1,1-dimethylethyl)oxy]carbonyl}-2-methylalanine from for example Aldrich, *N*-{[(1,1-dimethylethyl)oxy]carbonyl}-D-alanine from for example Aldrich, (2*R*)-2-({[(1,1-dimethylethyl)oxy]carbonyl}amino)butanoic acid from for example Bachem UK Ltd, *N*-{[(1,1-dimethylethyl)oxy]carbonyl}-D-isovaline from for example Nagase & Co Ltd.

N-protected amino acids of formula (V) can also be prepared from compounds of formula (VI) for example with Boc-anhydride in presence of a base e.g. aqueous $NaHCO_3$, aqueous sodium hydroxide in a solvent such as THF, methanol, dioxane. Many descriptions are available in the literature (for example Tetrahedron, 2006, 62(42), 9966 - 9972)

Scheme 5

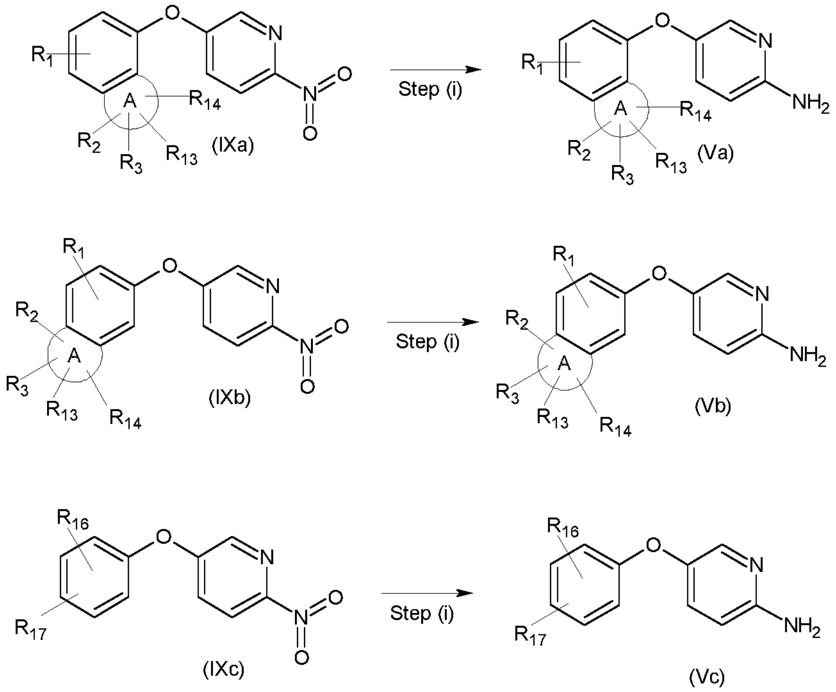


**step (i):** Anilines of formula (V) can be prepared from the nitro compounds (IX). Suitable reactions conditions to transform (IX) into (V) are for example:

- reduction in presence of Fe powder and ammonium chloride in a solvent such as a mixture THF/water for example at room temperature.

- reduction in presence of Fe powder and hydrochloric acid in a solvent such as a mixture of ethanol/water at temperature ranging from room temperature to reflux.

Scheme 6

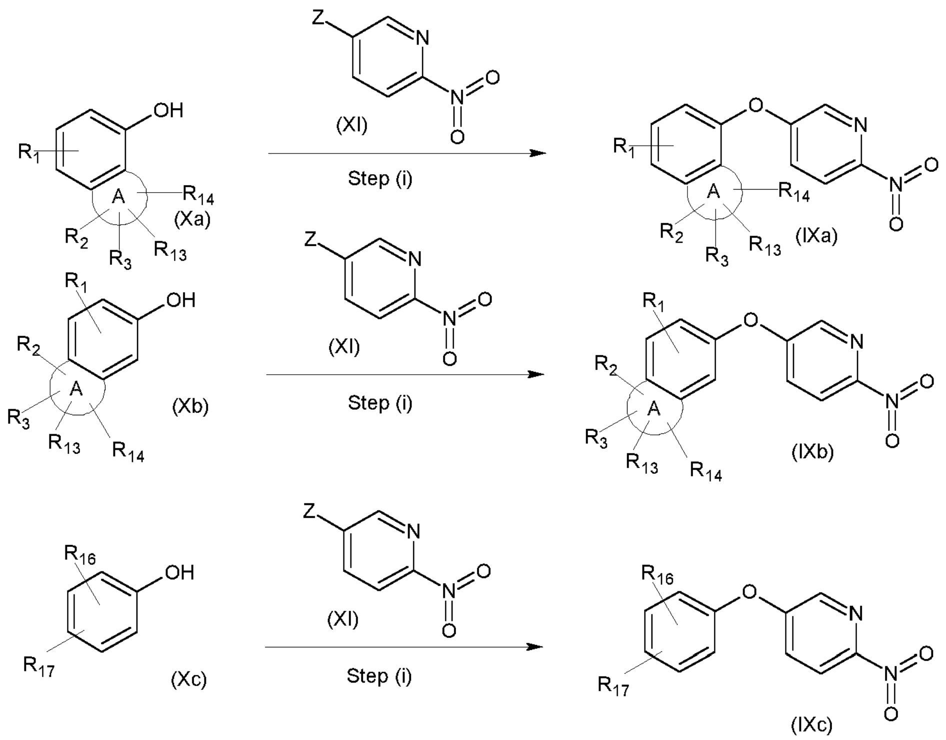


**step (i):** Compounds of formula (IX) can be prepared by nucleophilic aromatic substitution. In this reaction are used a commercially available nitro derivative of formula (XI) wherein Z=F or Z=Cl and a phenol of formula (X) in presence of a base such as potassium carbonate in a solvent e.g. in acetonitrile at temperature ranging from room temperature to reflux.

Scheme 7

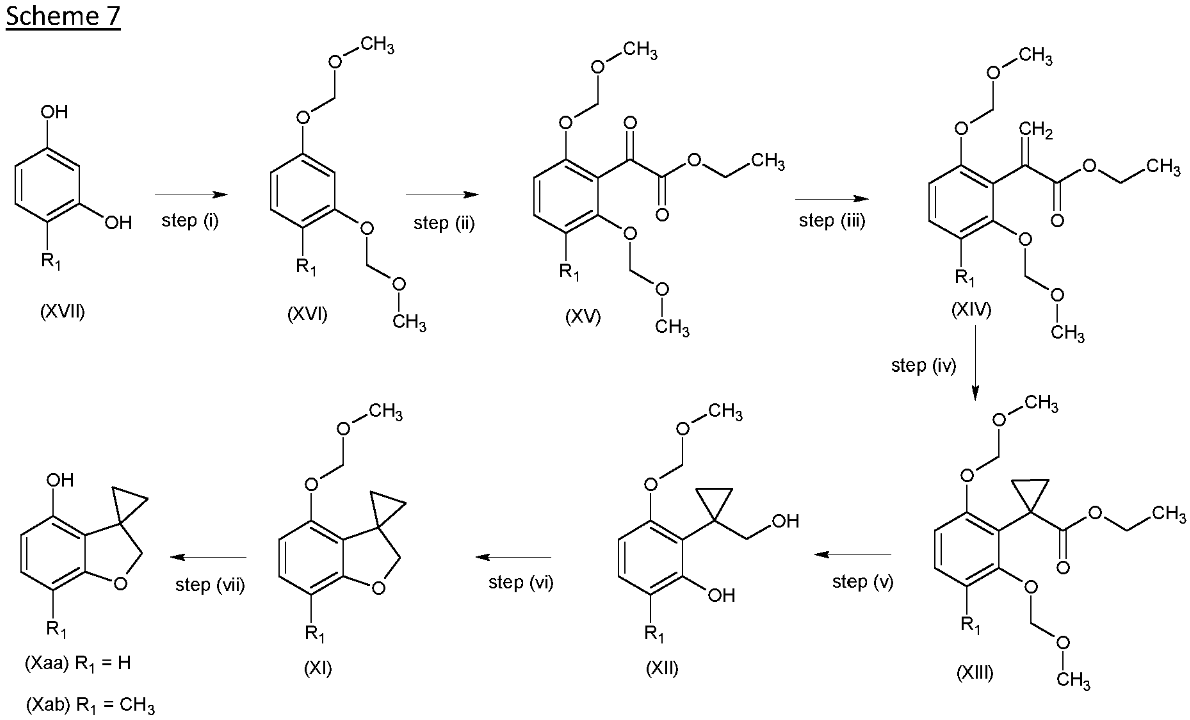


**step (vii):** Phenol of formula (Xaa) and (Xab) can be prepared from compound of formula (XI) by removal of the MOM protective group under acidic conditions using for example aqueous HCl in a solvent such as methanol heating

e.g. at 50°C.

**step (vi):** Compound of formula (XI) can be prepared from compound of formula (XII) by a Mitsonobu reaction using triphenylphosine in a solvent such as tetrahydrofuran and adding diisopropyl azodicarboxylate at temperature ranging from 0ºC to room temperature.

**step (v):** Compound of formula (XII) can be prepared from compound of formula (XIII) in a sequential mode - deprotection in acidic conditions such as HCl 2N in water in ethanol

- evaporation of the solvent, addition of MOMCl at 0°C and use of a strong base such as NaH in a solvent such as THF at 0°C
- reduction with lithium aluminium hydride at 0°C

**step (iv):** Compound of formula (XIII) can be prepared from compound of formula (XIV) using a Corey-Chaykovsky cyclopropanation reaction carried out at room temperature. To pre-form the dimethyloxosulfonium methylide, trimethylsulfoxonium iodide can be used in presence of a base such as NaH in a solvent such as DMSO, the compound of formula (XIV), (prediluted in DMSO) being added in a second time.

**step (iii)**: Compound of formula (XIV) can be prepared from compound of formula (XV) using a Wittig reaction. In order to pre-form the ylide, a phosphonium salt such as methyltriphenylphosphonium bromide and a strong base such as KHMDS can be used in a solvent such as THF from 0°C to room temperature. This solution can be added in a second time at 0°C to the compound of formula (XV) prediluted in a solvent such as THF.

**step (ii):** Compound of formula (XV) can be prepared from compound (XVI) by lithiation using BuLi in a solvent such as hexane or THF at temperature ranging from -78ºC to room temperature, this solution being added in a second time at -78°C to the electrophile *e.g.* ethyl chloro(oxo)acetate (prediluted *e.g.* in THF).

**step (i):** Compound of formula (XVI) can be prepared from commercially available phenols of formula (XVII) using MOM-Cl and a base such as NaH in a suitable solvent such as DMF at temperature ranging from 0°C to room temperature.

Scheme 8

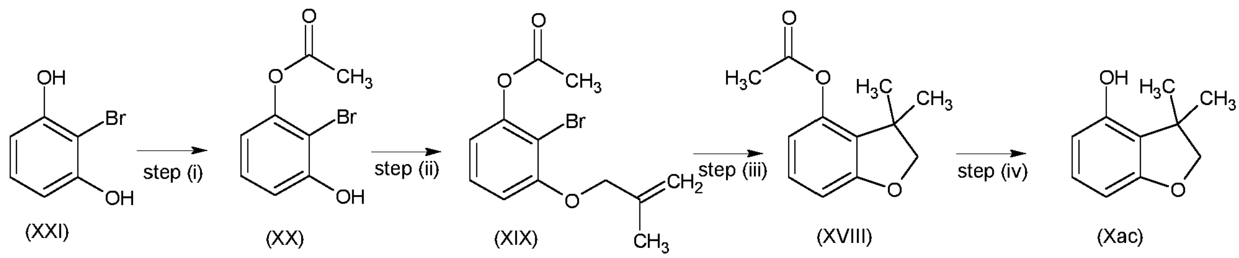


(XXI) (XX) (XIX) (XVIII) (Xac)

**step (iv):** phenol of formula (Xac) can be prepared from compound of formula (XVIII) using an hydroxide base for example sodium hydroxide, in a solvent such as a mixture of water and methanol, e.g. at room temperature.

**step (iii):** Compound of formula (XVIII) can be prepared from compound of formula (XIX) by cyclization using tributyltin hydride and AIBN in a suitable solvent such as toluene at a suitable temperature such as reflux.

**step (ii):** Compound of formula (XIX) can be prepared from compound of formula (XX) using a base such as potassium carbonate and an electrophile such as 3-bromo-2-methyl-1-propene, in a solvent such as acetonitrile e.g. at room temperature.

**step (i):** Compound of formula (XX) can be prepared from compound of formula (XXI) by acetylation using for example acetic anhydride, a base *e.g.* triethylamine in a solvent *e.g.* dichloromethane *e.g.* at room temperature.

Scheme 9

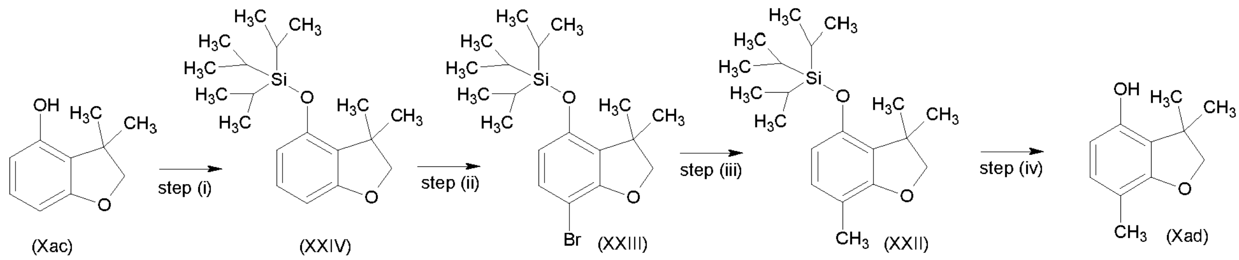


(Xac) (XXIV) (XXIII) (XXII) (Xad)

**Step (iv):** Phenol of formula Xad) can be prepared from TiPS protected compounds of formula (XXII) removing the protective group in presence of a Fluoride source such as tetrabutylammonium fluoride in a suitable solvent such as THF at room temperature.

**Step (iii):** compound of formula (XXII) can be prepared from compound of formula (XXIII) by metal-halogen exchange using butyllithium or sec- butyllithium or tert-butyllithium in a suitable solvent such as THF or Et2O or n-hexane at temperature ranging from -78°C to room temperature and adding in a second stage a methylating agent such as iodomethane at temperature ranging from -78°C to room temperature.

**Step (ii):** compound of formula (XXIII) can be prepared from a protected phenol of formula (XXIV) by using a brominating agent such as NBS in a suitable solvent such as DMF or acetonitrile or THF at room temperature.

**Step (i):** The compound of formula (XXIV) can be prepared from the starting phenol of formula (Xac) by silylation, using for example chloro triisopropylsilane in presence of a base such as butyllithium in a solvent such as THF at temperature ranging from 0°C and room temperature.

Scheme 10

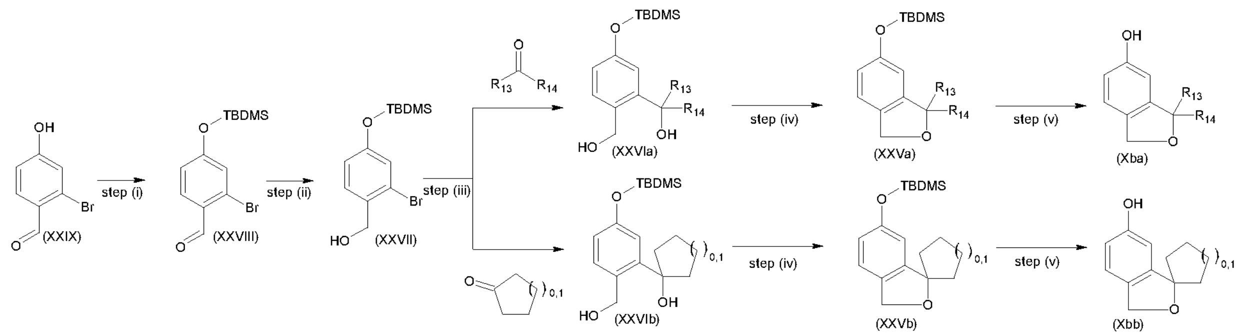


(XXIX) (XXVIII) (XXVII) (XXVIa) (XXVa) (Xba) (XXVIb) (XXVb) (Xbb)

**Step (v):** Phenol of formula (Xba) and (Xbb) can be prepared from TBDMS protected compounds of formula (XXVa) and (XXVb) removing the protective group in presence of a fluoride source such as tetrabutylammonium fluoride in a suitable solvent such as THF at temperature ranging from 0ºC to room temperature.

**step (iv):** Compounds of formula (XXVa) and (XXXVb) can be prepared by cyclisation of compounds of formula (XXVIa) and (XXXVIb) using a base such as nBuLi in a solvent such as THF e.g. at 0°C, adding in a second time 4-methylbenzenesulfonyl chloride e.g. at 0ºC, then a second equivalent of a base such as nBuLi e.g. from 0ºC to room temperature.

Optionally the two steps (iv) and (v) can be carried out in a one pot fashion.

**Step (iii):** compounds of formula (XXVIa) and (XXVIb) can be prepared from compounds of formula (XXVII) by metal-halogen exchange using 2 equivalents of butyllithium in a suitable solvent such as hexane or THF, or a mixture of them, at temperature ranging from -78°C to room temperature and adding in a second stage a suitable carbonylic compound (aldehyde or ketone) at temperature ranging from -78°C to room temperature.

**Step (ii):** Alcohols of formula (XXVII) can be prepared from aldehyde of formula (XXVIII) using a reductive reagent such as lithium aluminium hydride or lithium borohydride in a suitable solvent such as THF at 0ºC or sodium borohydride in a suitable solvent such as methanol at 0°C.

**step (i):** Compound of formula (XXVIII) can be prepared from compound of formula (XXIX) by silylation, using for example chloro(1,1-dimethylethyl)dimethylsilane, 1H imidazole in a solvent such as dichloromethane at room temperature.

A 'modulator' as used herein refers to a compound which is capable of producing at least 10% potentiation, and suitably at least 20% potentiation of whole-cell currents mediated by human Kv3.1 and/or human Kv3.2 and/or human Kv3.3 channels recombinantly expressed in mammalian cells.

Compounds of formula (I) of the present invention are modulators of Kv3.1. Compounds of formula (I) may also be modulators of Kv3.2 and/or Kv3.3. Compounds of the invention may be tested in the assay of Biological Example 1 to determine their modulatory properties for Kv3.1 and/or Kv3.2 and/or Kv3.3 channels.

The term 'Kv3.1, Kv3.2 and/or Kv3.3' shall be taken to mean the same as 'Kv3.1 and/or Kv3.2 and/or Kv3.3' and may also be referred to as 'Kv3.1/Kv3.2/Kv3.3'.

In one embodiment the modulator is capable of producing at least 10% potentiation and suitably at least 20% potentiation of whole-cell currents mediated by human Kv3.1 channels recombinantly expressed in mammalian cells. Suitably the pEC50 of the modulator is in the range of 4-7 (such as 5-6.5).

In one embodiment the modulator is capable of producing at least 10% potentiation and suitably at least 20% potentiation of whole-cell currents mediated by human Kv3.2 channels recombinantly expressed in mammalian cells. Suitably the pEC50 of the modulator is in the range of 4-7 (such as 5-6.5).

In one embodiment the modulator is capable of producing at least 10% potentiation and suitably at least 20% potentiation of whole-cell currents mediated by human Kv3.3 channels recombinantly expressed in mammalian cells. Suitably the pEC50 of the modulator is in the range of 4-7 (such as 5-6.5).

In another embodiment the modulator is capable of producing at least 10% potentiation and suitably at least 20% potentiation of whole-cell currents mediated by human Kv3.1 and Kv3.2 channels recombinantly expressed in mammalian cells.

In another embodiment the modulator is capable of producing at least 10% potentiation and suitably at least 20% potentiation of whole-cell currents mediated by human Kv3.1 and Kv3.3 channels recombinantly expressed in mammalian cells.

In another embodiment the modulator is capable of producing at least 10% potentiation and suitably at least 20% potentiation of whole-cell currents mediated by human Kv3.2 and Kv3.3 channels recombinantly expressed in mammalian cells.

In a further embodiment the modulator is capable of producing at least 10% potentiation and suitably at least 20% potentiation of whole-cell currents mediated by human Kv3.1, Kv3.2 and Kv3.3 channels recombinantly expressed in mammalian cells.

The present invention provides compounds of formula (I) or a pharmaceutically acceptable salt thereof for use in therapy.

The compounds of formula (I) or their pharmaceutically acceptable salts may be of use for the treatment or prophylaxis of a disease or disorder where a modulator of the Kv3.1 or Kv3.2 or Kv3.1 and Kv3.2 channels is required. As used herein, a modulator of Kv3.1 or Kv3.2 or Kv 3.1 and Kv3.2 is a compound which alters the properties of these channels, either positively or negatively. In a particular aspect of the invention, the compound of formula (I) is a positive modulator. Compounds of the invention may be tested in the assay of Biological Example 1 to determine their modulatory properties. In certain disorders it may be of benefit to utilise a modulator of Kv3.1 or Kv3.2 which demonstrates a particular selectivity profile between the two channels. For example, a compound may be selective for modulation of Kv3.1 channels over modulation of Kv3.2 channels demonstrating, for example, at least a 2 fold, 5 fold or 10 fold activity for Kv3.1 channels than for Kv3.2 channels. Alternatively, a compound may be selective for modulation of Kv3.2 channels over modulation of Kv3.1 channels demonstrating, for example, at least a 2 fold, 5 fold or 10 fold activity for Kv3.2 channels than for Kv3.1 channels. In other cases a compound may demonstrate comparable activity between modulation of Kv3.1 and Kv3.2 channels, for example the activity for each channel is less than 2 fold that for the other channel, such as less than 1.5 fold or less than 1.2 fold. The activity of a compound is suitably quantified by its potency as indicated by an EC50 value.

In certain disorders it may be of benefit to utilise a modulator of Kv3.3 or Kv3.1, or Kv3.3 and Kv3.1 which demonstrates a particular selectivity profile between the two channels. For example, a compound may be selective for modulation of Kv3.3 channels over modulation of Kv3.1 channels demonstrating, for example, at least a 2 fold, 5 fold or 10 fold activity for Kv3.3 channels than for Kv3.1 channels.

In another embodiment of the invention, the compounds of formula (I) or their pharmaceutically acceptable

salts and/or solvates and/or derivatives thereof are selective for modulation of Kv3.1 channels over modulation of Kv3.3 channels. Once again, by selective is meant that compounds demonstrate, for example at least a 2 fold, 5 fold or 10 fold activity for Kv3.1 channels than for Kv3.3 channels.

In a particular embodiment of the invention, a compound may demonstrate comparable activity between modulation of Kv3.3 and Kv3.1 channels, for example the activity for each channel is less than 2 fold that for the other channel, such as less than 1.5 fold or less than 1.2 fold.

In certain disorders it may be of benefit to utilise a modulator of Kv3.3 or Kv3.2, or Kv3.3 and Kv3.2 which demonstrates a particular selectivity profile between the two channels. A compound may be selective for modulation of Kv3.3 channels over modulation of Kv3.2 channels demonstrating, for example, at least a 2 fold, 5 fold or 10 fold activity for Kv3.3 channels than for Kv3.2 channels.

In another embodiment of the invention, the compounds of formula (I) or their pharmaceutically acceptable salts and/or solvates and/or derivatives thereof are selective for modulation of Kv3.2 channels over modulation of Kv3.3 channels. Once again, by selective is meant that compounds demonstrate, for example at least a 2 fold, 5 fold or 10 fold activity for Kv3.2 channels than for Kv3.3 channels.

In another particular embodiment a compound may demonstrate comparable activity between modulation of Kv3.3 and Kv3.2 channels, for example the activity for each channel is less than 2 fold that for the other channel, such as less than 1.5 fold or less than 1.2 fold.

In a yet further particular embodiment of the invention a compound may demonstrate comparable activity between modulation of Kv3.3, Kv3.2 and Kv3.1 channels, for example the activity for each channel is less than 2 fold that for any other channel, such as less than 1.5 fold or less than 1.2 fold. The activity of a compound is suitably quantified by its potency as indicated by an EC50 value.

Diseases or conditions that may be mediated by modulation of Kv3.1 and/or Kv3.2 channels may be selected from the list below. The numbers in brackets after the listed diseases below refer to the classification code in Diagnostic and Statistical Manual of Mental Disorders, 4th Edition, published by the American Psychiatric Association (DSM-IV) and/or the International Classification of Diseases, 10th Edition (ICD-10).

The compounds of formula (I) or their pharmaceutically acceptable salts and/or solvates and/or prodrugs may be of use for the treatment or prophylaxis of depression and mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90); Seasonal affective disorder.

The compounds of formula (I) or their pharmaceutically acceptable salts and/or solvates and/or prodrugs may be of use for the treatment or prophylaxis of schizophrenia including the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and With Hallucinations; Substance-Induced Psychotic Disorder including the subtypes With Delusions (293.81) and With Hallucinations (293.82); and Psychotic Disorder Not Otherwise Specified (298.9).

The compounds of formula (I) or their pharmaceutically acceptable salts and/or solvates and/or prodrugs may be of use for the treatment or prophylaxis of anxiety disorders including Panic Attack; Panic Disorder including Panic Disorder without Agoraphobia (300.01) and Panic Disorder with Agoraphobia (300.21); Agoraphobia; Agoraphobia Without History of Panic Disorder (300.22), Specific Phobia (300.29, formerly Simple Phobia) including the subtypes Animal Type, Natural Environment Type, Blood-Injection-Injury Type, Situational Type and Other Type), Social Phobia (Social Anxiety Disorder, 300.23), Obsessive-Compulsive Disorder (300.3), Posttraumatic Stress Disorder (309.81), Acute Stress Disorder (308.3), Generalized Anxiety Disorder (300.02), Anxiety Disorder Due to a General Medical Condition (293.84), Substance-Induced Anxiety Disorder, Separation Anxiety Disorder (309.21), Adjustment Disorders with Anxiety (309.24) and Anxiety Disorder Not Otherwise Specified (300.00). The compounds of formula (I) or their pharmaceutically acceptable salts and/or solvates and/or prodrugs may be of use for the treatment or prophylaxis of substance-related disorders including Substance Use Disorders such as Substance Dependence, Substance Craving and Substance Abuse; Substance-Induced Disorders such as Substance Intoxication, Substance Withdrawal, Substance-Induced Delirium, Substance-Induced Persisting Dementia, Substance-Induced Persisting Amnestic Disorder, Substance-Induced Psychotic Disorder, Substance-Induced Mood Disorder, Substance-Induced Anxiety Disorder, Substance-Induced Sexual Dysfunction, Substance-Induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium, Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89), Amphetamine Withdrawal (292.0), Amphetamine Intoxication Delirium, Amphetamine Induced Psychotic Disorder, Amphetamine-Induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse (305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-Like)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic-, or Anxiolytic-Persisting Dementia, Sedative-, Hypnotic-, or Anxiolytic- Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown) Substance-Related Disorders such as Anabolic Steroids, Nitrate Inhalants and Nitrous Oxide.

The compounds of formula (I) or their pharmaceutically acceptable salts and/or solvates and/or prodrugs may be of use for the enhancement of cognition including the treatment of cognition impairment in other diseases such as schizophrenia, bipolar disorder, depression, other psychiatric disorders and psychotic conditions associated with cognitive impairment, e.g. Alzheimer's disease. Alternatively, the compounds of formula (I) or their pharmaceutically acceptable salts and/or solvates may be of use for the prophylaxis of cognition impairment, such as may be associated with diseases such as schizophrenia, bipolar disorder, depression, other psychiatric disorders and psychotic conditions associated with cognitive impairment, e.g. Alzheimer's disease.

The compounds of formula (I) or their pharmaceutically acceptable salts and/or solvates (e.g. salts) and/or derivatives thereof may be of use for the treatment or prophylaxis of ataxia including ataxia, in particular spinocerebellar ataxia, especially ataxia associated with R420H, R423H or F448L mutations.

The compounds of formula (I) or their pharmaceutically acceptable salts and/or solvates and/or prodrugs may be of use for the treatment or prophylaxis of sleep disorders including primary sleep disorders such as Dyssomnias such as Primary Insomnia (307.42), Primary Hypersomnia (307.44), Narcolepsy (347), Breathing-Related Sleep Disorders (780.59), Circadian Rhythm Sleep Disorder (307.45) and Dyssomnia Not Otherwise Specified (307.47); primary sleep disorders such as Parasomnias such as Nightmare Disorder (307.47), Sleep Terror Disorder (307.46), Sleepwalking Disorder (307.46) and Parasomnia Not Otherwise Specified (307.47); Sleep Disorders Related to Another Mental Disorder

such as Insomnia Related to Another Mental Disorder (307.42) and Hypersomnia Related to Another Mental Disorder (307.44); Sleep Disorder Due to a General Medical Condition, in particular sleep disturbances associated with such diseases as neurological disorders, neuropathic pain, restless leg syndrome, heart and lung diseases; and Substance-Induced Sleep Disorder including the subtypes Insomnia Type, Hypersomnia Type, Parasomnia Type and Mixed Type; sleep apnea and jet-lag syndrome.

The compounds of formula (I) or their pharmaceutically acceptable salts and/or solvates and/or prodrugs may be of use for the treatment or prophylaxis of eating disorders such as Anorexia Nervosa (307.1) including the subtypes Restricting Type and Binge-Eating/Purging Type; Bulimia Nervosa (307.51) including the subtypes Purging Type and Nonpurging Type; Obesity; Compulsive Eating Disorder; Binge Eating Disorder; and Eating Disorder Not Otherwise Specified (307.50).

The compounds of formula (I) or their pharmaceutically acceptable salts may be of use for the treatment or prophylaxis of Autism Spectrum Disorders including Autistic Disorder (299.00), Asperger's Disorder (299.80), Rett's Disorder (299.80), Childhood Disintegrative Disorder (299.10) and Pervasive Disorder Not Otherwise Specified (299.80, including Atypical Autism).

The compounds of formula (I) or their pharmaceutically acceptable salts and/or solvates and/or prodrugs may be of use for the treatment or prophylaxis of Attention-Deficit/Hyperactivity Disorder including the subtypes Attention-Deficit /Hyperactivity Disorder Combined Type (314.01), Attention-Deficit /Hyperactivity Disorder Predominantly Inattentive Type (314.00), Attention-Deficit /Hyperactivity Disorder Hyperactive-Impulse Type (314.01) and Attention-Deficit /Hyperactivity Disorder Not Otherwise Specified (314.9); Hyperkinetic Disorder; Disruptive Behaviour Disorders such as Conduct Disorder including the subtypes childhood-onset type (321.81), Adolescent-Onset Type (312.82) and Unspecified Onset (312.89), Oppositional Defiant Disorder (313.81) and Disruptive Behaviour Disorder Not Otherwise Specified; and Tic Disorders such as Tourette's Disorder (307.23).

The compounds of formula (I) or their pharmaceutically acceptable salts and/or solvates and/or prodrugs may be of use for the treatment or prophylaxis of Personality Disorders including the subtypes Paranoid Personality Disorder (301.0), Schizoid Personality Disorder (301.20), Schizotypal Personality Disorder (301,22), Antisocial Personality Disorder (301.7), Borderline Personality Disorder (301,83), Histrionic Personality Disorder (301.50), Narcissistic Personality Disorder (301,81), Avoidant Personality Disorder (301.82), Dependent Personality Disorder (301.6), Obsessive-Compulsive Personality Disorder (301.4) and Personality Disorder Not Otherwise Specified (301.9).

The compounds of formula (I) or their pharmaceutically acceptable salts and/or solvates and/or prodrugs may be of use for the treatment or prophylaxis of Sexual dysfunctions including Sexual Desire Disorders such as Hypoactive Sexual Desire Disorder (302.71), and Sexual Aversion Disorder (302.79); sexual arousal disorders such as Female Sexual Arousal Disorder (302.72) and Male Erectile Disorder (302.72); orgasmic disorders such as Female Orgasmic Disorder (302.73), Male Orgasmic Disorder (302.74) and Premature Ejaculation (302.75); sexual pain disorder such as Dyspareunia (302.76) and Vaginismus (306.51); Sexual Dysfunction Not Otherwise Specified (302.70); paraphilias such as Exhibitionism (302.4), Fetishism (302.81), Frotteurism (302.89), Pedophilia (302.2), Sexual Masochism (302.83), Sexual Sadism (302.84), Transvestic Fetishism (302.3), Voyeurism (302.82) and Paraphilia Not Otherwise Specified (302.9); gender identity disorders such as Gender Identity Disorder in Children (302.6) and Gender Identity Disorder in Adolescents or Adults (302.85); and Sexual Disorder Not Otherwise Specified (302.9).

The compounds of formula (I) or their pharmaceutically acceptable salts and/or solvates and/or prodrugs may be of use for the treatment or prophylaxis of Impulse control disorder including: Intermittent Explosive Disorder (312.34), Kleptomania (312.32), Pathological Gambling (312.31), Pyromania (312.33), Trichotillomania (312.39), Impulse-Control Disorders Not Otherwise Specified (312.3), Binge Eating, Compulsive Buying, Compulsive Sexual Behaviour and Compulsive Hoarding.

The compounds of formula (I) or their pharmaceutically acceptable salts and/or solvates and/or prodrugs may be of use for the treatment or prophylaxis of hearing disorders including auditory neuropathy, auditory processing disorder, hearing loss, which includes sudden hearing loss, noise induced hearing loss, substance-induced hearing loss, and hearing loss in adults over 60 (presbycusis), and tinnitus.

The compounds of formula (I) or their pharmaceutically acceptable salts and/or solvates and/or prodrugs may be of use for the treatment or prophylaxis of Ménière's disease, disorders of balance, and disorders of the inner ear.

The compounds of formula (I) or their pharmaceutically acceptable salts and/or solvates and/or prodrugs may be of use for the treatment or prophylaxis of hyperacusis and disturbances of loudness perception, including Fragile-X syndrome and autism.

The compounds of formula (I) or their pharmaceutically acceptable salts and/or solvates and/or prodrugs may be of use for the treatment or prophylaxis of Epilepsy, (including, but not limited to, localization-related epilepsies, generalized epilepsies, epilepsies with both generalized and local seizures, and the like), seizures associated with Lennox-Gastaut syndrome, seizures as a complication of a disease or condition (such as seizures associated with encephalopathy, phenylketonuria, juvenile Gaucher's disease, Lundborg's progressive myoclonic epilepsy, stroke, head trauma, stress, hormonal changes, drug use or withdrawal, alcohol use or withdrawal, sleep deprivation, fever, infection,

and the like), essential tremor, restless limb syndrome, partial and generalised seizures (including tonic, clonic, tonic-clonic, atonic, myoclonic, absence seizures), secondarily generalized seizures, temporal lobe epilepsy, absence epilepsies (including childhood, juvenile, myoclonic, photo- and pattern-induced), severe epileptic encephalopathies (including hypoxia-related and Rasmussen's syndrome), febrile convulsions, epilepsy partialis continua, progressive myoclonus epilepsies (including Unverricht-Lundborg disease and Lafora's disease), post-traumatic seizures/epilepsy including those related to head injury, simple reflex epilepsies (including photosensive, somatosensory and proprioceptive, audiogenic and vestibular), metabolic disorders commonly associated with epilepsy such as pyridoxine-dependent epilepsy, Menkes' kinky hair disease, Krabbe's disease, epilepsy due to alcohol and drug abuse (e.g. cocaine), cortical malformations associated with epilepsy (e.g. double cortex syndrome or subcortical band heterotopia), chromosomal anomolies associated with seizures or epilepsy such as Partial monosomy (15Q) / Angelman syndrome.

The compounds of formula (I) or their pharmaceutically acceptable salts and/or solvates (e.g. salts) and/or derivatives thereof may be of use for the treatment or prophylaxis of pain including nociceptive, neuropathic, inflammatory or miscellaneous pain.

Nociceptive pain represents the normal response to noxious insult or injury of tissues such as skin, muscles, visceral organs, joints, tendons, or bones. Examples of nociceptive pain which form part of the invention include somatic pain: musculoskeletal (joint pain, myofascial pain) or cutaneous, which is often well localized; or visceral pain: hollow organs or smooth muscle.

Neuropathic pain is pain initiated or caused by a primary lesion or disease in the somatosensory nervous system. Sensory abnormalities range from deficits perceived as paraesthesia (numbness) to hypersensitivity (hyperalgesia or allodynia), and dysaesthesia (tingling and other sensations). Examples of neuropathic pain which form part of the invention include, but are not limited to, diabetic neuropathy, post-herpetic neuralgia, spinal cord injury pain, phantom limb (post-amputation) pain, and post-stroke central pain. Other causes of neuropathic pain include trauma, chemotherapy and heavy metal exposure.

The neuropathic pain that may be ameliorated by a modulator of Kv3.1 and/or Kv3.2 and/or Kv3.3 channels may be central or peripheral neuropathic pain. Central neuropathic pain is caused by damage to or dysfunction of the central nervous system (CNS), which includes but is not limited to the brain, brainstem, and spinal cord. Peripheral neuropathic pain is caused by damage to or dysfunction of the peripheral nervous system, which includes but is not limited to sensory nerves, motor nerves and autonomic nerves. In one embodiment, the neuropathic pain is central neuropathic pain. In another embodiment, the neuropathic pain is peripheral neuropathic pain.

Inflammatory pain occurs as a result of activation and sensitization of the nociceptive pain pathway by a variety of mediators released at a site of tissue inflammation. Mediators that have been implicated as key players in inflammatory pain are pro-inflammatory cytokines such IL-1-alpha, IL-1-beta, IL-6 and TNF-alpha, chemokines, reactive oxygen species, vasoactive amines, lipids, ATP, acid, and other factors released by infiltrating leukocytes, vascular endothelial cells, or tissue resident mast cells. Examples causes of inflammatory pain which form part of the invention include appendicitis, rheumatoid arthritis, inflammatory bowel disease, and herpes zoster.

Miscellaneous pain refers to pain conditions or disorders which are not easily classifiable. The current understanding of their underlying mechanisms is still rudimentary though specific therapies for those disorders are well known; they include cancer pain, migraine and other primary headaches and widespread pain of the fibromyalgia type.

Suitably, specific pain indications that may be mediated by a modulator of Kv3.1 and/or Kv3.2 and/or Kv3.3 channels are neuropathic pain and/or inflammatory pain.

Pain is a subjective condition and in a clinical setting tends to be measured by a patient's selfassessment. Therefore, it can be difficult to measure and quantify pain threshold. For chronic pain, typically a subjective 11-point rating scale is used where 0 is no pain and 10 is the worst pain imaginable. Subjects generally record their worst pain over a given period, usually a day. A minimum mean baseline score is also recorded and response to the medication is measured relative to the baseline, for example, a reduction of at least 10%, 20%, 30%, 40% or 50% in pain from the baseline score may be observed.

Since individual responses to medicaments may vary, not all individuals may experience a reduction in pain from the baseline score. Consequently, suitably a reduction is observed in at least at least 10%, 20%, 30%, 40%, 50%, 60%, 70%, 80%, 90% or all individuals tested.

Therefore, in one embodiment of the invention, a reduction of at least 10%, 20%, 30%, 40% or 50% in pain from the baseline score is observed upon administration of a Kv3.1/Kv3.2/Kv3.3 modulator, such as a compound of formula (I) or a pharmaceutically acceptable salt, solvate and/or derivative thereof to a subject in need thereof.

Administration of a Kv3.1/Kv3.2/Kv3.3 modulator can occur before an anticipated onset of pain or after the onset of pain. In cases where it is anticipated that development of a disease or disorder may lead to an increase in pain experienced by the subject, a Kv3.1/Kv3.2/Kv3.3 modulator, such as a compound of formula (I) or a pharmaceutically acceptable salt, solvate and/or derivative thereof can be administered. In cases where a subject is already experiencing pain, a Kv3.1/Kv3.2/Kv3.3 modulator, such as a compound of formula (I) or a pharmaceutically acceptable salt, solvate and/or derivative thereof may be administered to a subject in need thereof.

Treatment of the subject in need thereof may continue for as long as treatment is required, for example, 1 day, 1 week, 2 weeks, 3 weeks, 1 month, 6 months, 1 year, more than 1 year more than 2 years, more than 5 years or more than 10 years. Therefore in one embodiment of the invention, a therapeutically effective amount of a Kv3.1/Kv3.2/Kv3.3 modulator, such as a compound of formula (I) or a pharmaceutically acceptable salt, solvate and/or derivative thereof, is administered to a subject in need thereof for 1 day to 1 month, 1 week to 3 months, 1 month to 6 months, 3 months to 1 year or more than 1 year.

Reduction in pain in a subject can be measured by assessing the response to an external stimuli such as mechanical or thermal (e.g. cold) stimuli (such as described in the Experimental section). The reduction can either be considered as a percentage reversal (calculated by measuring the pre- and post-dose thresholds of the affected pain site with a non-affected pain site, such as described in more detail under Data Analysis in the Experimental Section) or by measuring withdrawal thresholds of the affected pain site. Preferably, the percentage reversal calculation is used.

Therefore, in one embodiment of the invention, the sensitivity to pain (such as neuropathic pain or inflammatory pain) is reversed by more than 20%, more than 30%, more than 40%, more than 50%, more than 60%, more than 70%, more than 80% or more than 90%, upon administration of a therapeutically effective amount of a Kv3.1/Kv3.2/Kv3.3 modulator, such as a compound of formula (I) or a pharmaceutically acceptable salt, solvate and/or derivative thereof. Suitably, the sensitivity to pain is reversed by more than 80% or more than 90%.

Subjects receiving the Kv3.1/Kv3.2/Kv3.3 modulator may experience secondary benefits, such as one or more of improved function, mood, sleep, quality of life, reduced time off work.

In a particular embodiment, the compounds of formula (I) or their pharmaceutically acceptable salts and/or solvates (e.g. salts) and/or derivatives thereof may be of use for the treatment or prophylaxis of neuropathic pain.

In a particular embodiment, the compounds of formula (I) or their pharmaceutically acceptable salts and/or solvates (e.g. salts) and/or derivatives thereof may be of use for the treatment or prophylaxis of inflammatory pain.

In a particular embodiment, the compounds of formula (I) or their pharmaceutically acceptable salts and/or solvates (e.g. salts) and/or derivatives thereof may be of use for the treatment or prophylaxis of miscellaneous pain.

In one embodiment is provided a compound of formula (I) for use in the prophylaxis of acute noise-induced hearing loss.

Acute noise-induced hearing loss may be caused by events such as exposure to loud noise or a blast. In these cases, where it is anticipated that a future event may result in acute noise-induced hearing loss, the compound of formula (I) or a pharmaceutically acceptable salt, solvate and/or derivative thereof may be administered before the event in order to prevent or reduce acute noise-induced hearing loss. The administration of compound (I) or a pharmaceutically acceptable salt, solvate and/or derivative thereof may prevent any acute noise-induced hearing loss, or may reduce the severity of the acute noise-induced hearing loss or may mitigate other symptoms arising from acute noise-induced hearing loss, such as tinnitus.

"Acute hearing loss" is defined as hearing loss which occurs rapidly over a period of hours or days. For example, hearing loss may occur over a period of minutes, hours or days (for example over a period of up to 1 day, such as up to 2 days, 3 days, 4 days, 5 days, 6 days or 7 days). Acute hearing loss will typically be caused by exposure to loud sound or blast. Hearing loss caused by exposure to loud sound or blast is referred to herein as "noise-induced induced hearing loss". "Acute noise induced hearing loss" is therefore hearing loss which occurs rapidly over a period of hours or days caused by exposure to loud sound or blast.

Important symptoms of acute hearing loss include:

1. a shift in the auditory threshold, i.e. an increase in the minimum sound level of a pure tone that can be heard with no other sound present;
2. tinnitus; and
3. degradation in central auditory processing, for example impaired auditory temporal processing and/or speech understanding.

A "loud" noise or blast may be at least 90dB, for example, at least 100dB, at least 110dB, at least 120 dB or at least 130 dB.

In one embodiment, administration of the compound of formula (I) or a pharmaceutically acceptable salt, solvate and/or derivative thereof is initiated before an event which is anticipated to cause noise-induced acute hearing loss. For example, administration of the compound of formula (I) or a pharmaceutically acceptable salt, solvate and/or derivative thereof may be initiated up to 2 weeks in advance, such as up to 1 week, 6 days, 5 days, 4 days, 3 days, 2 days, 24 h, 12 h, 6 h, 5 h, 4 h, 3 h, 2 h, 1 h, 30 minutes or up to 15 minutes in advance of an event which is anticipated to cause noise-induced acute hearing loss. The compound of formula (I) or a pharmaceutically acceptable salt, solvate and/or derivative thereof may be administered on multiple occasions before event which is anticipated to cause noise-induced acute hearing loss.

In one embodiment, a compound of formula (I) or a pharmaceutically acceptable salt, solvate and/or derivative

thereof is administered in advance of potential exposure to a noise or blast which is anticipated to cause acute noise-induced hearing loss, for preventing or reducing the development of permanent tinnitus; for preventing or reducing the development of a permanent shift in auditory thresholds; or for preventing or reducing the development of permanently degraded central auditory processing, including for example auditory temporal processing and/or speech understanding.

It will be appreciated that administration in advance may be in circumstances where the subject is considered to be at risk of exposure to a noise or blast which is anticipated to cause acute noise-induced hearing loss and is not limited to those circumstances where such exposure ultimately occurs.

In one embodiment, administration of the compound of formula (I) or a pharmaceutically acceptable salt, solvate and/or derivative thereof is initiated during an event which is anticipated to cause noise-induced acute hearing loss. The compound of formula (I) or a pharmaceutically acceptable salt, solvate and/or derivative thereof may be administered on multiple occasions during an event which is anticipated to cause noise-induced acute hearing loss.

In one embodiment, a compound of formula (I) or a pharmaceutically acceptable salt, solvate and/or derivative thereof is initially administered during a noise or blast which is anticipated to cause acute noise-induced hearing loss, for preventing or reducing the development of permanent tinnitus; for preventing or reducing the development of a permanent shift in the auditory threshold; or for preventing or reducing the development of permanently degraded central auditory processing, including for example auditory temporal processing and/or speech understanding.

In one embodiment, administration of the compound of formula (I) or a pharmaceutically acceptable salt, solvate and/or derivative thereof is initiated after an event which is anticipated to cause acute noise-induced hearing loss.

Thus, in one embodiment, a compound of formula (I) or a pharmaceutically acceptable salt, solvate and/or derivative thereof is initially administered after a noise or blast which is anticipated to cause acute noise-induced hearing loss, for preventing or reducing the development of permanent tinnitus; for preventing or reducing the development of a permanent shift in the auditory threshold; or for preventing or reducing the development of permanently degraded central auditory processing, including for example auditory temporal processing and/or speech understanding.

When the compound of formula (I) is administered after an event which is anticipated to cause acute noise-induced hearing loss, such administration is normally undertaken during the "acute phase" i.e. before the hearing loss has become established.

In one embodiment, administration of the compound of formula (I) or a pharmaceutically acceptable salt, solvate and/or derivative thereof may be initiated up to 2 months after an event which is anticipated to cause noise-induced acute hearing loss, such as up to 1 month, 2 weeks, 1 week, 6 days, 5 days, 4 days, 3 days, 2 days, 24 h, 12 h, 6 h, 5 h, 4 h, 3 h, 2 h, 1 h, 30 minutes or up to 15 minutes after an event which is anticipated to cause acute noise-induced hearing loss. The compound of formula (I) or a pharmaceutically acceptable salt, solvate and/or derivative thereof may be administered on multiple occasions after an event which is anticipated to cause noise-induced acute hearing loss.

The compound of formula (I) or a pharmaceutically acceptable salt, solvate and/or derivative thereof may be administered over a period of up to 7 days (for example, up to 1 day, up to 2 days, up to 3 days, up to 4 days, up to 5 days, up to 6 days or up to 7 days), for 1-2 weeks (for example, 7-8 days, 7-9 days, 7-10 days, 7-11 days, 7-12 days, 7-13 days or 7-14 days), for 2-4 weeks (for example, 2-3 weeks or 2-4 weeks) or for 1-2 months (for example, 4-6 weeks or 4-8 weeks).

The compound of formula (I) or a pharmaceutically acceptable salt, solvate and/or derivative thereof may initially be administered up to 1 day in advance, such as up to 2 days in advance, up to 3 days in advance, up to 5 days in advance, up to 1 week in advance, up to 2 weeks in advance or up to 1 month in advance of a noise or blast which is anticipated to cause acute noise-induced hearing loss, administration which is initiated at any point in advance exposure to a noise or blast which is anticipated to cause acute noise-induced hearing loss will typically continue for up to 2 months after exposure to the noise or blast which is anticipated to cause acute noise-induced hearing loss, such as for up to 1 month after, up to 3 weeks after, up to two weeks after, up to 1 week after, up to 5 days after, up to 3 days after, up to 2 days after, or up to 1 day after.

In one embodiment is provided a compound of formula (I) or a pharmaceutically acceptable salt, solvate and/or derivative thereof for use in preventing or reducing the development of a permanent shift in the auditory threshold, wherein the permanent shift in auditory threshold is reduced by at least 10dB, such as at least 15dB, at least 20dB, at least 30dB, at least 40dB, or completely.

Dementia with Lewy Bodies (DLB) and Parkinson’s disease (PD) are serious neurodegenerative disorders that are associated with the accumulation of the protein, alpha-synuclein in Lewy bodies, which leads to loss of connectivity and neuronal cell death. Symptoms of DLB include progressive cognitive deficits, in particular difficulties with planning and attention. Visual hallucinations are also common, occurring in approximately 60% of patients. PD is associated initially with motor deficits, primarily due to loss of dopamine neurons. While there are currently no studies directly linking Kv3 channels to DLB or PD, the location and role of Kv3 channels, in particular Kv3.1, in cortical and basal ganglia circuits suggets that modulators of these channels could improve symptoms of DLB or PD, either alone, or in combination with current treatments, such as acetyl-cholinesterase inhibitors for DLB or L-DOPA for PD.

In one embodiment of the invention, there is provided a compound of formula (I) or a pharmaceutically acceptable

salt thereof for the treatment or prophylaxis of depression and mood disorders, hearing disorders, schizophrenia, substance abuse disorders, sleep disorders or epilepsy.

In one embodiment of the invention, there is provided a compound of formula (I) or a pharmaceutically acceptable salt and/or solvate thereof and/or prodrug thereof for the treatment or prophylaxis of bipolar disorder or mania.

In one embodiment of the invention, there is provided a compound of formula (I) or a pharmaceutically acceptable salt and/or solvate thereof and/or prodrug thereof for the treatment or prophylaxis of ataxia, such as spinocerebellar ataxia.

In one embodiment of the invention, there is provided a compound of formula (I) or a pharmaceutically acceptable salt and/or solvate thereof and/or prodrug thereof for the treatment or prophylaxis of cognition impairment.

In one embodiment of the invention, there is provided a compound of formula (I) or a pharmaceutically acceptable salt and/or solvate thereof and/or prodrug thereof for the treatment or prophylaxis of hearing and hearing related disorders, including hearing loss or tinnitus.

The term "treatment" or "treating" as used herein includes the control, mitigation, reduction, or modulation of the disease state or its symptoms.

The term "prophylaxis" is used herein to mean preventing symptoms of a disease or disorder in a subject or preventing recurrence of symptoms of a disease or disorder in an afflicted subject and is not limited to complete prevention of an affliction.

The invention also provides a compound of formula (I), or a pharmaceutically acceptable salt and/or solvate and/or prodrug thereof, for use in the treatment or prophylaxis of a disease or disorder where a modulator of Kv3 is required, for example those diseases and disorders mentioned hereinabove.

In one embodiment, the compounds of formula (I) or their pharmaceutically acceptable salts and/or solvates and/or derivatives may be of use for the treatment or prophylaxis of a disease or disorder selected from the group consisting of hearing disorders, schizophrenia, depression and mood disorders, bipolar disorder, substance abuse disorders, anxiety disorders, sleep disorders, hyperacusis and disturbances of loudness perception, Ménière’s disease, disorders of balance, and disorders of the inner ear, impulse control disorder, personality disorders, attention-deficit/hyperactivity disorder, autism spectrum disorders, eating disorders, cognition impairment, ataxia, epilepsy, pain such as neuropathic pain, inflammatory pain and miscellaneous pain, Lewy body dementia and Parkinson’s disease.

In one embodiment of the invention, there is provided a compound of formula (I) or a pharmaceutically acceptable salt and/or solvate and/or prodrug thereof for use in the prophylaxis or treatment of schizophrenia.

In one embodiment of the invention, there is provided a compound of formula (I) or a pharmaceutically acceptable salt and/or solvate and/or prodrug thereof for use in the prophylaxis or treatment of pain.

In one embodiment of the invention, there is provided a compound of formula (I) or a pharmaceutically acceptable salt and/or solvate thereof and/or prodrug thereof for use in the prophylaxis or treatment of Fragile-X syndrome.

For use in therapy the compounds of the invention are usually administered as a pharmaceutical composition. The invention also provides a pharmaceutical composition comprising a compound of formula (I), or a pharmaceutically acceptable salt and/or solvate and/or prodrug thereof, and a pharmaceutically acceptable carrier.

The compounds of formula (I) or their pharmaceutically acceptable salts may be administered by any convenient method, e.g. by oral, parenteral, buccal, sublingual, nasal, rectal or transdermal administration, and the pharmaceutical compositions adapted accordingly. Other possible routes of administration include intratympanic and intracochlear.

The compounds of formula (I) or their pharmaceutically acceptable salts which are active when given orally can be formulated as liquids or solids, e.g. as syrups, suspensions, emulsions, tablets, capsules or lozenges.

A liquid formulation will generally consist of a suspension or solution of the active ingredient in a suitable liquid carrier(s) e.g. an aqueous solvent such as water, ethanol or glycerine, or a non-aqueous solvent, such as polyethylene glycol or an oil. The formulation may also contain a suspending agent, preservative, flavouring and/or colouring agent.

A composition in the form of a tablet can be prepared using any suitable pharmaceutical carrier(s) routinely used for preparing solid formulations, such as magnesium stearate, starch, lactose, sucrose and cellulose.

A composition in the form of a capsule can be prepared using routine encapsulation procedures, e.g. pellets containing the active ingredient can be prepared using standard carriers and then filled into a hard gelatin capsule; alternatively a dispersion or suspension can be prepared using any suitable pharmaceutical carrier(s), e.g. aqueous gums, celluloses, silicates or oils and the dispersion or suspension then filled into a soft gelatin capsule.

Typical parenteral compositions consist of a solution or suspension of the active ingredient in a sterile aqueous carrier or parenterally acceptable oil, e.g. polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active ingredient in a pharmaceutically acceptable aqueous or non-aqueous solvent and are usually presented in single or multidose quantities in sterile form in a sealed container which can take the form of a cartridge or refill for use with an atomising device. Alternatively, the sealed container may be a disposable dispensing device such as a single dose nasal inhaler or an aerosol dispenser fitted with a metering valve. Where the dosage form comprises an aerosol dispenser, it will contain a propellant which

can be a compressed gas e.g. air, or an organic propellant such as a fluorochlorohydrocarbon or hydrofluorocarbon. Aerosol dosage forms can also take the form of pump-atomisers.

Compositions suitable for buccal or sublingual administration include tablets, lozenges and pastilles where the active ingredient is formulated with a carrier such as sugar and acacia, tragacanth, or gelatin and glycerin. Compositions for rectal administration are conveniently in the form of suppositories containing a conventional suppository base such as cocoa butter. Compositions suitable for transdermal administration include ointments, gels and patches. In one embodiment the composition is in unit dose form such as a tablet, capsule or ampoule.

The composition may contain from 0.1% to 100% by weight, for example from 10 to 60% by weight, of the active material, depending on the method of administration. The composition may contain from 0% to 99% by weight, for example 40% to 90% by weight, of the carrier, depending on the method of administration. The composition may contain from 0.05mg to 1000mg, for example from 1.0mg to 500mg, of the active material, depending on the method of administration. The composition may contain from 50 mg to 1000 mg, for example from 100mg to 400mg of the carrier, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 500mg, and such unit doses may be administered more than once a day, for example two or three a day. Such therapy may extend for a number of weeks or months.

The invention provides, in a further aspect, a combination comprising a compound of formula (I) or a pharmaceutically acceptable salt, solvate and/or derivative thereof together with a further therapeutic agent or agents.

The invention provides a compound of formula (I), for use in combination with a further therapeutic agent or agents.

When the compounds are used in combination with other therapeutic agents, the compounds may be administered either sequentially or simultaneously by any convenient route.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations. The individual components of combinations may also be administered separately, through the same or different routes.

When a compound of formula (I) or a pharmaceutically acceptable derivative thereof is used in combination with a second therapeutic agent active against the same disease state the dose of each compound may differ from that when the compound is used alone. Appropriate doses will be readily appreciated by those skilled in the art.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Particular intermediates for producing the compounds of formula (I) include compounds of formula Va, Vb or Vc:

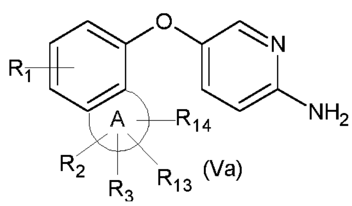

(Va)

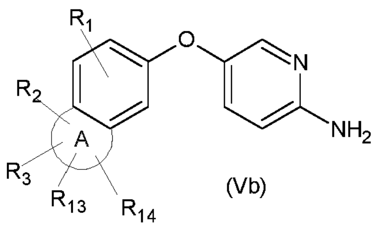

(Vb)

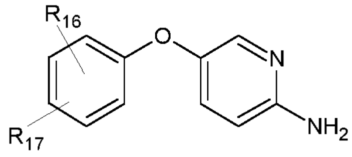

(Vc)

wherein $R_1$, $R_2$, $R_3$, $R_{13}$, $R_{14}$, $R_{16}$ and $R_{17}$ are as hereinabove defined. Including the specific compounds:

5-[(7-methylspiro[1-benzofuran-3,1'-cyclopropan]-4-yl)oxy]pyridin-2-amine

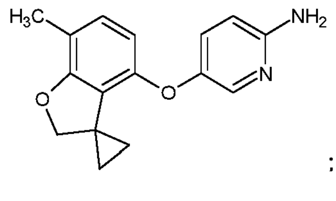

:

5-[(3,3-dimethyl-2,3-dihydro-1-benzofuran-4-yl)oxy]pyridin-2-amine

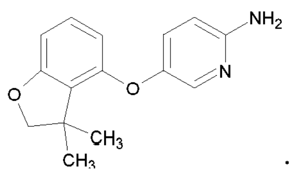

.

5-(spiro[1-benzofuran-3,1'-cyclopropan]-4-yloxy)pyridin-2-amine

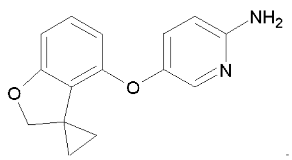

## Experimental

The invention is illustrated by the compounds described below. The following examples describe the laboratory synthesis of specific compounds of the invention and are not meant to limit the scope of the invention in any way with respect to compounds or processes. It is understood that, although specific reagents, solvents, temperatures and time periods are used, there are many possible equivalent alternatives that can be used to produce similar results.

## Analytical Equipment

Starting materials, reagents and solvents were obtained from commercial suppliers and used without further purification unless otherwise stated. Unless otherwise stated, all compounds with chiral centres are racemic. Where reactions are described as having been carried out in a similar manner to earlier, more completely described reactions, the general reaction conditions used were essentially the same. Work up conditions used were of the types standard in the art, but may have been adapted from one reaction to another. The starting material may not necessarily have been prepared from the batch referred to. Compounds synthesised may have various purities ranging from for example 85% to 98%. Calculations of number of moles and yield are in some cases adjusted for this.

Nuclear Magnetic Resonance (NMR) spectra ($^{1}H$; $^{13}C$ and $^{19}F$) were recorded either on Varian instruments at 300, 400, 500 or 600 MHz, or on Bruker instruments at 400 MHz. Chemical shifts are reported in ppm ($\delta$) using the residual solvent line as internal standard. Splitting patterns are designed as s (singlet), br.s (broad singlet), d (doublet), t (triplet), q (quartet), dd (doublet of doublets), dt (doublet of triplets) and m (multiplet). The NMR spectra were recorded at temperatures ranging from 25 to 30ºC.

Direct infusion Mass spectra (MS) were run on a mass spectrometer, operating in ES (+) and ES (-) ionization

mode coupled with an HPLC instrument Agilent 1100 Series [LC/MS-ESI(+) analyses were performed on a Supelcosil ABZ+Plus (33x4.6 mm, 3 um) (mobile phase: from 10%[$CH_3CN$+0.05%TFA] to 90 %[$CH_3CN$+0.05%TFA] and 10% [water] in 2.2 min, under these conditions for 2.8 min. T= 45 °C, flux = 0.9 mL/min)]. The use of this methodology is indicated by "MS_2 (ESI)" in the analytic characterization of the described compounds.

*Quality Control:* LC/MS-ES+ under acidic conditions was performed on a Zorbax SB C18 column (1.8 μm 3 x 50 mm). Mobile phase: **A:** (H2O + 0.05% TFA by vol.) / **B**: (CH3CN + 0.05% TFA by vol). Gradient: t = 0 min 0% (B), from 0 to 95% (B) in 2.5 min, 95% (B)for 0.2 min, from 95 to 100% (B) in 0.2 min, 100% (B) for 0.4 min, From 100% to 0% (B) in 0.1 min. Stop time 4 min. Column T = 60°C. Flow rate: 1.5 ml/min. Mass range ES+: (100-1000 amu, F=60 ). UV detection wavelengths : DAD 1A = 220.8, DAD 1B = 254.8. The use of this methodology is indicated by "LC/MS: QC_3_MIN" in the analytic characterization of the described compounds.

*Ultra Performance Liquid Chromatography with an acidic gradient:*

Total ion current (TIC) and DAD UV chromatographic traces together with MS and UV spectra associated with the peaks were taken on a UPLC/MS AcquityTM system equipped with 2996 PDA detector and coupled to a Waters Micromass ZQTM mass spectrometer operating in positive or negative electrospray ionisation mode [LC/MS - ES (+ or -): analyses were performed using an AcquityTM UPLC BEH C18 column (50 x 2.1 mm, 1.7 μm particle size). *General Method:* Mobile phase: **A**: (water + 0.1% HCO2H) / **B**: (CH3CN + 0.06% HCO2H). Gradient : t = 0 min 3% (B), t = 0.05 min 6% (B), t = 0.57 min 70% (B), t = 1.06 min 99% (B) lasting for 0.389 min, t = 1.45 min 3% (B), stop time 1.5 min. Column T = 40 ºC. Flow rate = 1.0 mL/min. Mass range: ES (+): 100-1000 amu. ES (-): 100-800 amu. UV detection range: 210-350 nm. The use of this methodology is indicated by "UPLC" in the analytic characterization of the described compounds.

*Ultra Performance Liquid Chromatography with a basic gradient:*

Total ion current (TIC) and DAD UV chromatographic traces together with MS and UV spectra associated with the peaks were taken on a UPLC/MS AcquityTM system equipped with PDA detector and coupled to a Waters SQD mass spectrometer operating in positive and negative alternate electrospray ionisation mode [LC/MS - ES+/-: analyses were performed using an AcquityTM UPLC BEH C18 column (50 x 2.1 mm, 1.7 μm particle size). Mobile phase: **A:** (10 mM aqueous solution of NH4HCO3 (adjusted to pH 10 with ammonia)) / **B:** CH3CN. Gradient: t = 0 min 3% (B), t = 1.06 min 99% (B) lasting for 0.39 min, t = 1.46 min 3% (B), stop time 1.5 min. Column T = 40 ºC. Flow rate = 1.0 mL/min. Mass range: ES (+): 100-1000 amu. ES (-): 100-1000 amu. UV detection range: 220-350 nm. The use of this methodology is indicated by "UPLC_B" in the analytic characterization of the described compounds.

In a number of preparations, purification was performed using Biotage automatic flash chromatography (SP1 and SP4) or Flash Master Personal systems.

Flash chromatographies were carried out on silica gel 230-400 mesh (supplied by Merck AG Darmstadt, Germany) or on silica gel 300-400 mesh (supplied by Sinopharm Chemical Reagent Co., Ltd.), Varian Mega Be-Si pre-packed cartridges, pre-packed Biotage silica cartridges (e.g. Biotage SNAP cartridge).

## Abbreviations

| | |
|---|---|
| AIBN | azobisisobutyronitrile |
| BuLi | butyllithium |
| $CDCl_3$ | deutrated chloroform |
| DCM | dichloromethane |
| DIAD | Diisopropyl azodicarboxylate |
| DIPEA | N,N-diisopropylethylamine |
| DMAP | 4-dimethylaminopyridine |
| DMF | N,N-dimethylformamide |
| DMSO | dimethylsulfoxide |
| DMSO-$d_6$ | deutrated dimethylsulfoxide |
| $Et_2O$ | diethyl ether |
| EtOAc | ethyl acetate |
| h | hours |
| HATU | (O-7-azabenzotriazol-1-yl)-N,N,N',N'-tetramethyluroniumhexafluoro phosphate) |
| HCO2H | formic acid |

| | |
|---|---|
| HCl | hydrogen chloride |
| KHMDS | potassium hexamethyldisilazide |
| KOH | potassium hydroxide |
| $LiAlH_4$ | Lithium aluminum hydride |
| MeCN /$CH_3CN$ | acetonitrile |
| MOM | methoxymethyl |
| MOM-Cl | chloromethyl methyl ether |
| MTBE | methyl tert-butyl ether |
| NaH | sodium hydride |
| $Na_2SO_4$ | sodium sulphate |
| NBS | *N*-Bromosuccinimide |
| NMR | Nuclear Magnetic Resonance |
| PE | petroleum ether |
| r.t. | room temperature |
| sec-BuLi | sec-Butyllithium |
| T3P | propylphosphonic anhydride |
| TEA | triethylamine |
| TFA | trifluoroacetic acid |
| THF | tetrahydrofuran |

Intermediate 1

**2-bromo-3-hydroxyphenyl acetate**

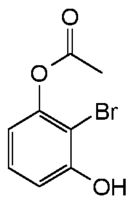

To a solution of 2-bromo-1,3-benzenediol (3.028 g, 16.02 mmol) in dichloromethane (70 ml), TEA (3.35 ml, 24.03 mmol) and acetic anhydride (1.512 ml, 16.02 mmol) were added under stirring. The reaction mixture was stirred at room temperature overnight. The reaction was quenched with a saturated solution of ammonium chloride (100 ml), and extracted with ethyl acetate (3 times 70 ml). The combined organic layers were dried over sodium sulphate, filtered and evaporated to afford the title compound as a black oil which was used directly used in the next step. (3.028g) UPLC_B: 0.41 min, 229 [M-H]-

Intermediate 2

**2-bromo-3-[(2-methyl-2-propen-1-yl))oxy]1phenyl acetate**

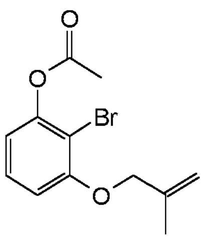

To a solution of 2-bromo-3-hydroxyphenyl acetate (Intermediate 1, 3028 mg) in acetonitrile (60 ml) potassium carbonate (3623 mg, 26.2 mmol) and 3-bromo-2-methyl-1-propene (2123 mg, 15.73 mmol) were added. The reaction

mixture was stirred at room temperature overnight. The mixture was washed with water (3 times 60 ml). The organic phase was separated, dried over sodium sulphate, filtered and evaporated. The residue was purified by flash chromatography on silica gel using a 100g-SNAP column and cyclohexane/ ethyl acetate from 100/0 to 80/20 as eluent to afford the title compound as a colourless oil (2.324 g).
1H NMR (400 MHz, $CDCl_3$): δ ppm 7.27 (1H, t), 6.68 (1H, dd), 5.19 (1H, s), 5.04 (1H, s), 4.53 (2H, s), 2.38 (3H, s), 1.88 (3H, s); UPLC: 0.81 min, 285 [M+H]+

Intermediate 3

**3,3-dimethyl-2,3-dihydro-1-benzofuran-4-yl acetate**

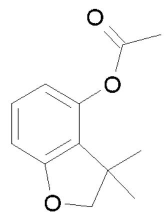

To a solution of 2-bromo-3-[(2-methyl-2-propen-1-yl)oxy]phenyl acetate (Intermediate 2, 2.324 g) in toluene (20 ml) AIBN (1.606 g, 9.78 mmol) and tributylstannane (4.73 g, 16.30 mmol) were added. The reaction mixture was stirred and heated at 100°C for 2 hours, then was left at room temperature for 4 hours. The reaction was quenched with water (60 ml) and extracted with ethyl acetate (3 times 50 ml). The combined organic layers were dried over sodium sulphate, filtered and evaporated. The residue was purified by flash chromatography on silica gel using a 100g-SNAP column and cyclohexane/ethyl acetate from 100/0 to 70/30 as eluent to afford the title compound as a colourless oil (1.290 g).
1H NMR (400 MHz, CDCl3): δ ppm 7.13 (1H, t), 6.68 (1H, d), 6.59 (1H, d), 4.22 (2H, s), 2.33 (3H, s), 1.39 (6H, s). UPLC: 0.72 min, 207 [M+H]+

Intermediate 4

**3,3-dimethyl-2,3-dihydro-1-benzofuran-4-ol**

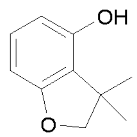

To a solution of 3,3-dimethyl-2,3-dihydro-1-benzofuran-4-yl acetate (Intermediate 3, 1.290 g) in methanol (50 ml) a solution of sodium hydroxide (0.375 g, 9.38 mmol) in water (25.00 ml) was added. The reaction mixture was stirred at room temperature for 30 minutes. The mixture was then acidified with HCl 5 % until pH=5 and extracted with ethyl acetate (3 times 50 ml). The combined organic layers were dried over sodium sulphate, filtered and evaporated. The residue was purified by flash chromatography on silica gel using a 25g-SNAP column and cyclohexane/ethyl acetate from 100/0 to 80/20 as eluent to afford the title compound as a white solid (855 mg).
$^1$H-NMR (400 MHz, $CDCl_3$) δ ppm: 6.98 - 6.94 (1H, t), 6.41 - 6.39 (1H, dd), 6.25 - 6.23 (1H, dd), 4.21 (2H, s), 1.45 (6H, s);UPLC: 0.65 min, 165 [M+H]+.

Intermediate 5

**1,3-bis{[(methyloxy)methyl]oxy}benzene**

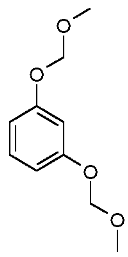

To a solution of 1,3-benzenediol (1.5 g, 13.62 mmol) in dry N,N-Dimethylformamide (13.62 ml) at 0°C sodium hydride (0.981 g, 40.9 mmol) was added and the reaction mixture was stirred for 15 minutes at the same temperature. MOM-Cl (3.10 ml, 40.9 mmol) was quickly added and the reaction mixture was stirred for 1 hour while the temperature was allowed to reach room temperature. The reaction was quenched with brine (20ml) and extracted with ethyl acetate (3x50ml). The organic layer was washed with brine (2x30ml), dried over sodium sulphate, filtered and evaporated and the residue was purified by flash chromatography (Biotage system) on silica gel using a 50g SNAP column and cyclohexane to cyclohexane/ethyl acetate 8:2 as eluents affording the title compound (1.59 g, 8.02 mmol) as a colourless oil.

1H NMR (400MHz, DMSO-$d_6$): δ ppm 7.16-7.23 (1H,d), 6.69-6.64 (3H, m), 5.17 (4H, s), 3.38 (6H, s).

Intermediate 6

**ethyl (2,6-bis{[(methyloxy)methyl]oxy}phenyl)(oxo)acetate**

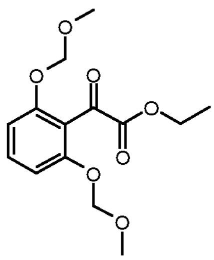

To a solution of 1,3-bis{[(methyloxy)methyl]oxy}benzene (Intermediate 5, 2.19 g) in dry tetrahydrofuran (10 ml) at room temperature BuLi 1.6M in hexane (8.29 ml, 13.26 mmol) was added and the reaction mixture was stirred for 30 minutes at the same temperature. The mixture was cooled to -78 °C and it was added (via cannulation) to a solution of ethyl chloro(oxo)acetate (2.263 g, 16.57 mmol) in dry tetrahydrofuran (10 ml) at -78 °C. The reaction mixture was stirred at -78°C for 30 minutes. The reaction was quenched with an aqueous saturated solution of ammonium chloride (10ml) and extracted with ethyl acetate (2x30ml). Combined organic layers were dried over sodium sulphate, filtered and evaporated. The residue was purified by flash chromatography (Biotage system) on silica gel using a 100g SNAP column and cyclohexane to cyclohexane/ethyl acetate 8:2 as eluent affording the title compound as a light yellow oil (1.75 g).

1H NMR (400MHz, DMSO-$d_6$): δ ppm 7.46 (1H, t), 6.87 (2H, d), 5.20 (4H, s), 4.29 (2H, q), 3.34 (6H, s), 1.27 (3H, t).

Intermediate 7

**ethyl 2-(2,6-bis{[(methyloxy)methyl]oxy}phenyl)-2-propenoate**

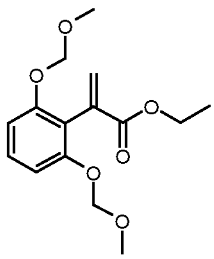

To a suspension of methyltriphenylphosphonium bromide (3.13 g, 8.75 mmol) in dry tetrahydrofuran (30 ml) at 0 °C KHMDS (1.745 g, 8.75 mmol) was slowly added and the reaction mixture was stirred for 15 minutes at 0 °C and for 45 minutes at room temperature. The reaction mixture was cooled to 0 °C and a solution of ethyl (2,6-bis{[(methyloxy)methyl]oxy}phenyl)(oxo)acetate (Intermediate 6, 1.74 g) in dry tetrahydrofuran (10 mL) was slowly added and the reaction mixture was stirred for 2 hours at 0 °C. The reaction was quenched with an aqueous saturated solution of ammonium chloride (10ml), diluted with water (20ml) and extracted with ethyl acetate (2x50ml). The organic layer was dried over sodium sulphate, filtered and evaporated. The residue was purified by flash chromatography (Biotage system) on silica gel using a 100g SNAP column and cyclohexane to cyclohexane/ethyl acetate 8:2 as eluents affording the title compound as a colourless oil (1.37 g).

1H NMR (400MHz, DMSO-ds): δ ppm 7.21 (1H, t), 6.78 (2H, d), 6.44 (1H, d), 5.74 (1H, d), 5.12 (4H, s), 4.12 (2H, q), 3.32 (6H, s), 1.17 (3H, t).

Intermediate 8

**ethyl 1-(2,6-bis{[(methyloxy)methyl]oxy}phenyl)cyclopropanecarboxylate**

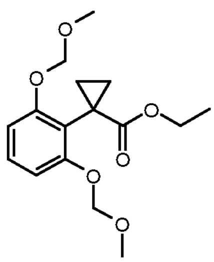

To a solution of trimethylsulfoxonium iodide (1.805 g, 8.20 mmol) in dry dimethyl sulfoxide (20 mL) sodium hydride 60% dispersion in mineral oil (0.310 g, 7.75 mmol) was added and the reaction mixture was stirred for 1 hour at room temperature. A solution of ethyl 2-(2,6-bis{[(methyloxy)methyl]oxy}phenyl)-2-propenoate (Intermediate 7, 1.35 g) in dry dimethyl sulfoxide (10 mL) was slowly added and the reaction mixture was stirred for 1hour at room temperature. The reaction was quenched with an aqueous saturated solution of ammonium chloride (10ml), diluted with water (20ml) and extracted with ethyl acetate (2x50ml). The organic layer was washed with water (50ml), dried over sodium sulphate, filtered and evaporated. The residue was purified by flash chromatography (Biotage system) on silica gel using a 50g SNAP column and cyclohexane to cyclohexane/ethyl acetate 8:2 as eluents affording the title as a colourless oil (1.14 g).

1H NMR (400MHz, DMSO-$d_6$): δ ppm 7.15 (1H, t), 6.71 (2H, d), 5.18 (4H, s), 3.97 (2H, q), 3.36 (6H, s), 1.53-1.58 (2H, m), 1.09-1.14 (2H, m), 1.04 (3H, t).

Intermediate 9

**2-[1-(hydroxymethyl)cyclopropyl]-3-{[(methyloxy)methyl]oxy}phenol**

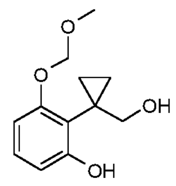

To a solution of ethyl 1-(2,6-bis{[(methyloxy)methyl]oxy}phenyl)cyclopropanecarboxylate (Intermediate 8, 490 mg) in ethanol (10ml) HCl 2N in water (0.789 mL, 1.579 mmol) was added and the reaction mixture was stirred overnight at 50ºC. Toluene (20 mL) was added and the combined solvents were removed under reduced pressure. The residue was re-suspended in toluene (20 ml) and the solvent evaporated. The obtained residue was dissolved in dry tetrahydrofuran (20 ml), the mixture was cooled to 0ºC and NaH 60% dispersion in mineral oil (126 mg, 3.16 mmol) was added and the reaction mixture was stirred for 30 minutes at the same temperature. MOM-Cl (0.120 mL, 1.579 mmol) was then added and the reaction mixture was stirred for 2 hours at 0ºC. $LiAlH_4$ (1M in THF, 1.579 ml, 1.579 mmol) was added and the reaction mixture was further stirred for 1 hour at the same temperature. The reaction was quenched with an aqueous saturated solution of ammonium chloride (10ml), diluted with water (10ml) and extracted with ethyl acetate (2x50ml). Combined organic layers were dried over sodium sulphate, filtered and evaporated and the residue was purified by flash chromatography (Biotage system) on silica gel using a 25g SNAP column and cyclohexane to cyclohexane/ethyl acetate 7:3 as eluents affording the title compound as a colourless oil (191 mg).

1H NMR (400MHz, DMSO-$d_6$): δ ppm 8.90 (1H, br.s) 6.96 (1H, t), 6.50 (1H, d), 6.45 (1H, d), 5.16 (2H, s), 4.93 (1H, br.s), 3.45 (2H, s), 3.40 (3H, s), 0.86-0.93 (2H, m), 0.56-0.62 (2H, m); UPLC: 0.59 min, 225 [M+H]+.

Intermediate 10

**4-{[(methyloxy)methyl]oxy}spiro[1-benzofuran-3,1'-cyclopropane]**

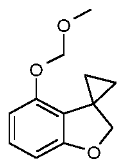

To a solution of 2-[1-(hydroxymethyl)cyclopropyl]-3-{[(methyloxy)methyl]oxy}phenol (Intermediate 9, 190 mg) in dry tetrahydrofuran (10 ml) triphenylphosphine (333 mg, 1.271 mmol) was added and the reaction mixture was stirred until complete dissolution of $PPh_3$. DIAD (0.198 ml, 1.017 mmol) was then added dropwise and the reaction mixture was stirred for 30 minutes at room temperature The solvent was removed under reduced pressure. The residue was purified by flash chromatography (Biotage system) on silica gel using a 25g SNAP column and cyclohexane to cyclohexane/ethyl acetate 9:1 as eluents affording the title compound as a light yellow oil (120 mg).

1H NMR (400MHz, DMSO-$d_6$): δ ppm 6.97 (1H, t), 6.51 (1H, d), 6.43 (1H, d), 5.12 (2H, s), 4.40 (2H, s), 3.35 (3H, s), 1.43-1.48 (2H, m), 0.85-0.90 (2H, m); UPLC_B: 0.88 min, 207 [M+H]+.

Intermediate 11

**spiro[1-benzofuran-3,1'-cyclopropan]-4-ol**

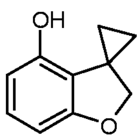

To a solution of 4-{[(methyloxy)methyl]oxy}spiro[1-benzofuran-3,1'-cyclopropane] (Intermediate 10, 118 mg) in methanol (5 ml), HCl 2N in water (0.286 mL, 0.572 mmol) was added and the reaction mixture was stirred overnight

at 50ºC. Combined solvents were removed under reduced pressure and the residue was re-dissolved in toluen (10ml) and the solvent was removed. The residue was purified by flash chromatography (Biotage system) on silica gel using a 10g SNAP column and cyclohexane to cyclohexane/ethyl acetate 7:3 as eluents affording the title compound as a white solid (70 mg).

1H NMR (400MHz, DMSO-$d_6$): δ ppm 9.28 (1H, s), 6.81 (1H, t), 6.24 (1H, d), 6.22 (1H, d), 4.34 (2H, s), 1.40-1.45 (2H, m), 0.77-0.82 (2H, m).

Intermediate 12

**2,4-bis(methoxymethoxy)-1-methyl-benzene**

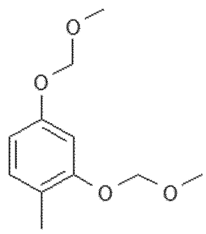

To a solution of 4-methylbenzene-1,3-diol (4 g, 32.26 mmol) in dry N,N-Dimethylformamide (30 ml) at 0°C sodium hydride (60% dispersion in mineral oil) (3.87 g, 96.78 mmol) was added and the reaction mixture was stirred for 15 minutes at the same temperature. MOM-Cl (7.35 ml, 96.78 mmol) was quickly added and the reaction mixture was stirred for 1 hour while the temperature was allowed to reach room temperature. The reaction was quenched with brine (40ml) and extracted with ethyl acetate (3x80ml). The organic layer was washed with ice cold brine (2x50ml), dried over sodium sulphate, filtered and evaporated and the residue was purified by flash chromatography (Biotage system) on silica gel using a 100g SNAP column and cyclohexane to cyclohexane/ethyl acetate 8:2 as eluents affording the title compound (6.1 g) as a colourless oil.

LC/MS: QC_3_MIN: Rt = 1.811 min; 213 [M+H]+.

Intermediate 13

**ethyl 2-[2,6-bis(methoxymethoxy)-3-methyl-phenyl]-2-oxo-acetate**

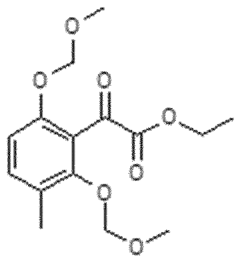

To a solution of 2,4-bis(methoxymethoxy)-1-methyl-benzene (Intermediate 12, 5.5 g, 25.94 mmol) in dry tetrahydrofuran (50 ml) at room temperature BuLi 1.6M in hexane (19.45 ml, 31.13 mmol) was added and the reaction mixture was stirred for 30 minutes at the same temperature. The mixture was cooled to -78 °C and it was added (via cannulation) to a solution of ethyl chlorooxoacetate (4.35 ml, 38.9 mmol) in dry tetrahydrofuran (30 ml) at -78 °C. The reaction mixture was stirred at -78°C for 30 minutes. The reaction was quenched with water (20ml), diluted with brine (50ml) and extracted with ethyl acetate (2×100ml). Combined organic layers were dried over sodium sulphate, filtered and evaporated. The residue was purified by flash chromatography (Biotage system) on silica gel using a 100g SNAP column and cyclohexane to cyclohexane/ethyl acetate 8:2 as eluent affording the title compound (4.65 g) as a light yellow oil.

LC/MS: QC_3_MIN: Rt = 1.865 min.

Intermediate 14

**ethyl 2-[2,6-bis(methoxymethoxy)-3-methyl-phenyl]prop-2-enoate**

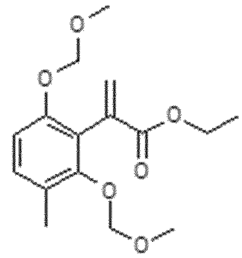

To a suspension of methyltriphenylphosphonium bromide (8.78 g, 24.6 mmol) in dry tetrahydrofuran (50 ml) at 0 °C KHMDS 0.5M solution in toluene (44.22 ml, 22.11 mmol) was slowly added and the reaction mixture was stirred for 15 minutes at 0 °C and for 45 minutes at room temperature. The reaction mixture was cooled to 0 °C and it was slowly added to a solution of ethyl 2-[2,6-bis(methoxymethoxy)-3-methyl-phenyl]-2-oxo-acetate (Intermediate 13, 4.6g, 14.74 mmol ) in dry tetrahydrofuran (25 mL) at 0 °C and the reaction mixture was stirred for 2 hours at 0 °C. The reaction was quenched with water (50ml), diluted with brine (50ml) and extracted with ethyl acetate (2×100ml). The organic layer was dried over sodium sulphate, filtered and evaporated. The residue was purified by flash chromatography (Biotage system) on silica gel using a 100g SNAP column and cyclohexane to cyclohexane/ethyl acetate 8:2 as eluents affording the title compound (3.8 g) as a colourless oil.

LC/MS: QC_3_MIN: Rt = 1.930 min.

Intermediate 15

**ethyl 1-[2,6-bis(methoxymethoxy)-3-methyl-phenyl]cyclopropanecarboxylate**

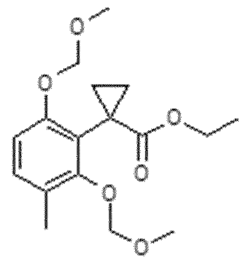

To a solution of trimethylsulfoxonium iodide (4.4 g, 20 mmol) in dry dimethyl sulfoxide (30 mL) sodium hydride (60% dispersion in mineral oil) (0.720 g, 18 mmol) was added and the reaction mixture was stirred for 1 hour at room temperature. A solution of ethyl 2-[2,6-bis(methoxymethoxy)-3-methylphenyl]prop-2-enoate (Intermediate 14, 3.5 g, 11.29 mmol) in dry dimethyl sulfoxide (15 mL) was slowly added and the reaction mixture was stirred for 1 hour at room temperature. The reaction was quenched with an aqueous saturated solution of ammonium chloride (10ml), diluted with water (40ml) and extracted with ethyl acetate (2×100ml). The organic layer was washed with water (2x50ml), dried over sodium sulphate, filtered and evaporated. The residue was purified by flash chromatography (Biotage system) on silica gel using a 100g SNAP column and cyclohexane to cyclohexane/ethyl acetate 8:2 as eluents affording the title compound (3.1g) as a colourless oil.

LC/MS: QC_3_MIN: Rt = 2.028 min.

Intermediate 16

**2-[1-(hydroxymethyl)cyclopropyl]-3-(methoxymethoxy)-6-methyl-phenol**

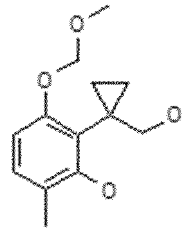

To a solution of ethyl 1-[2,6-bis(methoxymethoxy)-3-methyl-phenyl]cyclopropanecarboxylate (Intermediate 15, 300 mg, 0.93 mmol) in ethanol (10ml) HCl 6N in water (0.4 mL, 2.4 mmol) was added and the reaction mixture was stirred overnight at 50°C. Combined solvents were removed under reduced pressure. The residue was suspended in dry toluene (10 mL) and the solvent evaporated. The obtained residue was dissolved in dry tetrahydrofuran (10 ml), the mixture was cooled to 0°C and NaH (60% dispersion in mineral oil) (80 mg, 2 mmol) was added and the reaction mixture was stirred for 30 minutes at the same temperature. MOM-Cl (0.083 mL, 1.1 mmol) was then added and the reaction mixture was stirred for 1 hour at 0°C. $LiAlH_4$ (1M in THF, 1.2 ml, 1.2 mmol) was added and the reaction mixture was further stirred for 1 hour at the same temperature. The reaction was quenched with an aqueous saturated solution of ammonium chloride (10ml), diluted with water (20ml) and extracted with ethyl acetate (2x50ml). Combined organic layers were dried over sodium sulphate, filtered and evaporated and the residue was purified by flash chromatography (Biotage system) on silica gel using a 25g SNAP column and cyclohexane to cyclohexane/ethyl acetate 7:3 as eluents affording the title compound (70 mg) as a white solid.

LC/MS: QC_3_MIN: Rt = 1.690 min; 239 [M+H]+.

Intermediate 17

**4-(methoxymethoxy)-7-methyl-spiro[2H-benzofuran-3,1'-cyclopropane]**

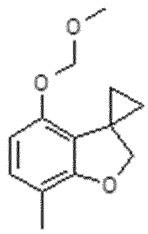

To a solution of 2-[1-(hydroxymethyl)cyclopropyl]-3-(methoxymethoxy)-6-methyl-phenol (Intermediate 16, 65 mg, 0.27 mmol) in dry tetrahydrofuran (5 ml) triphenylphosphine (84 mg, 0.32 mmol) was added and the reaction mixture was stirred until complete dissolution of PPh3. DIAD (0.056 ml, 0.285 mmol) was then added dropwise and the reaction mixture was stirred for 30 minutes at room temperature. The solvent was removed under reduced pressure and the residue was purified by flash chromatography (Biotage system) on silica gel using a 10g SNAP column and cyclohexane to cyclohexane/ethyl acetate 8:2 as eluents affording the title compound (40mg) as a light yellow oil.

LC/MS: QC_3_MIN: Rt = 2.024 min; 221 [M+H]+.

Intermediate 18

**7-methylspiro[2H-benzofuran-3,1'-cyclopropane]-4-ol**

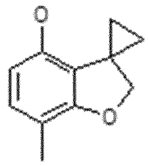

To a solution of 4-(methoxymethoxy)-7-methyl-spiro[2H-benzofuran-3,1'-cyclopropane] (Intermediate 17, 38 mg, 0.17 mmol) in ethanol (5 ml), HCl 6N in water (0.1 mL, 0.6 mmol) was added and the reaction mixture was stirred for 4 days at room temperature. Combined solvents were removed under reduced pressure and the residue was purified by flash chromatography (Biotage system) on silica gel using a 10g SNAP column and cyclohexane to cyclohexane/ethyl acetate 7:3 as eluents affording the title compound (24mg) as a light orange solid.

1H-NMR (400 MHz, DMSO-$d_6$) $\delta$ ppm: 9.02 (1H, s), 6.65 (1H, d), 6.06 (1H, d), 4.36 (2H, s), 2.02 (3H, s), 1.40-1.44 (2H, m), 0.77-0.82 (2H, m).

ROESY (400 MHz, DMSO-$d_6$): NOE correlation between proton at 6.65 ppm and protons (CH3) at 2.02 ppm, NOE correlation between proton at 9.02 ppm and proton at 6.06 ppm.

LC/MS: QC_3_MIN: Rt = 1.647 min; 177 [M+H]+.

Intermediate 19

**5-[(7-methylspiro[1-benzofuran-3,1'-cyclopropan]-4-yl)oxy]-2-nitropyridine**

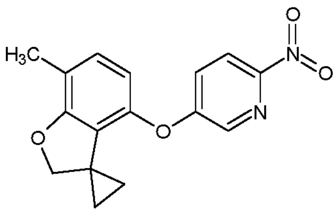

To a solution of 7-methylspiro[2H-benzofuran-3,1'-cyclopropane]-4-ol (Intermediate 18, 50mg, 0.2838mmol) in dry acetonitrile (1mL) dipotassium carbonate (58.826mg, 0.4256mmol) and 5-chloro-2-nitro-pyridine (42.737mg, 0.2696mmol) were added and the reaction mixture was stirred overnight at 90C. After cooling the reaction was quenched with water (1ml), diluted with brine (5ml), and extracted with ethyl acetate (2×10ml). The organic layer was dried (Na2SO4), filtered and evaporated to give the title compound as white solid that was used in the next step without further purification.

LC/MS: QC_3_MIN: Rt = 2.036 min; 299 [M+H]+.

The following compounds were prepared using the foregoing methodology, replacing 7-methylspiro[2H-benzofuran-3,1'-cyclopropane]-4-ol (Intermediate 18) with the appropriate phenol. Final products were purified by flash-chromatography (Silica cartridge; Cyclohexane/EtOAc or other appropriate solvent system).

| Int. | Structure | Name | Phenol | LCMS |
|---|---|---|---|---|
| 20 | 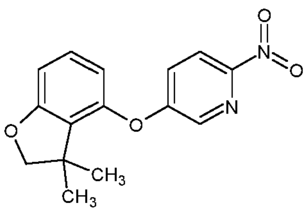 | **5-[(3,3-dimethyl-2,3-dihydro-1-benzofuran-4-yl)oxy]-2-nitropyridine** | 3,3-dimethyl-2,3-dihydro-1-benzofuran-4-ol (Intermediate 4) | LC/MS: QC_3_MIN: Rt = 1.966 min; 287 [M+H]+. |

(continued)

| Int. | Structure | Name | Phenol | LCMS |
|---|---|---|---|---|
| 21 | 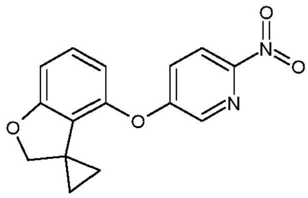 | **2-nitro-5-(spiro[1-benzofuran-3,1'-cyclopropan]-4-yloxy) pyridine** | spiro[1-benzofuran-3,1'-cyclopropan]-4-ol (Intermediate 11) | Not available |

Intermediate 22

**5-[(7-methylspiro[1-benzofuran-3,1'-cyclopropan]-4-yl)oxy]pyridin-2-amine**

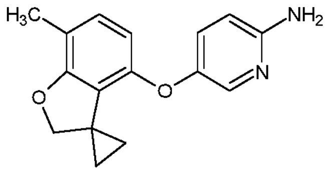

5-[(7-methylspiro[1-benzofuran-3,1'-cyclopropan]-4-yl)oxy]-2-nitropyridine (Intermediate 19) was dissolved in Ethanol (2.5mL)/Water (0.5000mL) and iron (106 mg,1.90mmol) and hydrogen chloride 6N in water (0.047ml, 0.284mmol) were added and the reaction mixture was stirred for 1 hour at 80°C. The reaction was diluted with an aqueous saturated solution of NaHCO3 (5ml) and ethyl acetate (10ml) and the catalyst was filtered off. Two phases were separated. The organic layer was dried (Na2SO4), filtered and evaporated and the residue was purified by flash chromatography (Biotage system) on silica gel using a SNAP 10g as column and cyclohexane/ethyl acetate from 80:20 to 50:50 as eluent affording the title compound (23mg) as white solid.

LC/MS: QC_3_MIN: Rt = 1.688 min; 269 [M+H]+.

The following compounds were prepared using the foregoing methodology, replacing 5-[(7-methylspiro[1-benzofuran-3,1'-cyclopropan]-4-yl)oxy]-2-nitropyridine (Intermediate 19) with the appropriate nitropyridine. Final products were purified by flash-chromatography (Silica cartridge; Cyclohexane/EtOAc or other appropriate solvent system).

| Int. | Structu re | Name | Nitropyridine | LCMS |
|---|---|---|---|---|
| 23 | 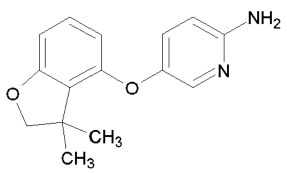 | **5-[(3,3-dimethyl-2,3-dihydro-1-benzofuran-4-yl)oxy]pyridin-2-amine** | 5-[(3,3-dimethyl-2,3-dihydro-1-benzofuran-4-yl)oxy]-2-nitropyridine (Intermediate 20) | LC/MS: QC_ 3_MIN: Rt = 1.555 min; 257 [M+H]+. |
| 24 | 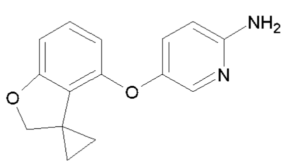 | **5-(spiro[1-benzofuran-3,1'-cyclopropan]-4-yloxy)pyridin-2-amine** | 2-nitro-5-(spiro[1-benzofuran-3,1'-cyclopropan]-4-yloxy) pyridine (Intermediate 21) | Not presently available |

Intermediate 25

**tert-butyl N-[(1R)-1-[[5-(7-methylspiro[2H-benzofuran-3,1'-cyclopropane]-4-yl)oxy-2-pyridyl]carbamoyl]propyl]carbamate**

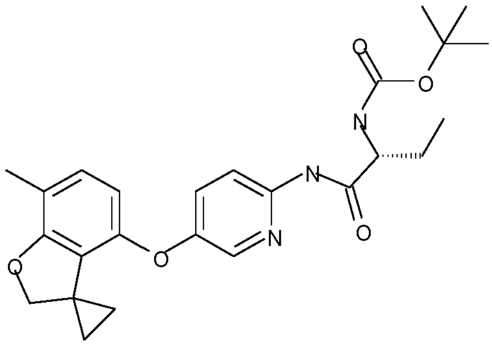

To a solution of (2R)-2-(tert-butoxycarbonylamino)butanoic acid (470mg,2,3mmol) in dry DMF (5mL) N,N-diisopropylethylamine (445mg, 3,44mmol) was added followed by a portionwise addition of [benzotriazol-1-yloxy(dimethylamino)methylene]-dimethyl-ammonium tetrafluoroborate (730mg,2,27mmol), the reaction mixture was stirred for 15 minutes and then a solution of 5-(7-methylspiro[2H-benzofuran-3,1'-cyclopropane]-4-yl)oxypyridin-2-amine (Intermediate 22, 560mg, 2,1mmol) in dry DMF (2mL) was added. The reaction mixture was stirred for 24 hours at room temperature.
The reaction was quenched with ice and then MTBE (30ml) and an aqueous 0.5N solution of HCl (10ml) were added. The mixture was shacken and two phases separated. The organic layer was washed with an aqueous 0.5N solution of HCl (10ml) and then with ice cold brine (2×10ml), then dried (Na2SO4), filtered and evaporated. The residue was purified by flash chromatography (Biotage system) on silica gel using a SNAP 25g as column and cyclohexane/ethyl acetate from 100:0 to 70:30 as eluent affording the title compound (540mg) as white solid
LC/MS: QC_3_MIN: Rt = 2.51 min; 454 [M+H]+.

Intermediate 26

**(2R)-2-amino-N-[5-(7-methylspiro[2H-benzofuran-3,1'-cyclopropane]-4-yl)oxy-2-pyridyl]butanamide**

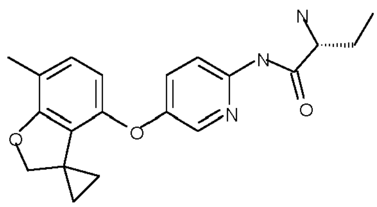

To a solution of tert-butyl N-[(1R)-1-[[5-(7-methylspiro[2H-benzofuran-3,1'-cyclopropane]-4-yl)oxy-2-pyridyl]carbamoyl]propyl]carbamate (Intermediate 25, 540mg, 1,19mmol in DCM (8mL) at 0°C 2,2,2-trifluoroacetic acid (2980mg, 26mmol) was slowly added and the reaction mixture was stirred for 3 hours at the same temperature. volatiles were removed under reduced pressure and the residue was partitionned between DCM and an aqueous saturated solution of NaHCO3. Two phases were separated and the organic layer was dried, filtered and evaporated affording the title compound (410mg) as white solid.
LC/MS: QC_3_MIN: Rt = 1.988 min; 354 [M+H]+.

Intermediate 27

**tert-butyl N-[(1R)-1-[(5-spiro[2H-benzofuran-3,1'-cyclopropane]-4-yloxy-2-pyridyl)carbamoyl]propyl]carbamate**

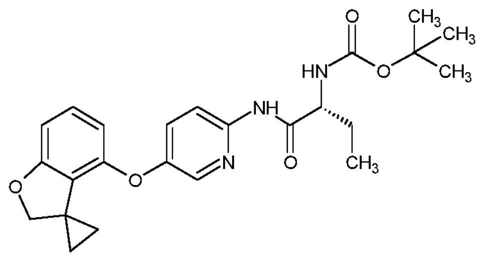

Intermediate 27 was prepared by GlaxoSmithKline s.p.a. and characterising data is presently not available.

Intermediate 28

**(2R)-2-amino-N-[5-(spiro[1-benzofuran-3,1'-cyclopropan]-4-yloxy)pyridin-2-yl]butanamide**

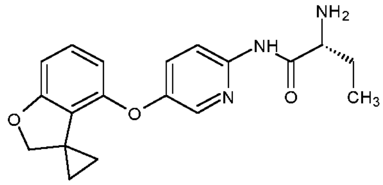

Intermediate 28 was prepared by GlaxoSmithKline s.p.a. and characterising data is presently not available.

Example 1

**(5R)-5-ethyl-5-methyl-3-[5-(7-methylspiro[2H-benzofuran-3,1'-cyclopropane]-4-yl)oxy-2-pyridyl]imidazolidine-2,4-dione**

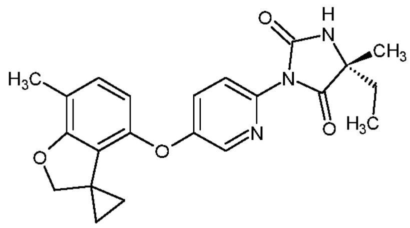

To a solution of bis(trichloromethyl) carbonate (12.7mg,0.043mmol) in Ethyl acetate (1mL) a solution of 5-(7-methylspiro[2H-benzofuran-3,1'-cyclopropane]-4-yl)oxypyridin-2-amine (Intermediate 22, 23mg,0.086mmol)/N-ethyl-N-isopropyl-propan-2-amine (0.15ml, 0.86mmol) in Ethyl acetate (1mL) was slowly added and the reaction mixture was stirred for 15 minutes at room temperature. Vacuum was applied (2 minutes) for removing the excess of phosgene and N,N-dimethylpyridin-4-amine (10.5mg, 0.086mmol) was added. A solution of methyl (2R)-2-amino-2-methyl-butanoate hydrochloride (28.7mg,0.17mmol) in Ethyl acetate (1mL) was added and the reaction mixture was stirred for 30 minutes at room temperature. The reaction was quenched with an aqueous saturated solution of NH4Cl (1ml), diluted with a 0.5M aqueous solution of HCl (5ml) and extracted with ethyl acetate (2×10ml). The organic layer was washed with a 0.5M aqueous solution of HCl (5ml), dried (Na2SO4), filtered and evaporated and the residue was purified by flash chromatography (Biotage system) on silica gel using a SNAP 10g as column and Dichloromethane/methanol from 99.5:0.5 to 95:5 as eluent affording the title compound as white solid.

$^1$H-NMR (400 MHz, DMSO-$d_6$): δ ppm 8.50 (br.s, 1H), 8.29 (d, 1H), 7.47 (dd, 1H), 7.37 (d, 1H), 6.95 (d, 1H), 6.36 (d, 1H), 4.48 (s, 2H), 2.14 (s, 3H), 1.71-1.82 (m, 1H), 1.59-1.70 (m, 1H), 1.38 (s, 3H), 1.20-1.25 (m, 2H), 0.92-0.97 (m, 2H), 0.87 (t, 3H). LC/MS: QC_3_MIN: Rt = 1.994 min; 394 [M+H]+.

The following compound was prepared using the foregoing methodology, replacing 5-(7-methylspiro[2H-benzofuran-3,1'-cyclopropane]-4-yl)oxypyridin-2-amine (Intermediate 22) with the appropriate aniline. Final products were purified by flash-chromatography (Silica cartridge; dichloromethane/methanol, Cyclohexane/EtOAc or other appropriate solvent system).

| Ex. | Structure | Name | Aniline | NMR | LCMS |
|---|---|---|---|---|---|
| 2 | 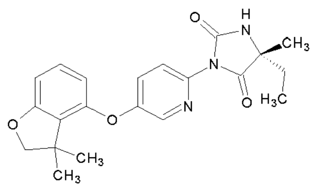 | **(5R)-3-[5-[(3,3-dimethyl-2H-benzofuran-4-yl)oxy]-2-pyridyl]-5-ethyl-5-methylimidazolidine-2,4-dione** | 5-[(3,3-dimethyl-2,3-dihydro-1-benzofuran-4-yl)oxy] pyridin-2-amine (Intermediate 23) | $^1$H-NMR (400 MHz, DMSO-$d_6$): δ ppm 8.51 (br.s, 1H), 8.37 (d, 1H), 7.55 (dd, 1H), 7.41 (d, 1H), 7.13-7.19 (m, 1H), 6.67 (d, 1H), 6.45 (d, 1H), 4.26 (s, 2H), 1.71-1.82 (m, 1H), 1.59-1.70 (m, 1H), 1.39 (s, 3H), 1.36 (s, 6H), 0.88 (t, 3H). | LC/MS: QC_ 3_MIN: Rt = 1.935 min; 382 [M+H]+. |

The following compound was prepared using the foregoing methodology (using Intermediate 22), replacing methyl (2R)-2-amino-2-methyl-butanoate hydrochloride with methyl α-aminoisobutyrate hydrochloride. Final products were purified by flash-chromatography (Silica cartridge; dichloromethane/methanol, Cyclohexane/EtOAc or other appropriate solvent system).

| Ex. | Structu re | Name | NMR | LCMS |
|---|---|---|---|---|
| 3 | 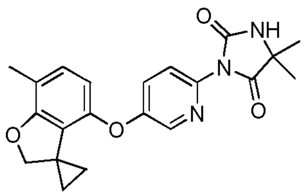 | **5,5-dimethyl-3-[5-(7-methylspiro[2H-benzofuran-3,1'-cyclopropane]-4-yl) oxy-2-pyridyl]imidazolidine-2,4-dione** | | LC/MS: QC_ 3_MIN: Rt = 2.438 min; 380 [M+H]+. |

Example 4

**(5R)-5-ethyl-3-[5-(7-methylspiro[2H-benzofuran-3,1'-cyclopropane]-4-yloxy-2-pyridyl]imidazolidine-2,4-dione**

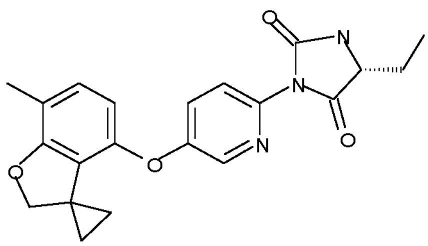

To a solution of (2R)-2-amino-N-[5-(7-methylspiro[2H-benzofuran-3,1'-cyclopropane]-4-yl)oxy-2-pyridyl]butanamide (Intermediate 26, 400mg, 1,13mmol) in DCM (20mL) triethylamine (363mg,3,6mmol) was added and the reaction mixture was cooled to 0°C. A solution of bis(trichloromethyl) carbonate (135mg, 0,45mmol) in DCM (5mL) was slowly added and the reaction mixture was stirred for 30 minutes at the same temperature.

The reaction was diluted with DCM (30ml) and quenched with water (20ml). Two phases were separated and the organic layer was washed with an aqueous 0.5N HCl solution (2x20ml) and then with brine (20ml). The organic layer was dried (Na2SO4), filtered and evaporated and The residue was purified by flash chromatography (Biotage system)

on silica gel using a SNAP 25g as column and cyclohexane/ethyl acetate from 80:20 to 40:60 as eluent affording the title compound (380mg) as white solid.

LC/MS: QC_3_MIN: Rt = 2.18 min; 380 [M+H]+.

Example 5

**5,5-dimethyl-3-(5-spiro[2H-benzofuran-3,1'-cyclopropane]-4-yloxy-2-pyridyl)imidazolidine-2,4-dione**

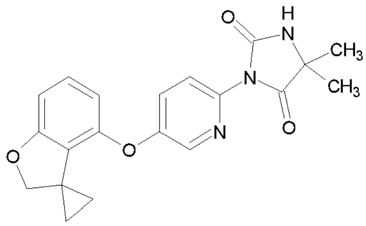

Example 5 was prepared by GlaxoSmithKline s.p.a. and characterising data is presently not available.

Example 6

**(5R)-5-ethyl-3-(5-spiro[2H-benzofuran-3,1'-cyclopropane]-4-yloxy-2-pyridyl)imidazolidine-2,4-dione**

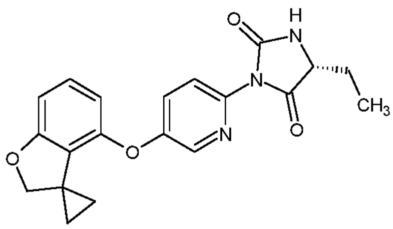

Example 6 was prepared by GlaxoSmithKline s.p.a. and characterising data is presently not available.

The following Reference Examples were prepared as described in WO2012/076877:

Reference Example RE1

**(5*R*)-5-ethyl-3-(6-{[4-methyl-3-(methyloxy)phenyl]oxy}-3-pyridinyl)-2,4-imidazolidinedione**

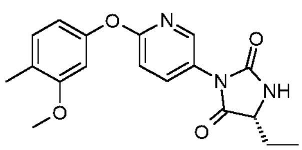

Reference Example RE3

**3-(1,1-dimethylethyl)-4-({5-[(4*R*)-4-ethyl-2,5-dioxo-1-imidazolidinyl]-2-pyridinyl}oxy)benzonitrile**

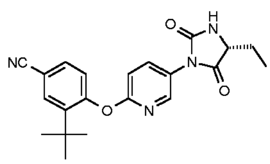

Reference Example RE9

**(5*R*)-5-ethyl-3-[6-(spiro[1-benzofuran-3,1'-cyclopropan]-4-yloxy)-3-pyridinyl]-2,4-imidazolidinedione**

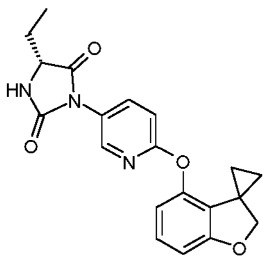

**Biological Example 1**

The ability of the compounds of the invention to modulate the voltage-gated potassium channel subtypes Kv3.2 or Kv3.1 may be determined using the following assay. Analogous methods may be used to investigate the ability of the compounds of the invention to modulate other channel subtypes, including Kv3.3 and Kv3.4.

*Cell biology*

To assess compound effects on human Kv3.2 channels (hKv3.2), a stable cell line expressing hKv3.2 was created by transfecting Chinese Hamster Ovary (CHO)-K1 cells with a pCIH5-hKv3.2 vector. Cells were cultured in DMEM/F12 medium supplemented by 10% Foetal Bovine Serum, 1X non-essential amino acids (Invitrogen) and 500ug/ml of Hygromycin-B (Invitrogen). Cells were grown and maintained at 37°C in a humidified environment containing 5% $CO_2$ in air.

To assess compound effects on human Kv3.1 channels (hKv3.1), CHO/Gam/E1A-clone22 alias CGE22 cells were transduced using a hKv3.1 BacMam reagent. This cell line was designed to be an improved CHO-K1-based host for enhanced recombinant protein expression as compared to wild type CHO-K1. The cell line was generated following the transduction of CHO-K1 cells with a BacMam virus expressing the Adenovirus-Gam1 protein and selection with Geneticin-G418, to generate a stable cell line, CHO/Gam-A3. CHO/Gam-A3 cells were transfected with pCDNA3-E1A-Hygro, followed by hygromycin-B selection and FACS sorting to obtain single-cell clones. BacMam-Luciferase and BacMam-GFP viruses were then used in transient transduction studies to select the clone based on highest BacMam transduction and recombinant protein expression. CGE22 cells were cultured in the same medium used for the hKv3.2 CHO-K1 stable cell line with the addition of 300ug/ml hygromycin-B and 300ug/ml G418. All other conditions were identical to those for hKv3.2 CHO-K1 cells. The day before an experiment 10 million CGE22 cells were plated in a T175 culture flask and the hKv3.1 BacMam reagent (pFBM/human Kv3.1) was added (MOI of 50). Transduced cells were used 24 hours later.

*Cell preparation for* IonWorks Quattro™ *experiments*

The day of the experiment, cells were removed from the incubator and the culture medium removed. Cells were washed with 5 ml of Dulbecco's PBS (DPBS) calcium and magnesium free and detached by the addition of 3 ml Versene (Invitrogen, Italy) followed by a brief incubation at 37°C for 5 minutes. The flask was tapped to dislodge cells and 10 ml of DPBS containing calcium and magnesium was added to prepare a cell suspension. The cell suspension was then placed into a 15 ml centrifuge tube and centrifuged for 2 min at 1200 rpm. After centrifugation, the supernatant was removed and the cell pellet re-suspended in 4 ml of DPBS containing calcium and magnesium using a 5ml pipette to break up the pellet. Cell suspension volume was then corrected to give a cell concentration for the assay of approximately 3 million cells per ml.

All the solutions added to the cells were pre-warmed to 37°C.

*Electrophysiology*

Experiments were conducted at room temperature using IonWorks Quattro™ planar array electrophysiology technology (Molecular Devices Corp.) with PatchPlate™ PPC. Stimulation protocols and data acquisition were carried out using a microcomputer (Dell Pentium 4). Planar electrode hole resistances(Rp) were determined by applying a 10 mV voltage step across each well. These measurements were performed before cell addition. After cell addition and seal formation, a seal test was performed by applying a voltage step from -80 mV to -70 mV for 160 ms. Following this,

amphotericin-B solution was added to the intracellular face of the electrode to achieve intracellular access. Cells were held at -70mV. Leak subtraction was conducted in all experiments by applying 50 ms hyperpolarizing (10 mV) prepulses to evoke leak currents followed by a 20 ms period at the holding potential before test pulses. From the holding potential of -70 mV, a first test pulse to -15 mV was applied for 100 ms and following a further 100 ms at -70 mV, a second pulse to 40 mV was applied for 50 ms. Cells were then maintained for a further 100 ms at -100 mV and then a voltage ramp from -100 mV to 40 mV was applied over 200 ms. Test pulses protocol may be performed in the absence (pre-read) and presence (post-read) of the test compound. Pre- and post-reads may be separated by the compound addition followed by a 3 minute incubation.

*Solutions and drugs*

The intracellular solution contained the following (in m*M*): K-gluconate 100, KCl 54, $MgCl_2$ 3.2, HEPES 5, adjusted to pH 7.3 with KOH. Amphotericin-B solution was prepared as 50mg/ml stock solution in DMSO and diluted to a final working concentration of 0.1 mg/ml in intracellular solution. The external solution was Dulbecco's Phosphate Buffered Saline (DPBS) and contained the following (in m*M*): $CaCl_2$ 0.90, KCl 2.67, $KH_2PO_4$ 1.47, $MgCl.6H_2O$ 0.493, NaCl 136.9, $Na_3PO_4$ 8.06, with a pH of 7.4.

Compounds of the invention (or reference compounds such as *N*-cyclohexyl-*N*-[(7,8-dimethyl-2-oxo-1,2-dihydro-3-quinolinyl)methyl]-*N*'-phenylurea were dissolved in dimethylsulfoxide (DMSO) at a stock concentration of 10 mM. These solutions were further diluted with DMSO using a Biomek FX (Beckman Coulter) in a 384 compound plate. Each dilution (1 μL) was transferred to another compound plate and external solution containing 0.05% pluronic acid (66 μL) was added. 3.5 μL from each plate containing a compound of the invention was added and incubated with the cells during the IonWorks Quattro™ experiment. The final assay dilution was 200 and the final compound concentrations were in the range 50 μM to 50 nM.

*Data analysis*

The recordings were analysed and filtered using both seal resistance (>20 MΩ) and peak current amplitude (>500pA at the voltage step of 40 mV) in the absence of compound to eliminate unsuitable cells from further analysis. Paired comparisons between pre- and post-drug additions measured for the - 15 mV voltage step were used to determine the positive modulation effect of each compound. Kv3 channel-mediated outward currents were measured determined from the mean amplitude of the current over the final 10ms of the -15mV voltage pulse minus the mean baseline current at -70mV over a 10ms period just prior to the -15mV step. These Kv3 channel currents following addition of the test compound were then compared with the currents recorded prior to compound addition. Data were normalised to the maximum effect of the reference compound (50microM of *N*-cyclohexyl-*N*-[(7,8-dimethyl-2-oxo-1,2-dihydro-3-quinolinyl)methyl]-*N*'-phenylurea) and to the effect of a vehicle control (0.5% DMSO). The normalised data were analysed using ActivityBase or Excel software. The concentration of compound required to increase currents by 50% of the maximum increase produced by the reference compound (EC50) was determined by fitting of the concentration-response data using a four parameter logistic function with ActivityBase or XL-fit software.

*N*-cyclohexyl-*N*-[(7,8-dimethyl-2-oxo-1,2-dihydro-3-quinolinyl)methyl]-*N*'-phenylurea was obtained from ASINEX (Registry Number: 552311-06-5).

All of the Example compounds were tested in the above assay measuring potentiation of Kv3.1 or Kv3.2 or Kv3.1 and Kv3.2 (herein after "Kv3.1 and/or Kv3.2"). Kv3.1 and/or Kv3.2 positive modulators produce in the above assay an increase of whole-cell currents of, on average, at least 20% of that observed with 50microM *N*-cyclohexyl-*N*-[(7,8-dimethyl-2-oxo-1,2-dihydro-3-quinolinyl)methyl]-*N*'-phenylurea. Thus, in the recombinant cell assays of Biological Example 1, all of the Example compounds act as positive modulators of Kv3.1 and Kv3.2 channels. As used herein, a Kv3.1 and/or Kv3.2 positive modulator is a compound which has been shown to produce at least 20% potentiation of whole-cell currents mediated by human Kv3.1 and/or human Kv3.2 channels recombinantly expressed in mammalian cells, as determined using the assays described in Biological Example 1 (Biological Assays).

A secondary analysis of the data from the assays described in Biological Example 1 may be used to investigate the effect of the compounds on rate of rise of the current from the start of the depolarising voltage pulses. The magnitude of the effect of a compound can be determined from the time constant ($Tau_{act}$) obtained from a non-linear fit, using the equation given below, of the rise in Kv3.1 or Kv3.2 currents following the start of the -15mV depolarising voltage pulse.

$$Y = (Y0 - Ymax) * exp(-K*X) + Ymax$$

where:

Y0 is the current value at the start of the depolarising voltage pulse;
Ymax is the plateau current;
K is the rate constant, and $Tau_{act}$ is the activation time constant, which is the reciprocal of K.

Similarly, the effect of the compounds on the time taken for Kv3.1 and Kv3.2 currents to decay on closing of the channels at the end of the -15mV depolarising voltage pulses can also be investigated. In this latter case, the magnitude of the effect of a compound on channel closing can be determined from the time constant ($Tau_{deact}$) of a non-linear fit of the decay of the current ("tail current") immediately following the end of the depolarising voltage pulse.
The time constant for activation ($Tau_{act}$) has been determined for all of the compounds of the Examples. Figure 1 shows the data for two compounds. Table 1 provides the $Tau_{act}$ data for all of the Examples analysed in this way.
Figure 1a shows hKv3.2 currents recorded using the assay described in Biological Example 1. Data shown are the individual currents over the period of the depolarising voltage step to -15mV recorded from 4 different cells at two concentrations of compound (Reference Example RE1). The data are fitted by a single exponential curve (solid lines) using the fitting procedure in Prism version 5 (Graphpad Software Inc).
Figure 1b shows hKv3.2 currents recorded using the assay described in Biological Example 1. Data shown are the individual currents over the period of the depolarising voltage step to -15mV recorded from 2 different cells at two concentrations of the compound of Reference Example RE3. The data are fitted by a single exponential curve (solid lines) using the fitting procedure in Prism version 5 (Graphpad Software Inc).

Table 1: Summary hKv3.2 data from the analysis of activation time ($Tau_{act}$). To allow for comparison between compounds, the compound concentration chosen was that which produced a similar current (~0.3nA) at the end of the voltage pulse, with the exception of the vehicle, where maximum currents were <0.1nA.

| Example | Concentration (μM) | $Tau_{act}$ mean (ms) | Standard Deviation | Number of experiments |
|---|---|---|---|---|
| Vehicle | - | 7.1 | 1.7 | 6 (cells) |
| RE1 | 6.25 | 9.9 | 2.2 | 5 |
| RE3 | 0.2 | 23.0 | 6.2 | 4 |
| RE9 | 0.2 | 50.1 | 7.5 | 5 |
| Example 1 | 0.8 | 18.2 | - | 1 |

As can be seen from Table 1, in the absence of compound and presence of vehicle the $Tau_{act}$ was $7.1\pm1.7$ msec. A range of $Tau_{act}$ values (7.3 - 50.1 msec) was observed in the presence of the test compounds when each was tested at a concentration that increased the Kv3.2 current to a similar level (~ 0.3nA). Kv3.1 and Kv3.2 channels must activate and deactivate very rapidly in order to allow neurons to fire actions potentials at high frequency (Rudy, 2001). Slowing of activation is likely to delay the onset of action potential repolarisation; slowing of deactivation could lead to hyperpolarising currents that reduce the excitability of the neuron and delay the time before the neuron can fire a further action potential. Together these slowing effects on channel activation and deactivation are likely to lead to a reduction rather than a facilitation of the neurons ability to fire at high frequencies. Thus compounds that have this slowing effect on the Kv3.1 and/or Kv3.2 channels may slow neuronal firing. This slowing of neuronal firing by a compound, such as Reference Example RE9 which markedly increases $Tau_{act}$ to $50.1 \pm 7.5$ msec (Table 1), can be observed from recordings made from "fast-firing" interneurons in the cortex of rat brain, using electrophysiological techniques, *in vitro.* As can be observed in Figure 2, the addition of Reference Example RE9 reduces the ability of the neurons to fire in response to trains of depolarising pulses at 300Hz.
Figure 2 shows recordings made from identified "fast-firing" interneurons in the somatosensory cortex of the mouse. The neurons are induced to fire at high frequencies by trains of high frequency depolarising current pulses at 100, 200, and 300Hz. The ability of the neuron to fire an action potential on each pulse is determined. A spike probability of 1 on the y-axis of the graph indicates that an action potential is generated by the neuron on each of the depolarising current pulses. In the absence of drug (closed circles, n=9), the neurons maintained a spike probability of 1 up to 300Hz. However, in the presence of Reference Example RE9 (1microM; open circles, n=6), the neurons were unable to follow trains at the highest frequency. * $p < 0.05$, ANOVA for repeated measures.
Therefore, although all the Examples herein identified act as positive modulators in the recombinant cell assay of Biological Example 1, those compounds which markedly increase the value of $Tau_{act}$, may reduce the ability of neurons in native tissues to fire at high frequency.

## Biological Example 2: Determination of blood and brain tissue binding

*Materials and Methods*

Rat whole blood, collected on the week of the experiment using K3-EDTA as an anti-coagulant, is diluted with isotonic phosphate buffer 1:1 (v/v). Rat whole brain, stored frozen at-20 °C, is thawed and homogenised in artificial cerebrospinal fluid (CSF) 1 :2 (w/v).

An appropriate amount of test compound is dissolved in DMSO to give a 5 millimolar solution. Further dilutions, to obtain a 166.7 micromolar working solution are then prepared using 50% acetonitrile in MilliQ water. This working solution is used to spike the blood to obtain a final concentration of 0.5micromolar in whole blood. Similarly, the working solution is used to spike brain samples to obtain a final concentration of 5 micromolar in whole brain. From these spiked blood and brain preparations, control samples (n=3), are immediately extracted and used to calculate the initial recovery of the test items.

150 microL of compound-free buffer (isotonic phosphate buffer for blood or artificial CSF buffer for brain) is dispensed in one half-well and 150 microL of spiked matrix (blood or brain) is loaded in the other half-well, with the two halves separated by a semi-permeable membrane. After an equilibration period of 5 h at 37°C, 50 microL of dialysed matrix (blood or brain) is added to 50 microL of corresponding compound-free buffer, and vice-versa for buffer, such that the volume of buffer to matrix (blood or brain) remains the same. Samples are then extracted by protein precipitation with 300 microL of acetonitrile containing rolipram (control for positive ionization mode) or diclofenac (control for negative ionization mode) as internal standards and centrifuged for 10min at 2800rpm. Supernatants are collected (100 microL), diluted with 18% ACN in MilliQ water (200 microL) and then injected into an HPLC-MS/MS or UPLC-MS/MS system to determine the concentration of test compound present.

*Analysis*

Blood and brain tissue binding are then determined using the following formulas:
Afu=Buffer/Blood or Afu=CSF/Brain

Where Afu = apparent fraction unbound; Buffer= analyte/intemal standard ratio determined in the buffer compartment; Blood= analyte/intemal standard ratio determined in the blood compartment; Brain= analyte/intemal standard ratio determined in the brain compartment.

$$\text{Fucr} = \frac{1/D}{[(1/\text{Afu} - 1) + 1/D]}$$

where: fucr = Fraction unbound corrected; D =matrix dilution factor (D=2 for blood and D=3 for brain).

Then:

$$\%\text{Binding} = (1 - \text{fucr}) \times 100$$

$$\%\text{Unbound} = 100 - \%\text{Bound}$$

*Brain/Blood partition ratio (Kbb) Determination*

For compounds freely permeable across the blood/brain barrier (BBB), the unbound concentrations in blood and brain would be equivalent under steady-state distribution conditions. Therefore, the Kbb value could be calculated as:
Fu(blood)/Fu(brain)
which is expected to be equivalent to the brain-to-blood concentration ratio (Ct(brain)/Ct(blood)) if efflux pump transporters are not involved.

## Biological Example 3: Determination of *in vivo* pharmacokinetic parameters

*Materials and Methods*

Adult male rats (Charles River, Italy) are dosed with test compound orally at 1mg/kg (5 ml/kg, in 5% v/v DMSO, 0.5% w/v HPMC in water) and intravenously at 0.5mg/kg (2ml/kg, in 5% v/v DMSO 40% w/v PEG400 in saline). After

oral administration, blood samples are collected under deep Isofluorane anesthesia from the portal vein and heart of each rat (1 rat per time point). After intravenous administration, serial blood samples are collected from the lateral tail vein of each rat. A further group of rats (n=1 per test compound) receive a single intravenous administration of the PgP transport inhibitor, Elacridar (3 mg/kg) shortly before the oral administration of the test compound at 1 mg/kg, as above. Blood and brain samples ar collected at a single timepoint of 0.5 h after dose administration for these animals. In all cases, blood samples are collected into potassium EDTA tubes.

Blood and brain samples can be assayed for test compound concentration using a method based on protein precipitation with acetonitrile followed by HPLC/MS-MS analysis with an optimized analytical method.

*Analysis*

The concentrations of test compound in blood (expressed as ng/ml) and brain (expressed as ng/g) at the different time points following either oral or intravenous dosing are analysed using a non-compartmental pharmacokinetic model using WinNonLin Professional version 4.1. The following parameters are derived:
Intravenous dosing: Maximum concentration over time (Cmax), integrated concentration over time (AUC), clearance (Clb), volume of distribution (Vss) and half-life (t1/2).

Oral dosing: Cmax, time of maximum concentration (Tmax), AUC, bioavailability (F%), fraction absorbed (Fa%), blood to brain ratio (AUC BB), and Fold-change in AUC BB in the presence of Elacridar.

Compounds of the invention may be expected to demonstrate good availability in brain tissue.

**Biological Example 4: Evaluation of the effect of modulators of Kv3.1/Kv3.2 channels on sensitivity to mechanical and cold stimuli in models of neuropathic and inflammatory pain in the rat**

The efficacy of the following compound was investigated using rat models of neuropathic and persistant inflammatory pain:

5,5-dimethyl-3-[2-(7-methylspiro[2H-benzofuran-3,1'-cyclopropane]-4-yl)oxypyrimidin-5-yl]imidazolidine-2,4-dione - Example 58 in WO2012/076877 (referred to herein as "Compound X").

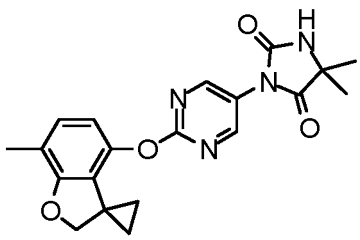

*Materials and Methods*

Subjects comprised male, Wistar Hanover rats, 6 animals per group (225 ± 2g).

Vehicle (12% Captisol®; 0.5% w/v HPMC and 0.1% w/v Tween-80; 5 ml/kg via the intraperitoneal route) was prepared using autoclaved deionized water not more than one week prior to use.

The details of the studies performed with 5,5-dimethyl-3-[2-(7-methylspiro[2H-benzofuran-3,1'-cyclopropane]-4-yl)oxypyrimidin-5-yl]imidazolidine-2,4-dione (Compound X) are outlined in Table 1.

Table 1: Dosing regimen for neuropathic and inflammatory pain models

| Compound | Neuropathic Pain | Inflammatory Pain |
|---|---|---|
| 5,5-dimethyl-3-[2-(7-methylspiro[2H-benzofuran-3,1'-cyclopropane]-4-yl)oxypyrimidin-5-yl]imidazolidine-2,4-dione (Compound X) | 10 mg/kg (i.p.)[1]<br>30 mg/kg (i.p.)<br>60 mg/kg (i.p.) | 10 mg/kg (i.p.)<br>30 mg/kg (i.p.)<br>60 mg/kg (i.p.) |
| [1](i.p.) intraperitoneal administration | | |

The control for the neuropathic pain model was lamotrigine, administered at 30 mg/kg via oral delivery. The control for the inflammatory pain model was diclofenac, administered at 30 mg/kg via oral delivery. Statistical analysis was performed using one-way ANOVA, and comparisons were performed with time-matched vehicle group using Tukey's HSD test wherein * $p < 0.05$, ** $p < 0.01$, *** $p < 0.001$.

*Experimental Protocol*

All experimental procedures were approved following review by an institutional ethics committee on the use of animals is research, and were carried out in accordance with the UK Home Office Animal Procedures Act (1986).
Treatment groups were randomised and blinded. Groups of 6 rats were used.

*Neuropathic pain*

Neuropathic pain was induced by partial ligation of the sciatic nerve. Briefly, the rats were anaesthetised (isoflurane/$O_2$ inhalation), the left sciatic nerve exposed at mid-thigh level through a small incision and 1/3 to 1/2 of the nerve thickness tightly ligated within a 7.0 silk suture. The wound was closed with surgical glue. Animals were allowed to recover and tested 12-15 days following surgery.
Withdrawal thresholds were measured on both the ipsilateral (ligated) and contralateral (non-ligated) paws, prior to (predose) and then up to 24 h following drug or vehicle administration.
Pre-dose behavioural measurements were obtained by measuring paw withdrawals 14 days following nerve ligation; before the initiation of drug treatment. Following treatment, further readings were taken at 1, 3, 6 and 24 hour after administration.

*Inflammatory pain*

Mechanical hyperalgesia was examined in a model of persistent inflammatory pain.
The hyperalgesia was induced by an intraplantar injection (25 μl) of Freund's Complete Adjuvant (FCA) into the left hind paw.
To assess the effect of the test compound, paw withdrawal thresholds were measured on both the ipsilateral (FCA-injected) and contralateral (non-injected) paws, prior to (naïve) and 24 h following FCA injection (predose), and then at 1, 3, 6 and 24 h after drug or vehicle administration.

*Behavioural tests*

Mechanical hyperalgesia was examined in a model of neuropathic pain by measuring paw withdrawal thresholds (PWT) to increasing mechanical force applied to the dorsal surface of the rat paw using an Analgesymeter (Ugo-Basile, Milan) equipped with a wedge-shaped probe (area 1.75 $mm^2$). Cut-off was set at 250 g and the end-point was taken as withdrawal of the hind paw. Both ipsilateral and contralateral paw withdrawal readings were taken.
Cold sensitivity can be assessed using a commercially available cold-plate (Ugo Basile, Milan). The cold plate is allowed to stabilize for 5 minutes at the set temperature prior to testing. Paw withdrawal latencies (PWL) are determined with the cold-plate set at 10 °C. The animals are lightly restrained and each hind paw in turn placed onto the surface of the cold-plate. The end point is taken as the withdrawal of the paw and recorded as the withdrawal latency for the ipsilateral and the contralateral paw. A maximum cut-off of 30 seconds is used for each paw.

*General observations*

In addition to behavioural pain readings, each rat was observed throughout the study for changes in general behaviour.

**Data Analysis**

*Neuropathic pain*

Data were expressed as withdrawal threshold (g) and percentage reversals calculated according to the following formula:

$$\%\ reversal = \frac{ipsilaterd\ threshold\ postdose - ipsilaterd\ threshold\ predose}{contralateral\ threshold\ predose - ipsilaterd\ threshold\ predose}\ X\ 100$$

*Inflammatory pain*

Data were expressed as withdrawal threshold (g) and percentage reversals calculated according to the following

formula:

$$\% \text{ reversal} = \left( \frac{\text{left postdose PWT/L - left predose PWT/L}}{\text{left naïve PWT/L - left predose PWT/L}} \right) \times 100$$

Statistical analysis was carried out on withdrawal threshold readings using ANOVA with repeated measures followed by Tukey's HSD test. The level for statistical significance was set as $p < 0.05$.

Results

*Neuropathic pain study*

Partial ligation of the sciatic nerve resulted in a marked decrease in withdrawal threshold to a mechanical stimulus and in withdrawal latency to a cold stimulus of the affected paw. Fourteen days after nerve ligation, predose threshold readings of 66 ± 1g were measured in the ipsilateral paws compared to 104 ± 1g in the contralateral paws.

Compound X produced a dose-related reversal of mechanical sensitivity with rapid onset and long duration of action.

Peak reversal of mechanical sensitivity was seen at 3 h post-dose (31% at 10 mg/kg, 73% at 30 mg/kg and 81% by 60 mg/kg). The positive control, lamotrigine, gave peak reversals at 3 h post-dose of 67%.

*Inflammatory pain study*

The intraplantar injection of FCA resulted in a marked decrease in withdrawal threshold to a mechanical stimulus of the affected paw. The mean naïve threshold readings were 105 ± 1g. 24 h after FCA injection, predose threshold readings of 65 ± 1.0g were measured in the ipsilateral paws compared to 104 ± 1.0g in the contralateral paws.

Compound X produced a dose-related reversal of mechanical sensitivity with rapid onset of action and peak reversal at 1-3 h post-dose. Peak reversal of mechanical sensitivity was seen at 1 h post-dose for 30 mg/kg and 60 mg/kg (74% and 92% respectively) and at 3 h post-dose for 10 mg/kg (64% reversal). The reversal was long lasting with significant activity still evident at 6 h post-dose: mechanical sensitivity was reversed by 45% with 60 mg/kg. The positive control, diclofenac, gave peak reversals of 64% in mechanical (1 h post-dose).

*Conclusions*

In a rat models of neuropathic and inflammatory pain, Compound X, which is a selective modulator of Kv3.1 and/or Kv3.2 and/or Kv3.3 channels, was effective at reversing behavioural measures of pain when administered acutely, but without causing significant changes in normal behaviour. These data strongly support the proposition that modulation of Kv3.1 and/or Kv3.2 and/or Kv3.3 channels has potential in the treatment of pain.

Additional animal models

Patent applications WO2011/069951, WO2012/076877, WO2012/168710, WO2013/175215 and WO2013/182851 (all cited for the purpose providing animal model data) demonstrate the activity of compounds which are modulators of Kv3.1 and Kv3.2 in animal models of seizure, hyperactivity, sleep disorders, psychosis, hearing disorders and bipolar disorders.

Patent application WO2013/175211 (cited for the purpose of providing animal model data) demonstrates the efficacy of a compound which is a modulator of Kv3.1 and Kv3.2 in a model of acute noise-induced hearing loss in the chinchilla, and also evaluates the efficacy of the compound in a model of central auditory processing deficit and in a model of tinnitus.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

The application of which this description and claims forms part may be used as a basis for priority in respect of any subsequent application. The claims of such subsequent application may be directed to any feature or combination of features described herein. They may take the form of product, composition, process, or use claims and may include, by way of example and without limitation, the claims which follow.

References


Aroniadou-Anderjaska V, Qashu F, Braga MFM. Mechanisms regulating GABAergic inhibitory transmission in the basolateral amygdala: implications for epilepsy and anxiety disorders. Amino Acids 2007 Aug;32:305-315.

Baranauskas G, Nistri A. Sensitization of pain pathways in the spinal cord: cellular mechanisms. Prog. Neurobiol. 1998 Feb;54(3):349-65.

Baron R, Hans G, Dickenson AH. Peripheral input and its importance for central sensitization. Ann. Neurol. 2013 Nov;74(5):630-6.

Ben-Ari Y . Seizure Beget Seizure: The Quest for GABA as a Key Player. Crit. Rev. Neurobiol. 2006;18(1-2):135-144.

Benes FM, Lim B, Matzilevich D, Subburaju S, Walsh JP. Circuitry-based gene expression profiles in GABA cells of the trisynaptic pathway in schizophrenics versus bipolars. PNAS 2008 Dec;105(52):20935-20940.

Bennett DL, Woods CG. Painful and painless channelopathies. Lancet Neurol. 2014 Jun;13(6):587-99.

Berge S, Bighley L, Monkhouse D. Pharmaceutical Salts. J. Pharm. Sci. 1977;66;1-19.

Brambilla P, Perez J, Schettini G, Soares JC. GABAergic dysfunction in mood disorders. Mol. Psych. 2003 Apr;8:721-737.

Brooke RE, Pyner S, McLeish P, Buchan S, Deuchars J, Deuchars SA. Spinal cord interneurones labelled transneuronally from the adrenal gland by a GFP-herpes virus construct contain the potassium channel subunit Kv3.1b. Auton. Neurosci. 2002 Jun;98(1-2):45-50.

Brooke RE, Atkinson L, Batten TF, Deuchars SA, Deuchars J. Association of potassium channel Kv3.4 subunits with pre- and post-synaptic structures in brainstem and spinal cord. Neuroscience 2004;126(4):1001-10.

Brooke RE, Atkinson L, Edwards I, Parson SH, Deuchars J. Immunohistochemical localisation of the voltage gated potassium ion channel subunit Kv3.3 in the rat medulla oblongata and thoracic spinal cord. Brain Res. 2006 Jan;1070(1):101-15.

Cervero F. Spinal cord hyperexcitability and its role in pain and hyperalgesia. Exp. Brain Res. 2009 Jun;196(1):129-37.

Chang SY, Zagha E, Kwon ES, Ozaita A, Bobik M, Martone ME, Ellisman MH, Heintz N, Rudy B. Distribution of Kv3.3 Potassium Channel Subunits in Distinct Neuronal Populations of Mouse Brain. J. Comp. Neuro. 2007 Feb;502:953-972.

Chien LY, Cheng JK, Chu D, Cheng CF, Tsaur ML. Reduced expression of A-type potassium channels in primary sensory neurons induces mechanical hypersensitivity. J. Neurosci. 2007 Sep;27(37):9855-65.

Chow A, Erisir A, Farb C, Nadal MS, Ozaita A, Lau D, Welker E, Rudy B. K+ Channel Expression Distinguishes Subpopulations of Parvalbumin- and Somatostatin-Containing Neocortical Interneurons. J. Neurosci. 1999 Nov;19(21):9332-9345.

Desai R, Kronengold J, Mei J, Forman S, Kaczmarek L. Protein Kinase C Modulates Inactivation of Kv3.3 Channels. J. Biol. Chem. 2008;283;22283-22294.

Deuchars SA, Brooke RE, Frater B, Deuchars J. Properties of interneurones in the intermediolateral cell column of the rat spinal cord: role of the potassium channel subunit Kv3.1. Neuroscience 2001;106(2):433-46.

Devulder J. Flupirtine in pain management: pharmacological properties and clinical use. CNS Drugs 2010 Oct;24(10):867-81.

Dib-Hajj SD, Yang Y, Black JA, Waxman SG. The Na(V)1.7 sodium channel: from molecule to man. Nat. Rev. Neurosci. 2013 Jan;14(1):49-62.

Diochot S, Schweitz H, Béress L, Lazdunski M. Sea Anemone Peptides with a Specific Blocking Activity against the Fast Inactivating Potassium Channel Kv3.4. J. Biol. Chem. 1998 Mar;273(12);6744-6749.

Engel AK, Fries P, Singer W. Dynamic Predictions: Oscillations and Synchrony in Top-Down Processing. Nat. Rev. Neurosci. 2001 Oct;2(10):704-716.

Espinosa F, McMahon A, Chan E, Wang S, Ho CS, Heintz N, Joho RH. Alcohol Hypersensitivity, Increased Locomotion, and Spontaneous Myoclonus in Mice Lacking the Potassium Channels Kv3.1 and Kv3.3. J. Neurosci. 2001 Sep;21(17):6657-6665.

Espinosa F, Torres-Vega MA, Marks GA, Joho RH. Ablation of Kv3.1 and Kv3.3 Potassium Channels Disrupts Thalamocortical Oscillations In Vitro and In Vivo. J. Neurosci. 2008 May;28(21):5570-5581.

Figueroa K, Minassian N, Stevanin G, Waters M, Garibyan V, Forlani S, Strzelczyk A, Burk K, Brice A, Durr A, Papazian D, Pulst S. KCNC3: phenotype, mutations, channel biophysics - a study of 260 familial ataxia patients. Human Mutation. 2010;31;191-196.

Finnerup NB, Attal N, Haroutounian S, McNicol E, Baron R, Dworkin RH, Gilron I, Haanpää M, Hansson P, Jensen TS, Kamerman PR, Lund K, Moore A, Raja SN, Rice AS, Rowbotham M, Sena E, Siddall P, Smith BH, Wallace M. Pharmacotherapy for neuropathic pain in adults: a systematic review and meta-analysis. Lancet Neurol. 2015 Feb;14(2):162-73.

Fisahn A. Kainate receptors and rhythmic activity in neuronal networks: hippocampal gamma oscillations as a tool. J. Physiol. 2005 Oct;561(1):65-72.

Joho RH, Ho CS, Marks GA. Increased γ- and Decreased δ-Oscillations in a Mouse Deficient for a Potassium Channel Expressed in Fast-Spiking Interneurons. J. Neurophysiol. 1999 Jun;82:1855-1864. Joho RH, Hurlock EC. The Role of Kv3-type Potassium Channels in Cerebellar Physiology and Behavior. Cerebellum 2009 Feb;8:323-333.

Jung D, Lee S, Kim D, Joo K, Cha C, Yang H, Lee W, Chung Y. Age-related changes in the distribution of Kv1.1 and Kv3.1 in rat cochlear nuclei. Neurol. Res.2005;27;436-440.

Kasten MR, Rudy B, Anderson MP. Differential regulation of action potential firing in adult murine thalamocortical neurons by Kv3.2, Kv1, and SK potassium and N-type calcium channels. J. Physiol. 2007;584(2):565-582.

Kaczmarek L, Bhattacharjee A, Desai R, Gan L, Song P, von Hehn C, Whim M, Yang B. Regulation of the timing of MNTB neurons by short-term and long-term modulation of potassium channels. Hearing Res. 2005;206;133-145.

Lau D, Vega-Saenz de Miera E, Contreras D, Ozaita A, Harvey M, Chow A, Noebels JL, Paylor R, Morgan JI, Leonard CS, Rudy B. Impaired Fast-Spiking, Suppressed Cortical Inhibition, and

Increased Susceptibility to Seizures in Mice Lacking Kv3.2 K+ Channel Proteins. J. Neurosci. 2000 Dec;20(24):9071-9085.

Li W, Kaczmarek K, Perney TM. Localization of Two High-Threshol Potassium Channel Subunits in the Rat Central Auditory System. J. Comp. Neuro. 2001 May;437:196-218.

Lu R, Bausch AE, Kallenborn-Gerhardt W, Stoetzer C, Debruin N, Ruth P, Geisslinger G, Leffler A, Lukowski R, Schmidtko A. Slack channels expressed in sensory neurons control neuropathic pain in mice. J. Neurosci. 2015 Jan;35(3):1125-35.

Markram H, Toledo-Rodriguez M, Wang Y, Gupta A, Silberberg, Wu C. Interneurons of the neocortical inhibitory system. Nat. Rev. Neurosci. 2004 Oct;5:793-807.

Martina M, Schultz JH, Ehmke H, Monyer H, Jonas P. Functional and Molecular Differences between Voltage-Gated K+ Channels of Fast-Spiking Interneurons and Pyramidal Neurons of Rat Hippocampus. J. Neurosci. 1998 Oct;18(20):8111-8125.

McCarberg BH, Nicholson BD, Todd KH, Palmer T, Penles L. The impact of pain on quality of life and the unmet needs of pain management: results from pain sufferers and physicians participating in an Internet survey. Am. J. Ther. 2008 Jul-Aug;15(4):312-20.

McDonald AJ, Mascagni F. Differential expression of Kv3.1b and Kv3.2 potassium channel subunits in interneurons of the basolateral amygdala. Neuroscience 2006;138:537-547.

McMahon A, Fowler SC, Perney TM, Akemann W, Knöpfel, Joho RH. Allele-dependent changes of olivocerebellar circuit properties in the absence of the voltage-gated potassium channels Kv3.1 and Kv3.3. Eur. J. Neurosci. 2004 Mar;19:3317-3327.

Pilati N, Large C, Forsythe I, Hamann M. Acoustic over-exposure triggers burst firing in dorsal cochlear nucleus fusiform cells. Hearing Research 2012;283;98-106.

Puente N, Mendizabal-Zubiaga J, Elezgarai I, Reuero L, Buceta I, Grandes P. Precise localization of the voltage-gated potassium channel subunits Kv3.1b and Kv3.3 revealed in the molecular layer of the rat cerebellar cortex by a pre-embedding immunogold method. Histochem. Cell. Biol. 2010 Sep;134:403-409.

Reynolds GP, Abdul-Monim Z, Neill JC, Zhang ZJ. Calcium Binding Protein Markers of GABA Deficits in Schizophrenia - Post Mortem Studies and Animal Models. Neurotox. Res. 2004 Feb;6(1):57-62.

Ritter DM, Ho C, O'Leary ME, Covarrubias M. Modulation of Kv3.4 channel N-type inactivation by protein kinase C shapes the action potential in dorsal root ganglion neurons. J. Physiol. 2012 Jan;590(Pt 1):145-61.

Ritter DM, Zemel BM, Hala TJ, O'Leary ME, Lepore AC, Covarrubias M. Dysregulation of Kv3.4 channels in dorsal root ganglia following spinal cord injury. J. Neurosci. 2015 Jan;35(3):1260-73.

Roberts L, Eggermont J, Caspary D, Shore S, Melcher J, Kaltenback J. Ringing Ears: The Neuroscience of Tinnitus. J. Neurosci. 2010:30(45);14972-14979.

Rudy B, McBain CJ. Kv3 channels: voltage-gated K+ channels designed for high-frequency repetitive firing. TRENDS in Neurosci. 2001 Sep;24(9):517-526.

Sacco T, de Luca A, Tempia F. Properties and expression of Kv3 channels in cerebellar Purkinje cells. Mol. Cell. Neurosci. 2006 Jul;33:170-179.

Schulz P, Steimer T. Neurobiology of Circadian Systems. CNS Drugs 2009;23(Suppl. 2):3-13.

Song P, Yang Y, Barnes-Davies M, Bhattacharjee A, Hamann M, Forsythe ID, Oliver DL, Kaczmarek LK. Acoustic environment determines phosphorylation state of the Kv3.1 potassium channel in auditory neurons Nat. Neurosci. 2005 Oct;8(10): 1335-1342.

Spencer KM, Nestor PG, Perlmutter R, Niznikiewicz MA, Klump MC, Frumin M, Shenton ME, McCarley RW. Neural synchrony indexes disordered perception and cognition in schizophrenia. PNAS 2004 Dec;101(49):17288-17293.

Sun S, Cohen CJ, Dehnhardt CM. Inhibitors of voltage-gated sodium channel Nav1.7: patent applications since 2010. Pharm. Pat. Anal. 2014 Sep;3(5):509-21.

U.S. Department of Health and Human Services, Food and Drug Administration. Draft Guidance for Industry Analgesic Indications: Developing Drug and Biological Products: http://www.fda.gov/downloads/drugs/guidancecomplianceregulatoryinformation/guidances/ucm3846 91.pdf 2014 Feb.

von Hehn C, Bhattacharjee A, Kaczmarek L. Loss of Kv3.1 Tonotopicity and Alterations in cAMP Response Element-Binding Protein Signaling in Central Auditory Neurons of Hearing Impaired Mice. J. Neurosci. 2004;24: 1936-1940.

Weiser M, Vega-Saenz de Miera E, Kentros C, Moreno H, Franzen L, Hillman D, Baker H, Rudy B. Differential Expression of Shaw-related K+ Channels in the Rat Central Nervous System. J. Neurosci. 1994 Mar;14(3):949-972.

Wickenden AD, McNaughton-Smith G. Kv7 channels as targets for the treatment of pain. Curr. Pharm. Des. 2009;15(15):1773-98.

Woolf CJ. What is this thing called pain? J. Clin. Invest. 2010 Nov;120(11):3742-4.

Woolf CJ. Central sensitization: implications for the diagnosis and treatment of pain. Pain 2011 Mar;152(3 Suppl):S2-15.

Yeung SYM, Thompson D, Wang Z, Fedida D, Robertson B. Modulation of Kv3 Subfamily Potassium Currents by the Sea Anemone Toxin BDS: Significance for CNS and Biophysical Studies. J. Neurosci. 2005 Mar;25(38):8735-8745.

Zamponi GW, Striessnig J, Koschak A, Dolphin AC. Pharmacol Rev. 2015 Oct;67(4):821-70.

## Claims

**1.** A compound of formula (I):

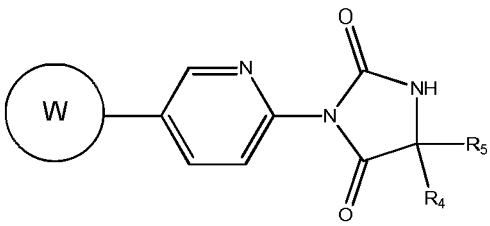

(I)

wherein:

W is group (Wa), group (Wb) or group (Wc):

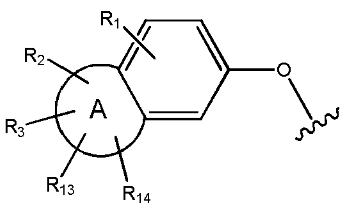

(Wa);

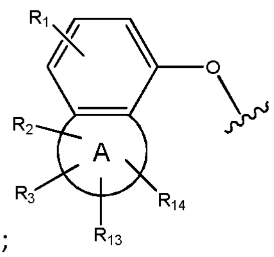

(Wb);

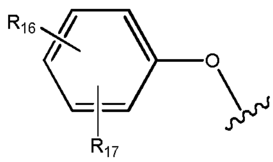

(Wc);

wherein:

$R_1$ is H, $C_{1-4}$alkyl, halo, halo$C_{1-4}$alkyl, CN, $C_{1-4}$alkoxy, or halo$C_{1-4}$alkoxy;
$R_2$ is H, $C_{1-4}$alkyl, $C_{3-5}$ spiro carbocyclyl, halo$C_{1-4}$alkyl or halo;
$R_3$ is H, $C_{1-4}$alkyl, halo$C_{1-4}$alkyl, halo; or $R_3$ is absent;
$R_{13}$ is H, $C_{1-4}$alkyl, halo$C_{1-4}$alkyl, halo; or $R_{13}$ is absent;
$R_{14}$ is H, $C_{1-4}$alkyl, halo$C_{1-4}$alkyl, halo; or $R_{14}$ is absent;
A is a 5 or 6 membered saturated or unsaturated heterocycle, with at least one O atom; which heterocycle is optionally fused with a cyclopropyl group, or a cyclobutyl group, or a cyclopentyl group to form a tricycle when considered together with the phenyl;
$R_{16}$ is halo, $C_{1-4}$ alkyl, $C_{1-4}$ alkoxy, halo-$C_{1-4}$alkyl, halo-$C_{1-4}$alkoxy, or CN;
$R_{17}$ is H, halo, cyano, $C_{1-4}$ alkyl or $C_{1-4}$ alkoxy; with the proviso that when $R_{17}$ is H, $R_{16}$ is not in the para position;
$R_4$ is $C_{1-4}$ alkyl;
$R_5$ is H or $C_{1-4}$ alkyl;
or $R_4$ and $R_5$ can be fused to form $C_{3-4}$ spiro carbocyclyl;

wherein $R_2$ and $R_3$ may be attached to the same or a different ring atom; $R_2$ may be attached to a fused ring atom; and wherein $R_{13}$ and $R_{14}$ may be attached to the same or a different ring atom;

or a pharmaceutically acceptable salt and/or solvate thereof, and/or prodrug thereof, wherein the prodrug is functionalised at the secondary nitrogen of the hydantoin, as defined below:

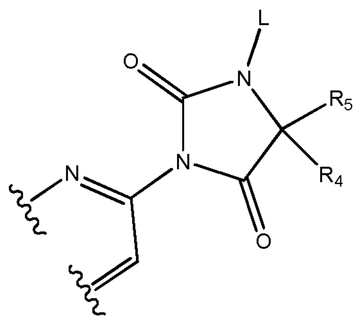

wherein L is selected from:

a) $-PO(OH)O^- \cdot M^+$, wherein $M^+$ is a pharmaceutically acceptable monovalent counterion,
b) $-PO(O^-)_2 \cdot 2M^+$,
c) $-PO(O^-)_2 \cdot D^{2+}$, wherein $D^{2+}$ is a pharmaceutically acceptable divalent counterion,
d) $-CH(R^X)-PO(OH)O^- \cdot M^+$, wherein $R^X$ is hydrogen or $C_{1-3}$ alkyl,
e) $-CH(R^X)-PO(O^-)_2 \cdot 2M^+$,
f) $-CH(R^X)-PO(O^-)_2 \cdot D^{2+}$
g) $-SO_3^- \cdot M^+$,
h) $-CH(R^X)-SO_3^- \cdot M^+$, and
i) $-CO-CH_2CH_2-CO_2 \cdot M^+$.

**2.** The compound according to claim 1, wherein W is group (Wa).

**3.** The compound according to claim 1, wherein W is group (Wb).

**4.** The compound according to any one of claims 1 to 3 wherein $R_1$ is H or methyl.

**5.** The compound according to any one of claims 1 to 4 wherein $R_2$ is H, $C_{1-4}$alkyl, $C_{3-5}$spiro carbocyclyl, or halo$C_{1-4}$alkyl.

**6.** The compound according to any one of claims 1 to 5, wherein $R_3$ is H or absent.

**7.** The compound according to any one of claims 1 to 6, wherein $R_{13}$ and $R_{14}$ are each independently H or absent.

**8.** The compound according to any one of claims 1 to 7, wherein $R_4$ is methyl or ethyl and $R_5$ is H or methyl.

**9.** The compound according to claim 1, wherein W is group (Wc).

**10.** The compound according to claim 1, selected from the group consisting of:

(5R)-5-ethyl-5-methyl-3-[5-(7-methylspiro[2H-benzofuran-3,1'-cyclopropane]-4-yl)oxy-2-pyridyl]imidazolidine-2,4-dione;
(5R)-3-[5-[(3,3-dimethyl-2H-benzofuran-4-yl)oxy]-2-pyridyl]-5-ethyl-5-methyl-imidazolidine-2,4-dione;
5,5-dimethyl-3-[5-(7-methylspiro[2H-benzofuran-3,1'-cyclopropane]-4-yl)oxy-2-pyridyl]imidazolidine-2,4-dione;
(5R)-5-ethyl-3-[5-(7-methylspiro[2H-benzofuran-3,1'-cyclopropane]-4-yloxy-2-pyridyl]imidazolidine-2,4-dione;
5,5-dimethyl-3-(5-spiro[2H-benzofuran-3,1'-cyclopropane]-4-yloxy-2-pyridyl)imidazolidine-2,4-dione; and
(5R)-5-ethyl-3-(5-spiro[2H-benzofuran-3,1'-cyclopropane]-4-yloxy-2-pyridyl)imidazolidine-2,4-dione;
or a pharmaceutically acceptable salt and/or solvate and/or prodrug thereof.

**11.** The compound according to any one of claims 1 to 10, for use as a medicament.

**12.** The compound for use according to claim 11, for use in the prophylaxis or treatment of schizophrenia.

**13.** The compound for use according to claim 11, for use in the prophylaxis or treatment of pain.

**14.** The compound for use according to claim 11, for use in the prophylaxis or treatment of Fragile-X syndrome.

**15.** The compound for use according to any one of claims 11 to 14, in conjunction with a further pharmaceutically active agent.

**Patentansprüche**

**1.** Verbindung der Formel (I):

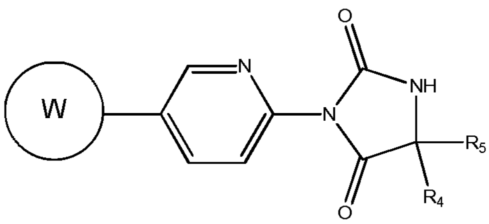
(I)

wobei:
W für Gruppe (Wa), Gruppe (Wb) oder Gruppe (Wc) steht:

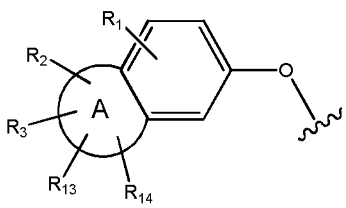
(Wa);

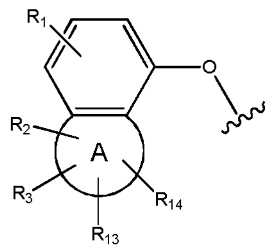
(Wb);

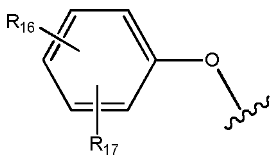
(Wc);

wobei:

$R_1$ für H, $C_{1-4}$-Alkyl, Halogen, Halogen-$C_{1-4}$-alkyl, CN, $C_{1-4}$-Alkoxy oder Halogen-$C_{1-4}$-alkoxy steht;
$R_2$ für H, $C_{1-4}$-Alkyl, $C_{3-5}$-Spirocarbocyclyl, Halogen-$C_{1-4}$-alkyl oder Halogen steht;
$R_3$ für H, $C_{1-4}$-Alkyl, Halogen-$C_{1-4}$-alkyl oder Halogen steht oder $R_3$ fehlt;
$R_{13}$ für H, $C_{1-4}$-Alkyl, Halogen-$C_{1-4}$-alkyl oder Halogen steht oder $R_{13}$ fehlt;
$R_{14}$ für H, $C_{1-4}$-Alkyl, Halogen-$C_{1-4}$-alkyl oder Halogen steht oder $R_{14}$ fehlt;
A für einen 5- oder 6-gliedrigen gesättigten oder ungesättigten Heterocyclus mit mindestens einem O-Atom steht; wobei der Heterocyclus gegebenenfalls mit einer Cyclopropylgruppe oder einer Cyclobutylgruppe oder einer Cyclopentylgruppe anelliert ist und so bei Betrachtung zusammen mit dem Phenyl einen Trizyklus bildet;
$R_{16}$ für Halogen, $C_{1-4}$-Alkyl, $C_{1-4}$-Alkoxy, Halogen-$C_{1-4}$-alkyl, Halogen-$C_{1-4}$-alkoxy oder CN steht;
$R_{17}$ für H, Halogen, Cyano, $C_{1-4}$-Akyl oder $C_{1-4}$-Alkoxy steht; mit der Maßgabe, dass dann, wenn $R_{17}$ für H steht, $R_{16}$ nicht in der para-Position steht;

$R_4$ für $C_{1-4}$-Alkyl steht;
$R_5$ für H oder $C_{1-4}$-Alkyl steht;
oder $R_4$ und $R_5$ zu einem $C_{3-4}$-Spirocarbocyclyl anelliert sein können;
wobei $R_2$ und $R_3$ an dasselbe Ringatom oder verschiedene Ringatome gebunden sein können;
$R_2$ an ein anelliertes Ringsystem gebunden sein kann und wobei $R_{13}$ und $R_{14}$ an dasselbe Ringatom oder verschiedene Ringatome gebunden sein können;

oder ein pharmazeutisch unbedenkliches Salz und/oder Solvat davon und/oder Prodrug davon, wobei das Prodrug am sekundären Stickstoff des Hydantoins funktionalisiert ist, wie unten definiert:

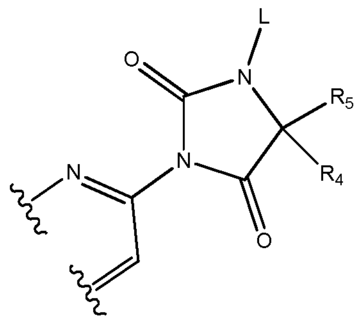

wobei L ausgewählt ist aus:

a) $-PO(OH)O^{-} \bullet M^{+}$, wobei $M^{+}$ für ein pharmazeutisch unbedenkliches einwertiges Gegenion steht,
b) $-PO(O^{-})_2 \bullet 2M^{+}$,
c) $-PO(O^{-})_2 \bullet D^{2+}$, wobei $D^{2+}$ für ein pharmazeutisch unbedenkliches zweiwertiges Gegenion steht,
d) $-CH(R^X)-PO(OH)O^{-} \bullet M^{+}$, wobei $R^X$ für Wasserstoff oder $C_{1-3}$-Alkyl steht,
e) $-CH(R^X)-PO(O^{-})_2 \bullet 2M^{+}$,
f) $-CH(R^X)-PO(O^{-})_2 \bullet D^{2+}$
g) $-SO_3^{-} \bullet M^{+}$,
h) $-CH(R^X)-SO_3^{-} \bullet M^{+}$ und
i) $-CO-CH_2CH_2-CO_2 \bullet M^{+}$.

2. Verbindung nach Anspruch 1, wobei W für Gruppe (Wa) steht.

3. Verbindung nach Anspruch 1, wobei W für Gruppe (Wb) steht.

4. Verbindung nach einem der Ansprüche 1 bis 3, wobei $R_1$ für H oder Methyl steht.

5. Verbindung nach einem der Ansprüche 1 bis 4, wobei $R_2$ für H, $C_{1-4}$-Alkyl, $C_{3-5}$-Spirocarbocyclyl oder Halogen-$C_{1-4}$-alkyl steht.

6. Verbindung nach einem der Ansprüche 1 bis 5, wobei $R_3$ für H steht oder fehlt.

7. Verbindung nach einem der Ansprüche 1 bis 6, wobei $R_{13}$ und $R_{14}$ jeweils unabhängig für H stehen oder fehlen.

8. Verbindung nach einem der Ansprüche 1 bis 7, wobei $R_4$ für Methyl oder Ethyl steht und $R_5$ für H oder Methyl steht.

9. Verbindung nach Anspruch 1, wobei W für Gruppe (Wc) steht.

10. Verbindung nach Anspruch 1, ausgewählt aus der Gruppe bestehend aus:

(5R)-5-Ethyl-5-methyl-3-[5-(7-methylspiro[2H-benzofuran-3,1'-cyclopropan]-4-yl)oxy-2-pyridyl]imidazolidin-2,4-dion;
(5R)-3-[5-[(3,3-Dimethyl-2H-benzofuran-4-yl)oxy]-2-pyridyl]-5-ethyl-5-methylimidazolidin-2,4-dion;
5,5-Dimethyl-3-[5-(7-methylspiro[2H-benzofuran-3,1'-cyclopropan]-4-yl)oxy-2-pyridyl]imidazolidin-2,4-dion;

(5R)-5-Ethyl-3-[5-(7-methylspiro[2H-benzofuran-3,1'-cyclopropan]-4-yloxy-2-pyridyl]imidazolidin-2,4-dion;
5,5-Dimethyl-3-(5-spiro[2H-benzofuran-3,1'-cyclopropan]-4-yloxy-2-pyridyl)imidazolidin-2,4-dion und
(5R)-5-Ethyl-3-(5-spiro[2H-benzofuran-3,1'-cyclopropan]-4-yloxy-2-pyridyl)imidazolidin-2,4-dion;
oder ein pharmazeutisch unbedenkliches Salz und/oder Solvat und/oder ein Prodrug davon.

**11.** Verbindung nach einem der Ansprüche 1 bis 10 zur Verwendung als Medikament.

**12.** Verbindung zur Verwendung nach Anspruch 11 zur Verwendung bei der Prophylaxe oder Behandlung von Schizophrenie.

**13.** Verbindung zur Verwendung nach Anspruch 11 zur Verwendung bei der Prophylaxe oder Behandlung von Schmerzen.

**14.** Verbindung zur Verwendung nach Anspruch 11 zur Verwendung bei der Prophylaxe oder Behandlung von Fragile-X-Syndrom.

**15.** Verbindung zur Verwendung nach einem der Ansprüche 11 bis 14 in Verbindung mit einem weiteren pharmazeutischen Wirkstoff.

## Revendications

**1.** Composé de formule (I) :

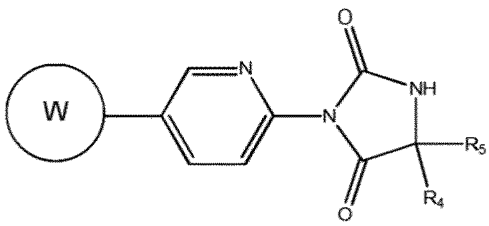

(I)

dans lequel :
W est un groupe (Wa), un groupe (Wb) ou un groupe (Wc) :

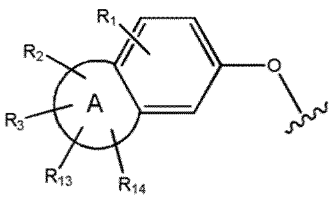
(Wa);
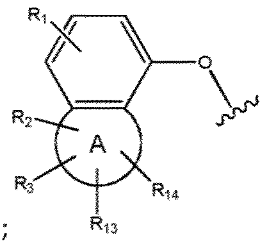
(Wb);
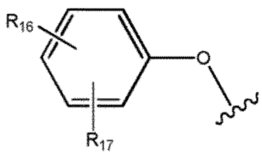
(Wc);

dans lequel :

$R_1$ est H ou un groupe alkyle en $C_{1-4}$, halogéno, halogénoalkyle en $C_{1-4}$, CN, alcoxy en $C_{1-4}$ ou halogénoalcoxy en $C_{1-4}$ ;
$R_2$ est H ou un groupe alkyle en $C_{1-4}$, carbocyclyle spiro en $C_{3-5}$, halogénoalkyle en $C_{1-4}$ ou halogéno ;
$R_3$ est H ou un groupe alkyle en $C_{1-4}$, halogénoalkyle en $C_{1-4}$, halogéno ; ou $R_3$ est absent ;
$R_{13}$ est H ou un groupe alkyle en $C_{1-4}$, halogénoalkyle en $C_{1-4}$, halogéno ; ou $R_{13}$ est absent ;
$R_{14}$ est H ou un groupe alkyle en $C_{1-4}$, halogénoalkyle en $C_{1-4}$, halogéno ; ou $R_{14}$ est absent ;
A est un hétérocycle à 5 ou 6 chaînons saturé ou insaturé, ayant au moins un atome O ; lequel hétérocycle est éventuellement condensé avec un groupe cyclopropyle ou un groupe cyclobutyle ou un groupe cyclopentyle pour former un tricycle lorsqu'il est considéré conjointement avec le groupe phényle ;
$R_{16}$ est un groupe halogéno, alkyle en $C_{1-4}$, alcoxy en $C_{1-4}$, halogénoalkyle en $C_{1-4}$, haloalcoxy en $C_{1-4}$ ou CN ;
$R_{17}$ est H ou un groupe halogéno, cyano, alkyle en $C_{1-4}$ ou alcoxy en $C_{1-4}$ ; à condition que lorsque $R_{17}$ est H,

$R_{16}$ ne soit pas en position para ;
$R_4$ est un groupe alkyle en $C_{1-4}$ ;
$R_5$ est H ou un groupe alkyle en $C_{1-4}$ ;
ou $R_4$ et $R_5$ peuvent être condensés pour former un groupe carbocyclyle spiro en $C_{3-4}$ ;
dans lequel $R_2$ et $R_3$ peuvent être liés au même atome de cycle ou à un atome de cycle différent ; $R_2$ peut être lié à un atome de cycle condensé ; et dans lequel $R_{13}$ et $R_{14}$ peuvent être liés au même atome de cycle ou à un atome de cycle différent ;

ou sel pharmaceutiquement acceptable et/ou solvate de celui-ci, et/ou promédicament de celui-ci, dans lequel le promédicament est fonctionnalisé au niveau de l'atome d'azote secondaire de l'hydantoïne, comme défini ci-dessous :

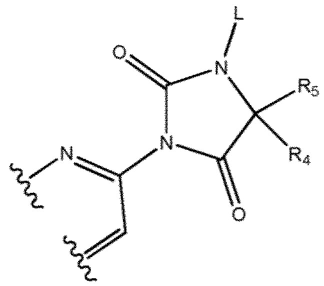

dans lequel L est choisi parmi :

a) $-PO(OH)O^{-}{\bullet}M^{+}$, dans lequel $M^{+}$ est un contre-ion monovalent pharmaceutiquement acceptable,
b) $-PO(O^{-})_2{\bullet}2M^{+}$,
c) $-PO(O^{-})_2{\bullet}D^{2+}$, dans lequel $D^{2+}$ est un contre-ion divalent pharmaceutiquement acceptable,
d) $-CH(R^{X})-PO(OH)O^{-}{\bullet}M^{+}$, dans lequel $R^{X}$ est l'atome d'hydrogène ou un groupe alkyle en $C_{1-3}$,
e) $-CH(R^{X})-PO(O^{-})_2{\bullet}2M^{+}$,
f) $-CH(R^{X})-PO(O^{-})_2{\bullet}D^{2+}$
g) $-SO_3^{-}{\bullet}M^{+}$,
h) $-CH(R^{X})-SO_3^{-}{\bullet}M^{+}$ et
i) $-CO-CH_2CH_2-CO_2{\bullet}M^{+}$.

**2.** Composé selon la revendication 1, dans lequel W est un groupe (Wa).

**3.** Composé selon la revendication 1, dans lequel W est un groupe (Wb).

**4.** Composé selon l'une quelconque des revendications 1 à 3 dans lequel $R_1$ est H ou un groupe méthyle.

**5.** Composé selon l'une quelconque des revendications 1 à 4 dans lequel $R_2$ est H ou un groupe alkyle en $C_{1-4}$, carbocyclyle spiro en $C_{3-5}$ ou halogénoalkyle en $C_{1-4}$.

**6.** Composé selon l'une quelconque des revendications 1 à 5, dans lequel $R_3$ est H ou absent.

**7.** Composé selon l'une quelconque des revendications 1 à 6, dans lequel $R_{13}$ et $R_{14}$ sont chacun indépendamment H ou absents.

**8.** Composé selon l'une quelconque des revendications 1 à 7, dans lequel $R_4$ est un groupe méthyle ou éthyle et $R_5$ est H ou un groupe méthyle.

**9.** Composé selon la revendication 1, dans lequel W est un groupe (Wc).

**10.** Composé selon la revendication 1, choisi dans le groupe constitué par :

la (5R)-5-éthyl-5-méthyl-3-[5-(7-méthylspiro[2H-benzofuran-3,1'-cyclopropane]-4-yl)oxy-2-pyridyl]imidazolidine-2,4-dione ;

la (5R)-3-[5-[(3,3-diméthyl-2H-benzofuran-4-yl)oxy]-2-pyridyl]-5-éthyl-5-méthylimidazolidine-2,4-dione ;
la 5,5-diméthyl-3-[5-(7-méthylspiro[2H-benzofuran-3,1'-cyclopropane]-4-yl)oxy-2-pyridyl]imidazolidine-2,4-dione ;
la (5R)-5-éthyl-3-[5-(7-méthylspiro[2H-benzofuran-3,1'-cyclopropane]-4-yloxy-2-pyridyl]imidazolidine-2,4-dione ;
la 5,5-diméthyl-3-(5-spiro[2H-benzofuran-3,1'-cyclopropane]-4-yloxy-2-pyridyl)imidazolidine-2,4-dione ; et
la (5R)-5-éthyl-3-(5-spiro[2H-benzofuran-3,1'-cyclopropane]-4-yloxy-2-pyridyl)imidazolidine-2,4-dione ;
ou un sel pharmaceutiquement acceptable et/ou solvate et/ou promédicament de celles-ci.

**11.** Composé selon l'une quelconque des revendications 1 à 10, destiné à être utilisé comme médicament.

**12.** Composé destiné à être utilisé selon la revendication 11, destiné à être utilisé dans la prophylaxie ou le traitement de la schizophrénie.

**13.** Composé destiné à être utilisé selon la revendication 11, destiné à être utilisé dans la prophylaxie ou le traitement de la douleur.

**14.** Composé destiné à être utilisé selon la revendication 11, destiné à être utilisé dans la prophylaxie ou le traitement du syndrome de l'X fragile.

**15.** Composé destiné à être utilisé selon l'une quelconque des revendications 11 à 14, conjointement avec un autre agent pharmaceutiquement actif.

Figure 1a: hKv3.2 currents recorded using the assay described in Biological Example 1, at two concentrations of the compound of Reference Example RE1

Figure 1a

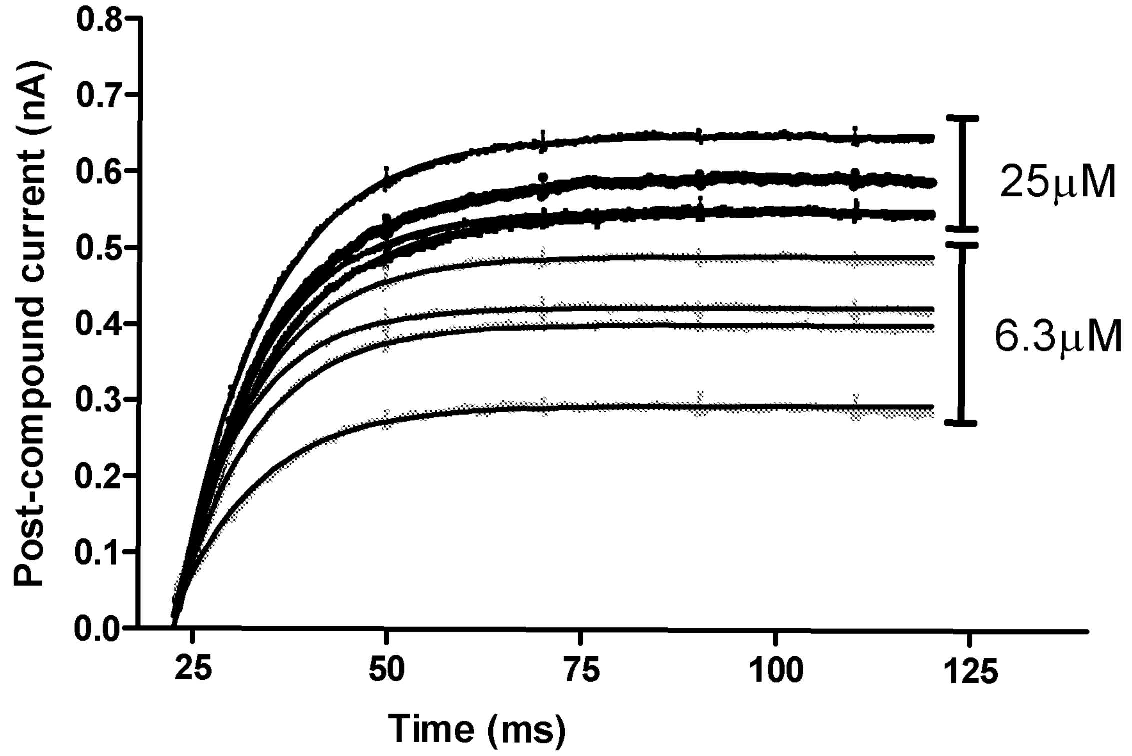

Figure 1b: hKv3.2 currents recorded using the assay described in Biological Example 1, at two concentrations of the compound of Reference Example RE3

Figure 1b

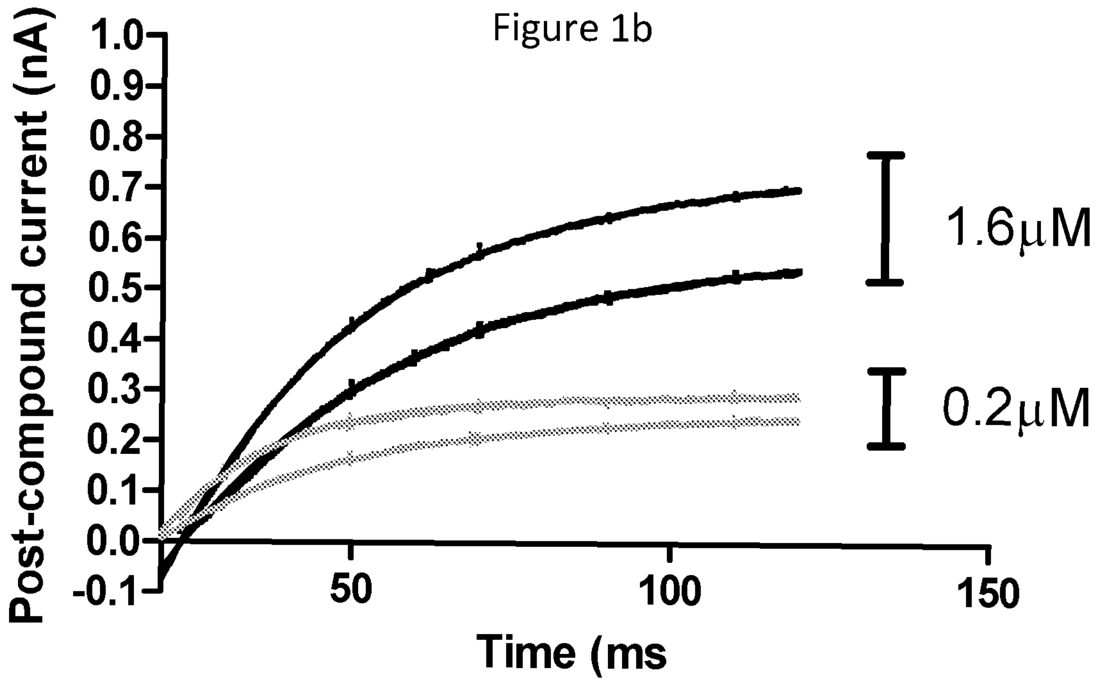

Figure 2

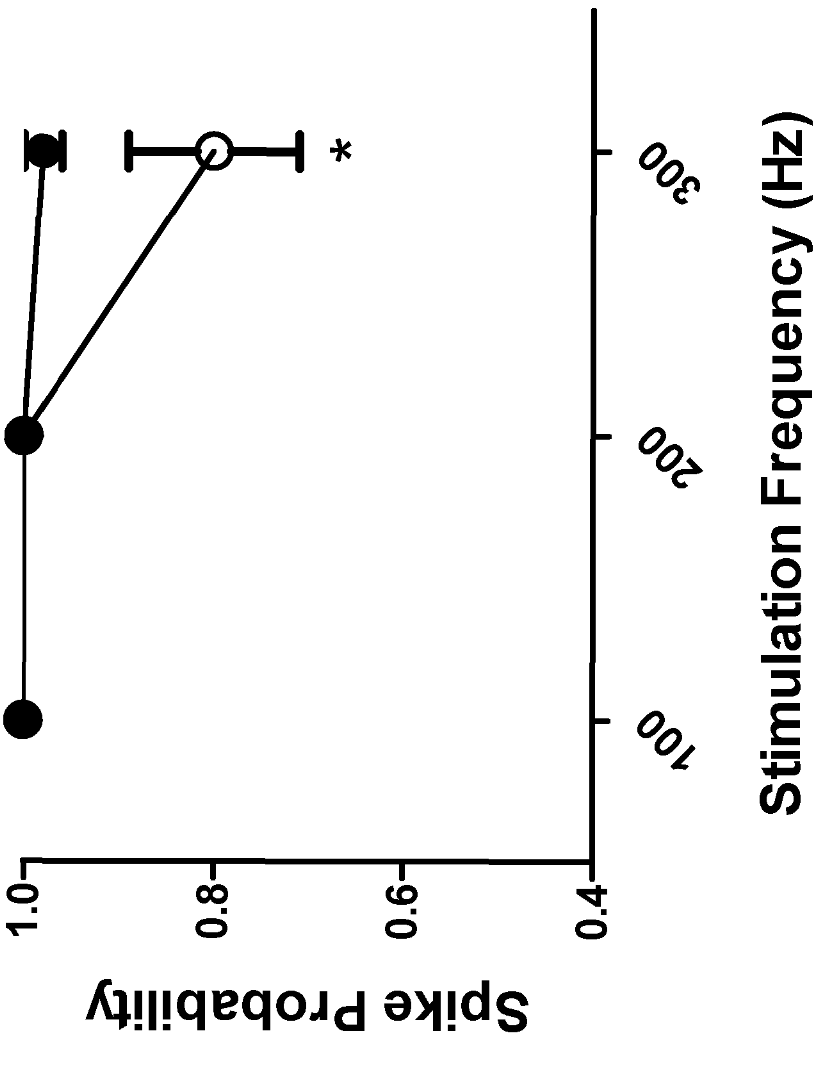
Spike Probability
1.0
0.8
0.6
0.4
*
100
200
300
Stimulation Frequency (Hz)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011069951 A
- WO 2012076877 A
- WO 2012168710 A
- WO 2013175215 A
- WO 2013182851 A
- WO 2013175211 A


### Non-patent literature cited in the description


- *Tetrahedron,* 2006, vol. 62 (42), 9966-9972
- **ARONIADOU-ANDERJASKA V ; QASHU F ; BRAGA MFM.** Mechanisms regulating GABAergic inhibitory transmission in the basolateral amygdala: implications for epilepsy and anxiety disorders. *Amino Acids,* August 2007, vol. 32, 305-315
- **BARANAUSKAS G ; NISTRI A.** Sensitization of pain pathways in the spinal cord: cellular mechanisms. *Prog. Neurobiol.,* February 1998, vol. 54 (3), 349-65
- **BARON R ; HANS G ; DICKENSON AH.** Peripheral input and its importance for central sensitization. *Ann. Neurol.,* November 2013, vol. 74 (5), 630-6
- **BEN-ARI Y.** Seizure Beget Seizure: The Quest for GABA as a Key Player. *Crit. Rev. Neurobiol.,* 2006, vol. 18 (1-2), 135-144
- **BENES FM ; LIM B ; MATZILEVICH D ; SUBBURAJU S ; WALSH JP.** Circuitry-based gene expression profiles in GABA cells of the trisynaptic pathway in schizophrenics versus bipolars. *PNAS,* December 2008, vol. 105 (52), 20935-20940
- **BENNETT DL ; WOODS CG.** Painful and painless channelopathies. *Lancet Neurol,* June 2014, vol. 13 (6), 587-99
- **BERGE S ; BIGHLEY L ; MONKHOUSE D ; PHARMACEUTICAL SALTS.** *J. Pharm. Sci.,* 1977, vol. 66, 1-19
- **BRAMBILLA P ; PEREZ J ; SCHETTINI G ; SOARES JC.** GABAergic dysfunction in mood disorders. *Mol. Psych.,* April 2003, vol. 8, 721-737
- **BROOKE RE ; PYNER S ; MCLEISH P ; BUCHAN S ; DEUCHARS J ; DEUCHARS SA.** Spinal cord interneurones labelled transneuronally from the adrenal gland by a GFP-herpes virus construct contain the potassium channel subunit Kv3.1b. *Auton. Neurosci.,* June 2002, vol. 98 (1-2), 45-50
- **BROOKE RE ; ATKINSON L ; BATTEN TF ; DEUCHARS SA ; DEUCHARS J.** Association of potassium channel Kv3.4 subunits with pre- and post-synaptic structures in brainstem and spinal cord. *Neuroscience,* 2004, vol. 126 (4), 1001-10
- **BROOKE RE ; ATKINSON L ; EDWARDS I ; PARSON SH ; DEUCHARS J.** Immunohistochemical localisation of the voltage gated potassium ion channel subunit Kv3.3 in the rat medulla oblongata and thoracic spinal cord. *Brain Res,* January 2006, vol. 1070 (1), 101-15
- **CERVERO F.** Spinal cord hyperexcitability and its role in pain and hyperalgesia. *Exp. Brain Res,* June 2009, vol. 196 (1), 129-37
- **CHANG SY ; ZAGHA E ; KWON ES ; OZAITA A ; BOBIK M ; MARTONE ME ; ELLISMAN MH ; HEINTZ N ; RUDY B.** Distribution of Kv3.3 Potassium Channel Subunits in Distinct Neuronal Populations of Mouse Brain. *J. Comp. Neuro.,* February 2007, vol. 502, 953-972
- **CHIEN LY ; CHENG JK ; CHU D ; CHENG CF ; TSAUR ML.** Reduced expression of A-type potassium channels in primary sensory neurons induces mechanical hypersensitivity. *J. Neurosci.,* September 2007, vol. 27 (37), 9855-65
- **CHOW A ; ERISIR A ; FARB C ; NADAL MS ; OZAITA A ; LAU D ; WELKER E ; RUDY B.** K+ Channel Expression Distinguishes Subpopulations of Parvalbumin- and Somatostatin-Containing Neocortical Interneurons. *J. Neurosci.,* November 1999, vol. 19 (21), 9332-9345
- **DESAI R ; KRONENGOLD J ; MEI J ; FORMAN S ; KACZMAREK L.** Protein Kinase C Modulates Inactivation of Kv3.3 Channels. *J. Biol. Chem.,* 2008, vol. 283, 22283-22294
- **DEUCHARS SA ; BROOKE RE ; FRATER B ; DEUCHARS J.** Properties of interneurones in the intermediolateral cell column of the rat spinal cord: role of the potassium channel subunit Kv3.1. *Neuroscience,* 2001, vol. 106 (2), 433-46
- **DEVULDER J.** Flupirtine in pain management: pharmacological properties and clinical use. *CNS Drugs,* October 2010, vol. 24 (10), 867-81

- **DIB-HAJJ SD ; YANG Y ; BLACK JA ; WAXMAN SG.** The Na(V)1.7 sodium channel: from molecule to man. *Nat. Rev. Neurosci.,* January 2013, vol. 14 (1), 49-62
- **DIOCHOT S ; SCHWEITZ H ; BÉRESS L ; LAZDUNSKI M.** Sea Anemone Peptides with a Specific Blocking Activity against the Fast Inactivating Potassium Channel Kv3.4. *J. Biol. Chem.,* March 1998, vol. 273 (12), 6744-6749
- **ENGEL AK ; FRIES P ; SINGER W.** Dynamic Predictions: Oscillations and Synchrony in Top-Down Processing. *Nat. Rev. Neurosci.,* October 2001, vol. 2 (10), 704-716
- **ESPINOSA F ; MCMAHON A ; CHAN E ; WANG S ; HO CS ; HEINTZ N ; JOHO RH.** Alcohol Hypersensitivity, Increased Locomotion, and Spontaneous Myoclonus in Mice Lacking the Potassium Channels Kv3.1 and Kv3.3. *J. Neurosci.,* September 2001, vol. 21 (17), 6657-6665
- **ESPINOSA F ; TORRES-VEGA MA ; MARKS GA ; JOHO RH.** Ablation of Kv3.1 and Kv3.3 Potassium Channels Disrupts Thalamocortical Oscillations In Vitro and In Vivo. *J. Neurosci.,* May 2008, vol. 28 (21), 5570-5581
- **FIGUEROA K ; MINASSIAN N ; STEVANIN G ; WATERS M ; GARIBYAN V ; FORLANI S ; STRZELCZYK A ; BURK K ; BRICE A ; DURR A.** KCNC3: phenotype, mutations, channel biophysics - a study of 260 familial ataxia patients. *Human Mutation,* 2010, vol. 31, 191-196
- **FINNERUP NB ; ATTAL N ; HAROUTOUNIAN S ; MCNICOL E ; BARON R ; DWORKIN RH ; GILRON I ; HAANPÄÄ M ; HANSSON P ; JENSEN TS.** Pharmacotherapy for neuropathic pain in adults: a systematic review and meta-analysis. *Lancet Neurol,* February 2015, vol. 14 (2), 162-73
- **FISAHN A.** Kainate receptors and rhythmic activity in neuronal networks: hippocampal gamma oscillations as a tool. *J. Physiol.,* October 2005, vol. 561 (1), 65-72
- **JOHO RH ; HO CS ; MARKS GA.** Increased $\gamma$- and Decreased $\delta$-Oscillations in a Mouse Deficient for a Potassium Channel Expressed in Fast-Spiking Interneurons. *J. Neurophysiol.,* June 1999, vol. 82, 1855-1864
- **JOHO RH ; HURLOCK EC.** The Role of Kv3-type Potassium Channels in Cerebellar Physiology and Behavior. *Cerebellum,* February 2009, vol. 8, 323-333
- **JUNG D ; LEE S ; KIM D ; JOO K ; CHA C ; YANG H ; LEE W ; CHUNG Y.** Age-related changes in the distribution of Kv1.1 and Kv3.1 in rat cochlear nuclei. *Neurol. Res.,* 2005, vol. 27, 436-440
- **KASTEN MR ; RUDY B ; ANDERSON MP.** Differential regulation of action potential firing in adult murine thalamocortical neurons by Kv3.2, Kv1, and SK potassium and N-type calcium channels. *J. Physiol.,* 2007, vol. 584 (2), 565-582
- **KACZMAREK L ; BHATTACHARJEE A ; DESAI R ; GAN L ; SONG P ; VON HEHN C ; WHIM M ; YANG B.** Regulation of the timing of MNTB neurons by short-term and long-term modulation of potassium channels. *Hearing Res,* 2005, vol. 206, 133-145
- **LAU D, VEGA-SAENZ DE MIERA E, CONTRERAS D, OZAITA A, HARVEY M, CHOW A, NOEBELS JL, PAYLOR R, MORGAN JI, LEONARD DS, RUDY B.** Rudy B. Impaired Fast-Spiking, Suppressed Cortical Inhibition, and Increased Susceptibility to Seizures in Mice Lacking Kv3.2 K+ Channel Proteins. *J. Neurosci.,* December 2000, vol. 20 (24), 9071-9085
- **LI W ; KACZMAREK K ; PERNEY TM.** Localization of Two High-Threshol Potassium Channel Subunits in the Rat Central Auditory System. *J. Comp. Neuro.,* May 2001, vol. 437, 196-218
- **LU R ; BAUSCH AE ; KALLENBORN-GERHARDT W ; STOETZER C ; DEBRUIN N ; RUTH P ; GEISSLINGER G ; LEFFLER A ; LUKOWSKI R ; SCHMIDTKO A.** Slack channels expressed in sensory neurons control neuropathic pain in mice. *J. Neurosci.,* January 2015, vol. 35 (3), 1125-35
- **MARKRAM H ; TOLEDO-RODRIGUEZ M ; WANG Y ; GUPTA A ; SILBERBERG ; WU C.** Interneurons of the neocortical inhibitory system. *Nat. Rev. Neurosci.,* October 2004, vol. 5, 793-807
- **MARTINA M ; SCHULTZ JH ; EHMKE H ; MONYER H ; JONAS P.** Functional and Molecular Differences between Voltage-Gated K+ Channels of Fast-Spiking Interneurons and Pyramidal Neurons of Rat Hippocampus. *J. Neurosci.,* October 1998, vol. 18 (20), 8111-8125
- **MCCARBERG BH ; NICHOLSON BD ; TODD KH ; PALMER T ; PENLES L.** The impact of pain on quality of life and the unmet needs of pain management: results from pain sufferers and physicians participating in an Internet survey. *Am. J. Ther.,* July 2008, vol. 15 (4), 312-20
- **MCDONALD AJ ; MASCAGNI F.** Differential expression of Kv3.1b and Kv3.2 potassium channel subunits in interneurons of the basolateral amygdala. *Neuroscience,* 2006, vol. 138, 537-547
- **MCMAHON A ; FOWLER SC ; PERNEY TM ; AKEMANN W ; KNÖPFEL ; JOHO RH.** Allele-dependent changes of olivocerebellar circuit properties in the absence of the voltage-gated potassium channels Kv3.1 and Kv3.3. *Eur. J. Neurosci.,* March 2004, vol. 19, 3317-3327
- **PILATI N ; LARGE C ; FORSYTHE I ; HAMANN M.** Acoustic over-exposure triggers burst firing in dorsal cochlear nucleus fusiform cells. *Hearing Research,* 2012, vol. 283, 98-106

- **PUENTE N ; MENDIZABAL-ZUBIAGA J ; ELEZGARAI I ; REUERO L ; BUCETA I ; GRANDES P.** Precise localization of the voltage-gated potassium channel subunits Kv3.1b and Kv3.3 revealed in the molecular layer of the rat cerebellar cortex by a pre-embedding immunogold method. *Histochem. Cell. Biol.,* September 2010, vol. 134, 403-409
- **REYNOLDS GP ; ABDUL-MONIM Z ; NEILL JC ; ZHANG ZJ.** Calcium Binding Protein Markers of GABA Deficits in Schizophrenia - Post Mortem Studies and Animal Models. *Neurotox. Res.,* February 2004, vol. 6 (1), 57-62
- **RITTER DM ; HO C ; O'LEARY ME.** Covarrubias M. Modulation of Kv3.4 channel N-type inactivation by protein kinase C shapes the action potential in dorsal root ganglion neurons. *J. Physiol.,* January 2012, vol. 590, 145-61
- **RITTER DM ; ZEMEL BM ; HALA TJ ; O'LEARY ME ; LEPORE AC ; COVARRUBIAS M.** Dysregulation of Kv3.4 channels in dorsal root ganglia following spinal cord injury. *J. Neurosci.,* January 2015, vol. 35 (3), 1260-73
- **ROBERTS L ; EGGERMONT J ; CASPARY D ; SHORE S ; MELCHER J ; KALTENBACK J.** Ringing Ears: The Neuroscience of Tinnitus. *J. Neurosci.,* 2010, vol. 30 (45), 14972-14979
- **RUDY B ; MCBAIN CJ.** Kv3 channels: voltage-gated K+ channels designed for high-frequency repetitive firing. *TRENDS in Neurosci,* September 2001, vol. 24 (9), 517-526
- **SACCO T ; DE LUCA A ; TEMPIA F.** Properties and expression of Kv3 channels in cerebellar Purkinje cells. *Mol. Cell. Neurosci.,* July 2006, vol. 33, 170-179
- **SCHULZ P ; STEIMER T.** Neurobiology of Circadian Systems. *CNS Drugs,* 2009, vol. 23 (2), 3-13
- **SONG P ; YANG Y ; BARNES-DAVIES M ; BHATTACHARJEE A ; HAMANN M ; FORSYTHE ID ; OLIVER DL ; KACZMAREK LK.** Acoustic environment determines phosphorylation state of the Kv3.1 potassium channel in auditory neurons. *Nat. Neurosci.,* October 2005, vol. 8 (10), 1335-1342
- **SPENCER KM ; NESTOR PG ; PERLMUTTER R ; NIZNIKIEWICZ MA ; KLUMP MC ; FRUMIN M ; SHENTON ME ; MCCARLEY RW.** Neural synchrony indexes disordered perception and cognition in schizophrenia. *PNAS,* December 2004, vol. 101 (49), 17288-17293
- **SUN S ; COHEN CJ ; DEHNHARDT CM.** Inhibitors of voltage-gated sodium channel Nav1.7: patent applications since 2010. *Pharm. Pat. Anal.,* September 2014, vol. 3 (5), 509-21
- **VON HEHN C ; BHATTACHARJEE A ; KACZMAREK L.** Loss of Kv3.1 Tonotopicity and Alterations in cAMP Response Element-Binding Protein Signaling in Central Auditory Neurons of Hearing Impaired Mice. *J. Neurosci.,* 2004, vol. 24, 1936-1940
- **WEISER M ; VEGA-SAENZ DE MIERA E ; KENTROS C ; MORENO H ; FRANZEN L ; HILLMAN D ; BAKER H ; RUDY B.** Differential Expression of Shaw-related K+ Channels in the Rat Central Nervous System. *J. Neurosci.,* March 1994, vol. 14 (3), 949-972
- **WICKENDEN AD ; MCNAUGHTON-SMITH G.** Kv7 channels as targets for the treatment of pain. *Curr. Pharm. Des.,* 2009, vol. 15 (15), 1773-98
- **WOOLF CJ.** What is this thing called pain. *J. Clin. Invest.,* November 2010, vol. 120 (11), 3742-4
- **WOOLF CJ.** Central sensitization: implications for the diagnosis and treatment of pain. *Pain,* March 2011, vol. 152 (3), S2-15
- **YEUNG SYM ; THOMPSON D ; WANG Z ; FEDIDA D ; ROBERTSON B.** Modulation of Kv3 Subfamily Potassium Currents by the Sea Anemone Toxin BDS: Significance for CNS and Biophysical Studies. *J. Neurosci.,* March 2005, vol. 25 (38), 8735-8745
- **ZAMPONI GW ; STRIESSNIG J ; KOSCHAK A ; DOLPHIN AC.** *Pharmacol Rev,* October 2015, vol. 67 (4), 821-70